(19) Europäisches Patentamt
European Patent Office
Office européen des brevets

(11) EP 2 553 026 B1

(12) **EUROPÄISCHE PATENTSCHRIFT**

(45) Veröffentlichungstag und Bekanntmachung des Hinweises auf die Patenterteilung:
**28.03.2018 Patentblatt 2018/13**

(21) Anmeldenummer: **11706183.8**

(22) Anmeldetag: **28.02.2011**

(51) Int Cl.:
***C09C 1/36*** (2006.01) ***A61K 8/29*** (2006.01)
***A61Q 19/04*** (2006.01) ***A61K 8/24*** (2006.01)
***A61K 8/35*** (2006.01) ***A61Q 17/04*** (2006.01)
***A61K 8/02*** (2006.01) ***A61K 9/50*** (2006.01)
***B01J 13/20*** (2006.01) ***C07F 7/28*** (2006.01)
***C07C 45/86*** (2006.01)

(86) Internationale Anmeldenummer:
**PCT/EP2011/000967**

(87) Internationale Veröffentlichungsnummer:
**WO 2011/116870 (29.09.2011 Gazette 2011/39)**

(54) **METALLOXID-PARTIKEL NACHBEHANDELT MIT ORGANISCHEN PHOSPHORVERBINDUNGEN**

METAL OXIDE PARTICLES AFTER-TREATED WITH ORGANIC PHOSPHORUS COMPOUNDS

PARTICULES D'OXYDE MÉTALLIQUE POST-TRAITÉES AVEC DES COMPOSÉS DE PHOSPHORE ORGANIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **26.03.2010 EP 10003251**

(43) Veröffentlichungstag der Anmeldung:
**06.02.2013 Patentblatt 2013/06**

(73) Patentinhaber:
- **Merck Patent GmbH**
  **64293 Darmstadt (DE)**
- **Sachtleben Chemie GmbH**
  **47198 Duisburg (DE)**

(72) Erfinder:
- **BECK, Joern**
  **64342 Seeheim-Jugenheim (DE)**
- **GRAF, Ruediger**
  **64853 Otzberg (DE)**
- **PFLUECKER, Frank**
  **64297 Darmstadt (DE)**
- **BENKNER, Gabriele**
  **47445 Moers (DE)**
- **HIRTHE, Bernd**
  **47918 Toenisvorst (DE)**
- **JOHN, Stephan**
  **47198 Duisburg (DE)**

(56) Entgegenhaltungen:
**EP-A1- 0 707 051**
**WO-A1-02/051945**
**WO-A2-2007/070204**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 553 026 B1

## Beschreibung

Die Erfindung betrifft die Verwendung von Metalloxid-Partikeln und/oder Metallhydroxid-Partikeln, nachbehandelt mit einer organischen Phosphorverbindung nach Anspruch 1, sowie spezielle Partikel nach Anspruch 2 und deren Herstellung und Verwendung.

Die vorliegende Erfindung betrifft weiter neue Zubereitungen zur topischen Anwendung, die insbesondere das Selbstbräunungsmittel Dihydroxyaceton in Kombination mit den erfindungsgemäßen Metalloxid/Metallhydroxid-Partikeln zum Schutze der Haut und/oder Haare gegen UV-Strahlung beinhalten.

Dihydroxyaceton (DHA) wird seit vielen Jahren als Selbstbräunungsmittel in der Kosmetik eingesetzt. In den letzten Jahren geht der Trend auch verstärkt dazu, Selbstbräunungsprodukte mit UV-Filtern, beispielsweise für die Tagespflege, zu kombinieren. Dies war in der Vergangenheit jedoch nur beschränkt möglich, da DHA in Kombination mit konventionellen anorganischen UV-Filtern, insbesondere $TiO_2$, einen starken Abbau während der Lagerung zeigt.

Konventionelle mikronisierte Titandioxid-Partikel werden zur Verringerung der photokatalytischen Aktivität mit anorganischen Oxiden bzw. Hydroxiden der Elemente Al, Si, Zr, Ce und Fe nachbehandelt. Als besonders geeignet erweist sich hier die Nachbehandlung mit $Al_2O_3$. Neben der photokatalyischen Aktivität, die beim Einsatz dieser Produkte zum Abbau bzw. zur Zerstörung der umgebenden Matrix führt, kommt es insbesondere in kosmetischen Formulierungen zu weiteren unerwünschten Effekten:

- Abbau von DHA wenn dies in Formulierungen mit $TiO_2$ kombiniert wird
- Farbreaktionen der $TiO_2$-Partikel mit DHA sowie mit weiteren organischen Molekülen in der Zubereitung
- Vergrauung der Formulierungen bei UV-Bestrahlung.

Einige dieser Effekte, beispielsweise die Vergrauung oder Farbreaktionen mit beispielsweise Ascorbylpalmitat, können durch diverse Nachbehandlungen der Titandioxid-Partikel reduziert werden, beispielsweise erreicht durch die Qualitäten der Marktprodukte Parsol® TX von DSM oder UV-TITAN M263 von Sachtleben.

Parsol® TX ist ein weisses Pulver, bestehend aus Titandioxid der Rutilkristallstruktur mit einem $TiO_2$-Gehalt von 82.0 bis 87.0 Gewichtsprozent, mit einer $SiO_2$-Beschichtung mit einem $SiO_2$-Gehalt von 10.5 bis 14.5 Gewichtsprozent und einer Dimethicone-Beschichtung. UV-TITAN M263 ist ein weisses Pulver bestehend aus Titandioxid (88%) mit einem anorganischen Bestandteil von 7% an Aluminiumphosphat und einem organischen Bestandteil von 3% von Polyvinylpyrrolidon (INCI des Produktes: Titanium Dioxide, Alumina, Sodium Hexametaphosphate, Polyvinylpyrrol idone).

WO 2007/070204 offenbart Titandioxid-Nanopartikel, die mit einer Aminoalylenphosphonsäure beschichtet ist, beispielsweise mit Ethylendiamintetra(methylenphosphonsäure) oder Diethylentriaminpenta(ethylenphosphonsäure), und die in Polymeren eingesetzt die Dispersion in Kunststoffen ermöglichen.

EP 0707051 offenbart die Oberflächenbehandlung von anorganischen Feststoffen mit Alkylphosphonsäuren, wie n-Octylphosphonsäure, oder Estern der Alkylphosphonsäure.

WO 02/051945 offenbart nanopartikuläres Titandioxid, welche von einem polaren organischen Oberflächenmodifikationsmittel, vorzugsweise einer Phosphonsäure, ummantelt sind. Besonders bevorzugte Oberflächenmodifikationsmittel sind Mono-, Di- und Trisphosphonsäuren, wie beispielsweise Tris(phosphonomethyl)amin, Azacycloheptan-2,2-diphosphonsäure und Hydroxyethan-1,1-diphosphonsäure.

Es hat sich aber gezeigt, dass selbst bekannte konventionelle mikronisierte Titandioxidpartikel, wie zuvor beschrieben, die mit diversen Nachbehandlungen wie $SiO_2$ und/oder weiteren Nachbehandlungen versehen sind, und diese gute photokatalytische Stabilität und/oder geringe Reaktivitäten mit anderen organischen Molekülen zeigen, diese nicht automatisch auch mit Dihydroxyaceton kompatibel sind.

Die Aufgabe der Erfindung liegt daher in der Bereitstellung von Metalloxid-Partikeln und/oder Metallhydroxid-Partikeln, insbesondere von Titandioxid-Partikeln, die mit Dihydroxyaceton oder Dihydroxyacetonderivaten, insbesondere mit Dihydroxyaceton, kompatibel sind.

Jetzt wurde überraschend gefunden, dass es möglich ist, bestimmte Metalloxid-Partikel und/oder Metallhydroxid-Partikel, insbesondere Titandioxid-Partikel als UV-Schutzmittel, in kosmetischen Formulierungen, die Dihydroxyaceton enthalten, einzusetzen und dabei den Abbau des Dihydroxyacetons in den kosmetischen Formulierungen zu vermindern oder zu verhindern. Der Abbau des Dihydroxyacetons in der kosmetischen Zubereitung ist insbesondere geringer im Vergleich zu kosmetischen Zubereitungen enthaltend Dihydroxyaceton und Partikel, die identisch vom Aufbau sind, jedoch keine erfindungsgemäße Nachbehandlung aufweisen. Der Abbau des Dihydroxyacetons in einer kosmetischen Zubereitung enthaltend die erfindungsgemäßen Partikel und Dihydroxyaceton ist beispielsweise geringer im Vergleich zu kosmetischen Zubereitungen enthaltend Dihydroxyaceton und kommerziell verfügbare bekannte Titandioxid-UV-Schutzmittel. Weiterhin wurde gefunden, dass die bevorzugten erfindungsgemäßen Titandioxid-Partikel auch die Eigenschaft haben, Farbreaktionen des Titandioxids mit organischen Verbindungen weitgehend zu verhindern, wobei die photokatalytische Aktivität sowie die Vergrauungsstabilität vergleichbar zu bekannten Titandioxid-UV-Schutzmitteln

bleibt.

Gelöst wird die Aufgabe durch eine Nachbeschichtung der Metalloxid-Partikel und/oder Metallhydroxid-Partikel, insbesondere durch eine Nachbeschichtung von Titandioxid-Grundpartikeln, mit einer speziellen organischen Phosphorverbindung.

Gegenstand der Erfindung ist daher die Verwendung von Metalloxid-Partikeln und/oder Metallhydroxid-Partikeln mit einer Primärpartikelgröße nach der Scherrer-Methode im Bereich von 5 nm bis 100 nm, nachbehandelt mit einer organischen Phosphorverbindung, ausgewählt aus der Gruppe der Hydroxyalkyldiphosphonsäuren, Alkylphosphonsäuren, die durch mindestens eine COOH-Gruppe substituiert sein können, Aminoalkylenphosphonsäuren oder organische Phosphorsäureester oder deren Salze nach Anspruch 1.

Das erhaltene Produkt ist vorzugsweise durch einen Wassergehalt von größer 1,5Gew.-% gekennzeichnet. Der Wassergehalt kann beispielsweise durch Karl-Fischer-Titration bestimmt werden, wie beschrieben in Eugen Scholz, Karl-Fischer-Titration, Springer-Verlag 1984.

Aus JP 2950495 sind Anstrichfarben mit Titandioxidpartikeln bekannt, wobei die Titandioxidpartikel mit einem organischen Phosphorsäureester nachbehandelt sind. Es wurden hierbei jedoch Titandioxidpartikel mit einer durchschnittlichen Partikelgröße zwischen 150 bis 400 nm verwendet, d.h. Pigmente, die für Anstrichfarben geeignet sind. Die nachbehandelten Titandioxidpartikel in der Anstrichfarbe bewirken nach der Lehre von JP 2950495 eine Verbesserung der wasserabweisenden Eigenschaften der Farbschicht. JP 2950495 enthält weder die Lehre, Titandioxidpartikel mit kleinerer Größe generell nachzubehandeln, noch einen Ausblick, dass die erfindungsgemäßen Pigmente für die Kosmetik relevant wären.

EP 154150 beschreibt ein Make-up-Kosmetikum oder ein Körperkosmetikum enthaltend anorganische Füllpigmente und Kompositpigmente, nachbehandelt mit einem Dialkylphosphat mit Alkylgruppen mit 14 bis 22 C-Atomen, welche dem Kosmetikum ein verbessertes Waserabstoßungsvermögen und eine verbesserte Weichheit auf der Haut ermöglichen. Verwendete anorganische Füllpigmente sind Talk, Sericit, Mica nachbehandelt mit Titandioxid. Erfindungsgemäße Partikel, insbesondere erfindungsgemäße Titandioxidpartikel, werden nicht beschrieben.

Erfindungsgemäß wird die organische Phosphorverbindung in einer Menge von 5 bis 50 Gewichstprozent, bevorzugt von 5 bis 20 Gewichtsprozent, besonders bevorzugt von 6 bis 15 Gewichtsprozent aufgebracht. Ganz besonders bevorzugt wird ein organischer Phosphorsäuremonoalkylester in einer Menge von 6 bis 9 Gewichtsprozent aufgebracht.

Die Ermittlung des Gehalts an organischer Phosphorverbindung erfolgt durch Bestimmung des organischen Kohlenstoffes. Dazu wird die Probe bei 1300°C im Sauerstoffstrom verbrannt und das entstandene Kohlendioxid infrarotanalytisch detektiert. Gemessen wird z.B. mit dem CS-Bestimmungsautomat Eltra CS580. Die genaue Methodenbeschreibung wird im experimentellen Teil dargestellt.

Das Metalloxid und/oder -hydroxid für die erfindungsgemäße Verwendung kann ausgewählt werden aus der Gruppe der Oxide bzw. Hydroxide von Silizium, Titan, Zink, Aluminium, Cer, Eisen, Yttrium oder Zirkonium oder Mischungen hieraus. Diese Metalloxide und/oder Metallhydroxide bilden den Grundpartikel, der mindestens mit der organischen Phosphorverbindung nachbehandelt wird.

Bevorzugt wird das Metalloxid und/oder Metallhydroxid aus der Gruppe der Oxide bzw. Hydroxide von Silizium, Titan oder Zink ausgewählt. Besonders bevorzugte erfindungsgemäße Partikel sind Titandioxid-Partikel.

Die Grundpartikel können vor Nachbehandlung mit der organischen Phosphorverbindung mit mindestens einer weiteren Beschichtung versehen sein, die beispielsweise Metalloxide bzw. -hydroxide von Silizium, Titan, Zink, Aluminium, Cer, Eisen, Yttrium, Mangan oder Zirkonium enthalten, wobei vorzugsweise das Metall unterschiedlich zu dem Metall des Grundpartikels gewählt wird.

Die weitere Beschichtung kann auch organische Säuren, ausgewählt aus der Gruppe Stearinsäure, Laurinsäure, Capronsäure oder Palmitinsäure, Polyole, Polymere oder silikonorganische Verbindungen enthalten.

Polyole sind beispielsweise die Verbindungen Glycerin, Sorbitol, Propylenglycol, Pentandiol, Hexandiol, Ethylhexyl-Glycerin, Pentaerythritol, Dipentaerythritol oder Trimethylolpropan. Eine silikonorganische Verbindung ist beispielsweise Octyltrimethylsilan, Methicon, Dimethicon (= Triethoxy-caprylsilan), Trimethoxycaprylsilan, Dimethicon/Methicon Copolymer, Diphenyl Capryl Methicone, Polymethyl-methacrylate dimethicone oder Simethicon.

Polymere sind beispielsweise Polyvinylpyrrolidon (PVP) oder Polyethylen (PE).

Gegebenenfalls ist es vorteilhaft, den Grundpartikel vor der Nachbehandlung mit einer organischen Phosphorverbindung mit Aluminium- und/oder Siliziumhydroxid oder - oxid/dioxid und/oder Manganoxid/hydroxid vorzubehandeln, vorzugsweise mit Siliziumhydroxid oder -oxid/dioxid und Manganoxid/hydroxid oder Siliziumhydroxid oder - oxid/dioxid, besonders bevorzugt mit Siliziumhydroxid oder -oxid/dioxid.

In einer bevorzugten Ausführungsform der Partikel, insbesondere der erfindungsgemäßen Titandioxid-Partikel, wird neben der Nachbehandlung mit einer organischen Phosphorverbindung oder derjenigen gemäß Anspruch 2 eine weitere Beschichtung aufgebracht, enthaltend Aluminium- und/oder Siliziumhydroxid oder - oxid/dioxid und/oder Manganoxid/hydroxid, vorzugsweise Siliziumhydroxid oder-oxid/dioxid und Manganoxid/hydroxid oder Siliziumhydroxid oder -oxid/dioxid, besonders bevorzugt Siliziumhydroxid oder -oxid/dioxid, die gegebenenfalls noch weitere Beschichtungen

enthalten kann, wie zuvor beschrieben. Der Gehalt an Aluminium- und/oder Siliziumhydroxid oder -oxid/dioxid liegt beispielsweise im Bereich von 5 bis 60 Gewichtsprozent, bevorzugt im Bereich von 8 bis 45 Gewichtsprozent, besonders bevorzugt im Bereich von 10 bis 35 Gewichtsprozent, bezogen auf den $TiO_2$-Gehalt der zu beschichtenden Partikel und danach erfolgt die Nachbehandlung mit einer/der organischen Phosphorverbindung. Der Anteil der manganhaltigen Schicht, insbesondere der Anteil an Mangandioxid und/oder Manganhydroxid, bezogen auf das Gesamtpartikel beträgt 0.1 Gew.% bis 1 Gew.%, vorzugsweise 0.2 Gew.% bis 0.7 Gew.% und insbesondere bevorzugt 0.2 Gew.% oder 0.5 Gew.%. Vorzugsweise wird der Grundpartikel, insbesondere der Titandioxid-Grundpartikel, mit Siliziumhydroxid- oder -oxid/dioxid behandelt, wie zuvor beschrieben.
Die Beschichtung mit Siliziumhydroxid- oder -oxid/dioxid wird im folgenden auch als Siliziumdioxid-Beschichtung bezeichnet.

Die Bestimmung des Gehalts an $SiO_2$ der Siliziumhydroxid- oder - oxid/dioxid-Beschichtung erfolgt basierend auf ICP-OES. Die Schwankungsbreite der erhaltenen Werte des Gehalts an $SiO_2$ liegt in dem angegebenen Bereich von 25 bis 45 Gewichtsprozent, bezogen auf das Titandioxid bei plus/minus 0.25%.
ICP-OES steht für Inductively Coupled Plasma Optical Emission Spectrometry, also der optischen Emissionsspektrometrie mittels induktiv gekoppeltem Plasma. Gemessen wird mit dem Gerät Optima 3300DV der Firma Perkin Elmer, mit der Methode beschrieben im experimentellen Teil der Beschreibung unter dem Beispiel $SiO_2$-Bestimmung.

Die Siliziumdioxid-Beschichtung soll die Grundpartikel oder mit anderen Worten das partikuläre Metalloxid und/oder Metallhydroxid, insbesondere das nanopartikuläre Metalloxid und/oder Metallhydroxid, möglichst vollständig bedecken.

Ganz besonders bevorzugt wird der Titandioxid-Grundpartikel daher zunächst mit einer Silizium-Beschichtung versehen, wobei der Gehalt an Siliziumhydroxid oder -oxid/dioxid im Bereich von 20 bis 35 Gewichtsprozent liegt, bezogen auf den $TiO_2$-Gehalt der zu beschichtenden Partikel und danach erfolgt die Nachbehandlung mit einer organischen Phosphorverbindung, wie nachfolgend beschrieben oder nachfolgend als bevorzugt beschrieben. Vorzugsweise liegt der Gehalt an Siliziumhydroxid oder -oxid/dioxid bei 20 oder 35 Gewichtsprozent.

Die beschriebenen Partikel, insbesondere die Titandioxid-Partikel, haben eine Primärpartikelgröße nach der Scherrer-Methode im Bereich von 5 nm bis 100 nm, vorzuzugsweise im Bereich 8 bis 50 nm und insbesondere bevorzugt unterhalb von 25 nm. Die im Transmissionselektronenmikroskop ermittelbaren Abmessungen des erfindungsgemäßen nanopartikulären Titandioxids liegen üblicherweise bei einer Länge von 10 bis 100 nm und einer Breite von 5 bis 70 nm. Vorzugsweise liegt die Länge im Bereich von 30 bis 70 nm und die Breite im Bereich von 10 bis 40 nm.

Nanopartikel im Sinne der Erfindung sind Partikel, deren drei Dimensionen im Nanobereich vorliegen, d.h. <100 nm. Dies sind die drei Dimensionen der Primärpartikel.

Aggregate im Sinne der Erfindung sind ein Zusammenschluss von Primärpartikeln, durch chemische oder physikalische Bindung, wobei die externe Oberfläche signifikant kleiner ist als die Summe der berechneten Oberflächen der individuellen Partikel, d.h. der Primärpartikel. Aggregate im Sinne der Erfindung haben Abmessungen von 30 nm bis 150 nm.

Agglomerate im Sinne der Erfindung sind ein Zusammenschluss von Primärpartikeln oder/und Aggregaten, durch chemische oder physikalische Bindung, wobei die externe Oberfläche nicht signifikant kleiner ist als die Summe der berechneten Oberflächen der individuellen Partikel, wobei jedoch die Abmessungen in Bereichen zwisschen 0,1 μm und 100 μm liegen.

Der Begriff Titandioxid-Partikel umfasst daher sowohl die Primärpartikel, als auch Aggregate oder Agglomerate. Im Zuge einer Vermahlung kann die Größe der Zusammenschlüsse der Primärpartikel, auf die gewünschte Größe für den Einsatz in der jeweiligen Anwendung eingestellt werden.

In einer besonders bevorzugten Ausführungsform der Partikel, insbesondere der erfindungsgemäßen Titandioxid-Partikel, wird ein zuvor hydrothermal behandelter Grundpartikel eingesetzt.

Als Hydrothermalbehandlung wird das Erhitzen einer wässrigen Lösung, bzw. Suspension oder Dispersion in einem geschlossenen Behälter, gegebenenfalls unter Druck, bezeichnet (vgl. auch Ullmanns Enzyklopädie der Technischen Chemie, 4. Auflage, 1978, Band 15, S.117 ff: K.Recker, Einkristallzüchtung).

Die Hydrothermalbehandlung der Grundpartikel wird vorzugsweise bei Temperaturen im Bereich von 40 bis 360°C, vorzugsweise im Bereich von 80 bis 220°C und insbesondere bevorzugt im Bereich von 140 bis 200°C durchgeführt.

Durch die Hydrothermalbehandlung kommt es zur Ausbildung stabiler Nano-Kristallite mit gleichmäßiger Größe und Form. Bei niedrigen Temperaturen entstehen "nadelförmige" Kristallite. Mit zunehmenderTemperatur verrunden die Kristallite. Es bilden sich ovale Fomen, die bis zu runden Partikeln bei sehr hohen Temperaturen gehen. Zusätzlich kommt es zu einem gleichmäßigem Kristallwachstum.

Vorteile der Hydrothermalbehandlung gegenüber einer üblichen thermischen Behandlung (Temperaturbehandlung eines getrockneten Pulvers) sind :

- Ausbildung gleichmäßiger Kristallitgrößen mit enger Kornverteilung

- Verhinderung von Sintereffekten (Bildung von unerwünschten Aggregaten).

Titandioxid kann dabei in Form von Rutil oder Anatas oder in amorpher Form, vorzugsweise aber in Form von Rutil und/oder Anatas, vorliegen. Bevorzugte Primärpartikelgröße der Titandioxid-Grundpartikel liegt im Bereich vom 5 bis 50 nm. Dabei sind diese Primärpartikel insbesondere bei Anatas vorzugsweise rund, während Rutil-Primärpartikel häufig in Nadel-oder Spindelform bis hin zu Ovalen ("eiförmig") auftreten. Es können erfindungsgemäß jedoch auch runde Rutil-Primärpartikel eingesetzt werden.
Titandioxid-Grundpartikel können nach herkömmlichen Methoden, die dem Fachmann geläufig sind, vorzugsweise nasschemisch hergestellt werden.

Grundpartikel der Metalle Silizium, Zink, Aluminium, Cer, Eisen, Yttrium, Mangan oder Zirkonium können nach herkömmlichen Methoden, die dem Fachmann geläufig sind, vorzugsweise nasschemisch hergestellt werden. Viele Grundpartikel sind kommerziell erhältlich.

Zur Reduzierung der photokatalytischen Aktivität, sowie zur Reduzierung der Vergrauung von kosmetischen Formulierungen oder zur Verstärkung der antioxidativen Eigenschaften kann der Titandioxid-Grundpartikel mit geeigneten Elementen, beispielsweise Eisen oder Mangan dotiert sein. Besonders bevorzugt wird ein mit Eisen dotierter Titandioxid-Grundpartikel verwendet.

Die organische Phosphorverbindung wird für die erfindungsgemäße Verwendung ausgewählt aus der Gruppe der Hydroxyalkyldiphosphonsäuren oder deren Salze, Alkylphosphonsäuren, die durch mindestens eine COOH-Gruppe substituiert sein können, oder deren Salze, Aminoalkylenphosphonsäuren oder deren Salze oder organische Phosphorsäureester oder deren Salze.

Bevorzugt werden die entsprechenden Phosphonsäuren oder die organischen Phosphorsäureester verwendet. Hydroxyalkyldiphosphonsäuren sind beispielsweise Verbindungen der Formel I, wobei R eine lineare oder verzweigte Alkylgruppe mit 1 bis 20 C-Atomen bedeutet.

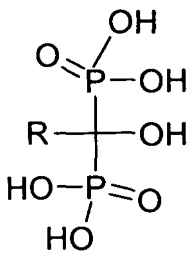

I

Die $C_1$-$C_{20}$-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl, Dodecyl, Tridecyl, Tetradecyl, Pentadecyl, Hexadecyl, Heptadecyl, Octadecyl, Nonadecyl oder Eicosyl.

Bevorzugte Verbindungen der Formel I sind 1-Hydroxyethan-1,1-diphosphonsäure oder 1-Hydroxydodecan-1,1-diphosphonsäure. 1-Hydroxyethan-1,1-diphosphonsäure ist käuflich erwerbbar unter dem Namen Cublen® K 60 der Firma Zschimmer & Schwarz. 1-Hydroxydodecan-1,1-diphosphonsäure ist beispielsweise in dem Handelsprodukt Tensan AO der Firma Polygon Chemie enthalten (75%).

Bevorzugte Salze der Hydroxyalkyldiphosphonsäuren, sogenannte Hydroxyalkyldiphosphonate, sind Alkali- oder Erdalkalisalze der Verbindungen der Formel I, wie zuvor beschrieben, insbesondere Natrium-oder Kaliumsalze.

Alkylphosphonsäuren, die durch mindestens eine COOH-Gruppe substituiert sein können, sind beispielsweise Alkylphosphonsäuren mit einer Alkylgruppe mit einer linearen oder verzweigten Alkylgruppe mit 1 bis 20 C-Atomen, die gegebenenfalls mit mindestens einer COOH-Gruppe ersetzt sein können. Bevorzugte Alkylphosphonsäuren sind beispielsweise Laurylphosphonsäure oder Octylphosphonsäure, die von der Firma Rhodia kommerziell angeboten werden. Eine bevorzugte Alkylphosphonsäure mit mindestens einer COOH-Gruppe ist beispielweise 2-Phosphonobutan-1,2,4-tricarbonsäure, die von der Firma Zschimmer & Schwarz unter dem Markennamen Cublen® P 50 angeboten wird.

Bevorzugte Salze der Alkylphosphonsäuren, wie zuvor beschrieben, sind Alkali- oder Erdalkalisalze, insbesondere Natrium- oder Kaliumsalze.

Aminoalkylenphosphonsäuren sind beispielsweise Verbindungen der Formel II,

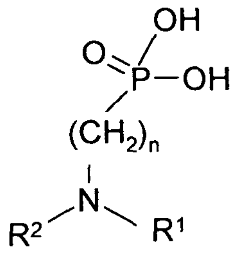

II

,

wobei n 1, 2, 3 oder 4 bedeutet, vorzugsweise 1 oder 2 und $R^1$ und $R^2$ stehen jeweils unabhängig voneinander für $-(CH_2)_{n1}-P(O)(OH)_2$ oder $-(CH_2)_{n1}-N\{[(CH_2)_{n2}-P(O)(OH)_2][(CH_2)_{n3}-P(O)(OH)_2]\}$, wobei n1, n2 oder n3 jeweils unabhängig 1, 2, 3 oder 4 bedeuten, vorzugsweise 1 oder 2 bedeuten.

Bevorzugte Verbindungen der Formel II sind

Aminotrimethylenphosphonsäure,

Diethylentriaminpenta(methylenphosphonsäure) oder Ethylendiamintetra(methylenphosphonsäure).

Aminotrimethylenphosphonsäure ist beispielsweise kommerziell erhältlich unter dem Markennamen Cublen® AP 5.

Bevorzugte Salze der Aminoalkylenphosphonsäuren sind Alkali- oder Erdalkalisalze der Verbindungen der Formel II, wie zuvor beschrieben, insbesondere Natrium- oder Kaliumsalze.

Organische Phosphorsäureester sind beispielsweise Phosphorsäuremonoalkylester mit einer linearen oder verzweigten Alkylgruppe mit 1 bis 20 C-Atomen, Phosphorsäuredialkylester mit linearen oder verzweigten Alkylrgruppen mit 1 bis 20 C-Atomen, die jeweils unabhängig voneinander ausgewählt werden, Phosphorsäuretrialkylester mit linearen oder verzweigten Alkylgruppen mit 1 bis 20 C-Atomen, die jeweils unabhängig voneinander ausgewählt werden, Salze der Phosphorsäuremonoalkylester oder Phosphorsäuredialkylester oder Gemische der genannten Phosphorsäureester, Phosphorsäure-Oxiester, Phosphorsäuremischester oder Nucleotide. Es können auch Gemische von Phosphorsäuremonoalkylestern und Phosphorsäuredialkylestern verwendet werden.

Phosphorsäuremonoalkylester sind beispielsweise Phosphorsäuremonomethylester, Phosphorsäuremonoethylester, Phosphorsäuremonooctylester, Phosphorsäuremonocetylester, Phosphorsäuremonostearylester oder Phosphorsäuremonododecylester. Phosphorsäuremonodecylester ist beispielsweise kommerziell erhältlich unter dem Namen Hostaphat® CC100 der Firma Clariant. Ein Gemisch aus Phosphorsäure-mono- und di-stearylester ist kommerziell erhältlich unter dem Namen Hostaphat® CS120 oder Amphisol® A der Firma DSM.

Die Alkylgruppen der Di- oder Trialkylester der Phosphorsäure können gleich oder verschieden sein. Bevorzugt sind sie gleich. Ausgewählte Di-oder Trialkylester sind beispielsweise Phosphorsäuredimethylester, Phosphorsäuretrimethylester, Phosphorsäurediethylester, Phosphorsäuretriethylester, Phosphorsäuredioctylester, Phosphorsäuretrioctylester, Phosphorsäuredicetylester, Phosphorsäuredistearylester oder Phosphorsäuredidodecylester.

Bevorzugte Salze der Phosphorsäuremonoalkylester oder Phosphorsäuredialkylester sind Alkali- oder Erdalkalisalze, insbesondere Natrium- oder Kaliumsalze. Kaliumcetylphosphat wird beispielsweise unter dem Handelsnamen Amphisol® K der Firma DSM angeboten.

Phosporsäure-Oxiester sind beispielsweise Phosphorsäureascorbylester oder Alkyltetraglycolether-Phosphorsäureester.

Ein Phosphorsäuremischester ist beispielsweise (3-Aminopropyl)-tocopheryl-phosphorsäureester.

Einsetzbare Nucleotide sind beispielsweise Adenosin-5-Monophosphat (AMP), Adenosin-3,5-cyclophosphat (c-AMP), Adenosindiphosphat (ADP) oder Adenosintriphosphat (ATP).

Besonders bevorzugt werden Phosphorsäuremonoalkylester eingesetzt, wie zuvor beschrieben. Die Verwendung von Phosphorsäuremonocetylester ist insbesondere bevorzugt.

Ein Verfahren zur Herstellung der erfindungsgemäß zu verwendenden Partikel ist beispielsweise dadurch gekennzeichnet, dass eine rührfähige Dispersion eines Metalloxid-Grundpartikels und/oder eines Metallhydroxid-Grundkörpers erhitzt wird, die organische Phosphorverbindung zugegeben wird und nach vollständiger Nachbehandlung gegebenenfalls gewaschen und getrocknet wird. Gegebenenfalls wird der Grundpartikel zuvor hydrothermal behandelt und gegebenenfalls nachbehandelt, bevor die Umsetzung mit der organischen Phosphorverbindung stattfindet.

Die optionalen Waschschritte sind vorteilhaft, wenn anfallende Salzfrachten entfernt werden müssen. Ob und in welchem Umfang solche Waschschritte durchgeführt werden müssen, liegt in der Fachkenntnis des Fachmanns.

Ein Verfahren zur Herstellung der erfindungsgemäßen Titandioxid-Partikel ist beispielsweise dadurch gekennzeichnet, dass eine rührfähige Dispersion eines Titandioxid-Grundpartikels erhitzt wird, die organische Phosphorverbindung gemäß Anspruch 2 zugegeben wird und nach vollständiger Nachbehandlung gegebenenfalls gewaschen und getrocknet wird. Gegebenenfalls wird der Titandioxid-Grundpartikel zuvor hydrothermal behandelt und mit Siliziumhydroxid- oder -oxid/dioxid nachbehandelt, bevor die Umsetzung mit der organischen Phosphorverbindung stattfindet.

Ein bevorzugtes Verfahren zur Herstellung bevorzugter Titandioxid-Partikel gemäß der Erfindung ist dadurch

gekennzeichnet, dass

a) Titandioxid-Partikel, d.h. sogenannte Titandioxid-Grundpartikel, hydrothermal behandelt werden,
b) anschließend eine Siliciumdioxid-Beschichtung derart aufgebracht wird, dass der Gehalt $SiO_2$ in der Beschichtung den zuvor beschriebenen Werten entspricht, insbesondere bevorzugt den Werten zwischen 25 bis 45 Gewichtsprozent bezogen auf den $TiO_2$-Gehalt der zu beschichtenden Partikel entspricht,
c) die rührfähige Dispersion aus b) erhitzt wird, die organische Phosphorverbindung gemäß Anspruch 2 zugegeben wird, optional gewaschen wird und
d)) die Suspension getrocknet wird.

Die Siliciumdioxid-Beschichtung in Schritt b) kann vorzugsweise als Sol-Gel-Prozess durchgeführt werden, wobei insbesondere bevorzugt eine Wasserglaslösung zu einer Suspension des Titandioxids gegeben wird.
Dabei wird der Sol-Gel-Prozeß in einer vorteilhaften Variante bei konstant gehaltenem pH-Wert durchgeführt. Der konstant gehaltene pH-Wert kann in einem Bereich von pH 2 bis pH 11 liegen, wobei der pH-Wert vorzugsweise im Bereich von pH = 5 bis pH = 8, insbesonders bevorzugt im Bereich von pH = 6 bis pH = 7 liegt.
Eine weitere vorteilhafte Variante ist die Gesamtzugabe des zur Nachbehandlung notwendigen Wasserglases bei einem pH = 7 bis pH = 11 ohne pH-Konstanthaltung. Anschließend wird der pH-Wert auf einen Wert von pH = 5 bis pH = 8, vorzugsweise auf pH = 6 bis pH = 7 abgesenkt.
Weiter ist es bevorzugt, wenn Schritt b) bei erhöhter Temperatur, vorzugsweise bei einer Temperatur im Bereich von 50°C bis 110°C durchgeführt wird.
Bei allen genannten Varianten des angegebenen Verfahrens ist eine Reifezeit nach beendeter Beschichtung vorteilhaft. Die Reifezeit sollte zwischen 1 h und 8 h, bevorzugt 2 h bis 4 h betragen und bei einer Temperatur von 50°C bis 110°C durchgeführt werden.
Die aus c) erhaltene Suspension kann direkt zur Herstellung von Zubereitungen, enthaltend die erfindungsgemäßen Titandioxid-Partikel verwendet werden. Die Suspension kann jedoch auch getrocknet werden.
Weiter kann es im Hinblick auf die spätere Verarbeitung von Vorteil sein, wenn das Produkt nachträglich vermahlen wird. Hier können die üblichen bei Nanopartikeln verwendbaren Mahltechniken eingesetzt werden.
Das erfindungsgemäße nanopartikuläre Titandioxid besitzt ebenfalls die vorteilhaften Eigenschaften der Vergleichsprodukte aus dem Stand der Technik hinsichtlich:

- UV-Absorption, insbesondere Breitband- bzw. UV-B-Absorption,
- Transparenz im sichtbaren Licht (VIS),
- gute, insbesondere erhöhte Photostabilität,
- verringerte bzw. verhinderte Photoaktivität,
- Silica-Oberfläche, die ggf. leicht, mit bekannten Techniken hydrophob modifiziert werden kann,
- leichte Dispergierbarkeit in wässrigen und öligen Phasen,
- in Kombinaton mit Dibenzoylmethan-Derivaten, insbesondere:
    - ◦ verringerte Verfärbung der Formulierung und/oder
    - ◦ nachlassende Verfärbung der Formulierung während der Lagerung und/oder
    - ◦ keine oder reduzierte Auskristallisation von Komplexen der Dibenzoylmethan-Derivate und/oder
    - ◦ erhöhte Lagerstabilität der Dibenzoylmethan-Derivate und/oder
    - ◦ verbesserte Lichtschutzwirkung, insbesondere nach Lagerung,
- in Kombination mit reaktiven instabilen Bestandteilen z.B. Benzophenon-Derivaten oder Ascorbinsäurederivaten, insbesondere 2-Hydroxy-4-methoxy-Benzophenon oder Ascorbylpalmitat, wird eine Stabilisierung der Benzophenon-Derivate beobachtet.

Die erfindungsgemäß zu verwendenden Metalloxid-Partikel und/oder Metallhydroxid-Partikel gemäß Anspruch 1, insbesondere das erfindungsgemäße nanopartikuläre Titandioxid nach Anspruch 2, zeigen/zeigt in Kombination mit Dihydoxyaceton einen deutlich verringerten Abbau des DHA nach Lagerung im Vergleich zu konventionellen, bisher bekannten, Titandioxid-UV-Schutzmitteln, d.h. es wird eine gegenüber dem Stand der Technik verminderte signifikante Destabilisierung des Selbstbräuners beobachtet. Das erfindungsgemäße Titandioxid wird im Sinne dieser Erfindung daher als DHA-kompatibel bezeichnet. Weiterhin hat sich gezeigt, dass in Kombination mit DHA keine oder nur eine minimale Verfärbung der Zubereitung nach Herstellung sowie nach 3 Monaten Lagerung bei 40°C in Dunkelheit auftritt.
Dabei hat es sich insbesondere gezeigt, dass es zur gleichzeitigen Verwirklichung der oben genannten Vorteile vorteilhaft sein kann, wenn das nanopartikuläre Titandioxid mit Eisen dotiert ist.
Weitere bevorzugte Kombinationen von Ausführungsformen sind in den Ansprüchen offenbart.

Aufgrund der oben genannten Vorteile ist ein weiterer Gegenstand der vorliegenden Erfindung eine Zubereitung enthaltend die erfindungsgemäßen Titandioxid-Partikel, wie zuvor beschrieben.

Im Sinne der vorliegenden Erfindung wird neben dem Begriff Zubereitung gleichbedeutend auch der Begriff Mittel oder Formulierung verwendet.

Alle Verbindungen oder Komponenten, die in den Zubereitungen verwendet werden können, sind entweder bekannt und käuflich erwerbbar oder können nach bekannten Verfahren synthetisiert werden.

Bei den Zubereitungen handelt es sich in einer Erfindungsvariante um wässrige oder ölige Dispersionen. Bevorzugte Öle sind beispielsweise natürliche Öle, wie Sonnenblumenöl; oder die für den Einsatz in der Kosmetik bekannten Öle wie Isononylisononanoat; Isopropylpalmitat, Propylheptylcaprylat; Dicaprylylcarbonat, Butylenglycol Dicaprylat/Dicaprat, Isopropyllauroyl-Sarcosinat, C12-15 Alkyl Benzoat (beispielsweise Tegosoft TN der Evonik), Capric/Caprylic-Triglycerid, Cyclopentasiloxane oder Cyclohexasiloxane.

Bei den Zubereitungen handelt es sich in einer Erfindungsvariante vorzugsweise um topisch anwendbare Zubereitungen, beispielsweise kosmetische oder dermatologische Formulierungen. Die Zubereitungen enthalten in diesem Fall einen kosmetisch oder dermatologisch geeigneten Träger und je nach gewünschtem Eigenschaftsprofil optional weitere geeignete Inhaltsstoffe.

Topisch anwendbar bedeutet im Sinne der Erfindung, dass die Zubereitung äußerlich und örtlich angewendet wird, d.h. dass die Zubereitung geeignet sein muss, um beispielsweise auf die Haut aufgetragen werden zu können.

Weitere bevorzugte Zubereitungen sind aus der Gruppe Fasern, Textilien, einschließlich deren Beschichtungen, Anstrichstoffe, Beschichtungssysteme, Folien und Verpackungen für den Schutz von Lebensmittel, Pflanzen oder technischen Gütern ausgewählt.

Verwendet werden kann das erfindungsgemäße nanopartikuläre Titandioxid, wie zuvor beschrieben, zur Einarbeitung in Anstrichstoffe, Beschichtungssysteme, Folien, Verpackungen, Fasern, Textilien und Formteile aus Kautschuk oder Silikonkautschuk, wie Reifen oder Isolatoren.

Besonders bevorzugte Zubereitungen sind die wässrige oder ölige Dispersion sowie topisch anwendbare Zubereitungen, insbesondere kosmetische Zubereitungen.

Neben den bereits oben genannten Vorteilen kann der Einsatz der erfindungsgemäßen Partikel in Zubereitungen, die Emulsionen sind, insbesondere auch zur Stabilisierung der Emulsion beitragen. Dadurch kann in der Regel der Einsatz von Emulgatoren verringert werden oder im Einzelfall (Pickering-Emulsion), sogar ganz auf den Einsatz von Emulgatoren verzichtet werden. Erfindungsgemäß bevorzugt sind daher auch emulgatorfreie Emulsionen, welche das erfindungsgemäße nanopartikuläre Titandioxid enthalten.

Die erfindungsgemäßen Partikel können in den erfindungsgemäßen Zubereitungen in Mengenanteilen vorliegen, die im allgemeinen im Bereich von 0,1 Gew.-% bis 50 Gew.-% liegen und vorzugsweise in Mengenanteilen, die im Bereich von 0,5 Gew.-% bis 20 Gew.-% liegen, wobei diese Mengenanteile auf das Gesamtgewicht der Zubereitung bezogen sind.

Bevorzugte Zubereitungen, insbesondere kosmetische Zubereitungen, enthalten Dihydroxyaceton oder ein Dihydroxyacetonderivat, ganz besonders bevorzugt Dihydroxyaceton.

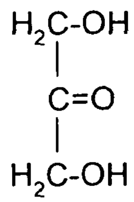

1,3-Dihydroxyaceton (DHA)

Die Konzentration an DHA in der Zubereitung liegt im Bereich von 1 bis 12 Gewichtsprozent, bevorzugt bei 1,5 Gewichstsprozent bis 7,5 Gewichtsprozent, besonders bevorzugt bei 2 Gew.-% bis 5 Gew.-%, bezogen auf die Zubereitung.

Diese Formulierungen sind dadurch gekennzeichnet, dass der Abbau des DHA nach 3 Monaten Lagerung bei 40°C in Dunkelheit bei unterschiedlichen Einsatzverhältnissen des erfindungsgemäßen Titandioxid-Partikels, zu DHA in Gewichtsprozent nicht größer ist als maximal 20%, bevorzugt nicht größer ist als 10% im Bezug auf den DHA-Abbau in der gleichen Formulierung ohne Zusatz von von Titandioxid-Partikeln. Hierzu wird auf den experimentellen Teil verwiesen. Die Untersuchungsergebnisse belegen, dass der DHA-Abbau nach Lagerung von 3 Monaten bei 40°C in Dunkelheit für die erfindungsgemäßen Titandioxid-Partikel immer geringer ist, als der Abbau bei Titandioxid-Partikeln mit anderen Beschichtungen ohne der erfindungsgemäßen Beschichtung. Dort werden auch Daten angegeben, die den absoluten DHA-Abbau beschreiben, bezogen auf die DHA-Einsatzkonzentration.

In einer weiteren Ausführungsform der vorliegenden Erfindung kann die erfindungsgemäße Zubereitung neben Dihydroxyaceton mindestens einen weiteren Selbstbräuner enthalten.

Als vorteilhafte Selbstbräuner können unter anderem eingesetzt werden:

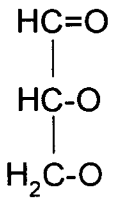

Glycerolaldehyd

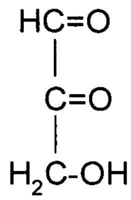

Hydroxymethylglyoxal

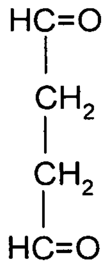

γ-Dialdehyd

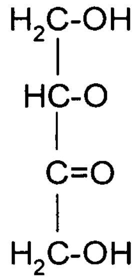

Erythrulose

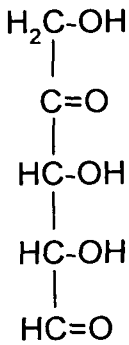

6-Aldo-D-Fructose

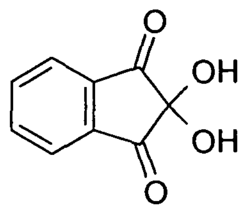

Ninhydrin

Ferner ist das 5-Hydroxy-1,4-naphtochinon (Juglon) zu nennen, das aus den Schalen frischer Walnüsse extrahiert wird

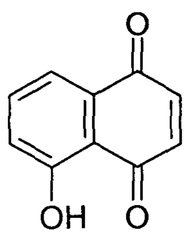

5-Hydroxy-1,4-naphtochinon (Juglon)

sowie das in den Henna-Blättern vorkommende 2-Hydroxy-1,4-naphtochinon (Lawson).

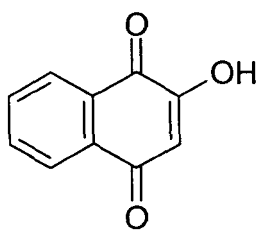

2-Hydroxy-1,4-naphtochinon (Lawson)

Die erfindungsgemäßen Zubereitungen, die Dihydroxyaceton enthalten, neigen bei der Anwendung auf der menschlichen Haut zu Fehlgerüchen, die vermutlich durch Abbauprodukte des Dihydroxyacetons selbst oder durch Produkte von Nebenreaktionen verursacht werden und die von den Anwendern teilweise als unangenehm empfunden werden. Es hat sich gezeigt, dass diese Fehlgerüche bei Verwendung von Formaldehydfängern und/oder Flavonoiden vermieden werden. Auch bei kombinierter Verwendung mit weiteren Selbstbräunern, wie zuvor beschrieben, ist die Entstehung von Fehlgerüchen nicht auszuschließen. Daher kann die erfindungsgemäße Zubereitung vorzugsweise auch Formaldehydfänger sowie gegebenenfalls Flavonoide zur Verbesserung des Geruches enthalten.

Vorzugsweise wird der Formaldehydfänger ausgewählt aus der Gruppe Alkalimetall-, Erdalkalimetall- oder Ammoniumbisulfit. Besonders bevorzugt ist eine Zubereitung, die in Kombination DHA Plus, eine Mischung aus DHA, Natriumbisulfit und Magnesiumstearat, enthält.

DHA Plus ist eine Produktmischung, welche zur Maskierung, Eliminierung oder Neutralisierung des Formaldehyds Natriumbisulfit, gleichbedeutend mit $Na_2S_2O_5$ oder INCI: sodium disulfite, enthält. Der Zusatz von Natriumbisulfit in fertigen Formulierungen führt zur signifikanten Reduktion oder Unterbindung des unangenehmen Geruchs. DHA Plus wird von der Firma Merck, Darmstadt vertrieben.

Das gegebenenfalls in der erfindungsgemäßen Zubereitung vorhandene Flavonoid wirkt dabei zusätzlich als Stabilisator für den Selbstbräuner bzw. die Selbstsbräunungssubstanzen und/oder reduziert oder vermeidet oder verbessert lagerabhängige Fehlgerüche, die auch durch enthaltene Zusatz-oder Hilfsstoffe entstehen können.

Vorzugsweise handelt es sich um ein Flavonoid, bei dem eine oder mehrere phenolische Hydroxygruppen durch Veretherung oder Veresterung blockiert sind. Beispielsweise haben sich Hydroxyethyl-substituierte Flavonoide, wie vorzugsweise Troxerutin, Troxequercetin, Troxeisoquercetin oder Troxeluteolin, und Flavonoid-sulfate oder Flavonoid-phosphate, wie vorzugsweise Rutinsulfate, dabei als besonderes gut geeignete Flavonoide erwiesen. Besonders bevorzugt im Sinne der erfindungsgemäßen Verwendung sind Rutinsulfat und Troxerutin. Ganz besonders bevorzugt ist die Verwendung von Troxerutin.

Die bevorzugten Flavonoide verfügen über einen nicht positiv geladenen Flavangrundkörper. Es wird vermutet, dass durch diese Flavonoide Metallionen wie z.B. $Fe^{2+}/Cu^{2+}$ komplexiert werden und so Autooxidations-vorgänge bei Duftstoffen oder Verbindungen, deren Abbau zu Fehlgerüchen führt, verhindert bzw. vermindert werden.

Besonders bevorzugt ist eine Zubereitung, die DHA Rapid und/oder Natriumbisulfit enthalten. DHA Rapid ist eine Produktmischung enthaltend Dihydroxyaceton und Troxerutin, der Firma Merck, Darmstadt.

Entsprechende Vormischungen und Zubereitungen, die Formaldehydfänger sowie gegebenenfalls Flavonoide zur Verbesserung des Geruches auf der Haut enthalten, sind in der Deutschen Patentanmeldung mit dem ktenzeichen DE 10 2007 013 368 beschrieben.

Des Weiteren können die erfindungsgemäßen Partikel auch mit folgenden Substanzen alleine oder in Kombination mit DHA zur Bräunungsintensivierung eingesetzt werden:

- Vegetan Premium (INCI Dihydroxyacetone / Melanin) von Soliance,
- MelanoBronze (INCI: Vitex agnus castus extract (and) acetyl tyrosine (and) glycerin (and) alcohol (and) water (aqua)) Mibelle AG Biochemistry.
- Instabronze® (INCI Dihydroxyaceton (DHA), N-acétyl tyrosine), Alban-Müller,
- Substanzen welche penetrationsfördernd wirken, wie z.B. Leerliposome, Propylenglykol, Isohexadecan (Arlamol HD; Croda), Dimethylisosorbid (Arlasolve DMI; CRODA), Trimethylpentanediol/Adipic Acid/Isononanoic Acid Copolymer (INCI-Bezeichnung) oder (Lexorez TC-8, Inolex).

Die Verwendung des erfindungsgemäßen nanopartikulären Titandioxids, zur Stabilisierung von Dihydroxyaceton oder Dihydroxyacetonderivaten, insbesondere von Dihydroxyaceton, ist ein weiterer Gegenstand der vorliegenden Erfindung.

Ferner sind auch Kombinationen der erfindungsgemäßen erfindungsgemäßen Titandioxid-Partikel, mit weiteren partikulären UV-Filtern, sowohl als Pulver als auch als Dispersion oder Paste, der folgenden Typen möglich.

Hierbei sind sowohl solche aus der Gruppe der Titandioxide wie z.B. gecoatetes Titandioxid (z.B. Eusolex® T-2000, Eusolex®T-AQUA, Eusolex®T-AVO, Eusolex®T-OLEO), Zinkoxide (z.B. Sachtotec®), Eisenoxide oder auch Ceroxide und/oder Zirkonoxide bevorzugt.

Ferner sind auch Kombinationen mit pigmentärem Titandixoxid oder Zinkoxid möglich, wobei die Partikelgröße dieser Pigmente größer oder gleich 200 nm sind, beispielsweise Hombitan® FG oder Hombitan® FF-Pharma.

Weiter kann es möglich sein, wenn die Zubereitungen anorganische UV-Filter enthalten, die mit üblichen Methoden, wie beispielsweise in Cosmetics & Toiletries, February 1990, Vol. 105, pp. 53-64 beschrieben, nachbehandelt wurden. Hierbei können eine oder mehrere der folgenden Nachbehandlungskomponenten gewählt sein: Amino Säuren, Bienenwachs, Fettsäuren, Fettsäurealkohole, anionische Tenside, Lecithin, Phospholipide, Natrium-, Kalium-, Zink-, Eisen- oder Aluminiumsalze von Fettsäuren, Polyethylene, Silikone, Proteine (besonders Collagen oder Elastin), Alkanolamine, Siliciumdioxid, Aluminiumoxid, weitere Metalloxide, Phosphate, wie Natriumhexametaphosphat oder Glycerin.

Einsetzbare weitere partikuläre UV-Filter sind dabei:

- unbehandelte Titandioxide wie z.B. die Produkte Microtitanium Dioxide MT 500 B der Fa. Tayca; Titandioxd P25 der Fa. Degussa,
- Nachbehandelte mikronisierte Titandioxide mit Aluminiumoxid und Siliciumdioxid Nachbahandlung wie z.B. das Produkt "Microtitanium Dioxide MT 100 SA der Tayca; oder das Produkt "Tioveil Fin" der Fa. Uniqema,
- Nachbehandelte mikronisierte Titandioxide mit Aluminiumoxid und/oder Aluminiumstearate/laurate Nachbehandlung wie z.B. Microtitanium Dioxide MT 100 T der Fa. Tayca, Eusolex T-2000 der Firma Merck,
- Nachbehandelte mikronisierte Titandioxide mit Eisenoxid und/oder Eisenstearate Nachbehandlung wie z.B. das Produkt "Microtitanium Dioxide MT 100 F" der Fa. Tayca,
- Nachbehandelte mikronisierte Titandioxide mit Siliciumdioxide, Aluminiumoxid und Silicon Nachbehandlung wie z.B. das Produkt "Microtitanium Dioxide MT 100 SAS",der Fa. Tayca,
- Nachbehandelte mikronisierte Titandioxide mit Natrumhexametaphosphate, wie z.B. das Produkt "Microtitanium Dioxide MT 150 W" der Fa. Tayca.

Die zur Kombination eingesetzten behandelten mikronisierten Titandioxide können auch nachbehandelt sein mit:

- Octyltrimethoxysilane; wie z.B. das Produkt Tego Sun T 805 der Fa. Degussa,
- Siliciumdioxid; wie z.B. das Produkt Parsol T-X der Fa. DSM,
- Aluminiumoxid und Stearinsäure; wie z.B. das Produkt UV-Titan M160 der Fa. Sachtleben,
- Aluminium und Glycerin; wie z.B. das Produkt UV-Titan der Fa. Sachtleben,
- Aluminium und Silikonölen, wie z.B. das Produkt UV-Titan M262 der Fa. Sachtleben,
- Natriumhexamethaphosphat und Polyvinylpyrrolidon,
- Polydimethylsiloxane, wie z.B. das Produkt 70250 Cardre UF TiO2SI3" der Fa. Cardre,
- Polydimethylhydrogensiloxane, wie z.B. das Produkt Microtitanium Dioxide USP Grade Hydrophobic" der Fa. Color Techniques,
- Polymeren wie beispielsweise Polyvinylpyrrolidon (PVP) oder Polyethylen (PE).

Ferner kann auch die Kombination mit folgenden Produkten vorteilhaft sein:

- Unbehandelte Zinkoxide wie z. B. das Produkt Z-Cote der Fa. BASF (Sunsmart), Nanox der Fa. Elementis
- Nachbehandelte Zinkoxide wie z.B die folgenden Produkte:
    - "Zinc Oxide CS-5" der Fa. Toshibi (ZnO nachbehandelt mit polymethylhydrogenosiloxane)
    - Nanogard Zinc Oxide FN der Fa. Nanophase Technologies
    - "SPD-Z1" der Fa Shin-Etsu (ZnO nachbehandelt mit einem Silikongepfropften Acrylpolymer, dispergiert in Cyclodimethylsiloxane
    - "Escalol Z100" der Fa ISP (Aluminiumoxid nachbehandeltes ZnO dispergiert in einer ethylhexyl methoxycinnamate/PVPhexadecene/methicone copolymer Mischung)

◦ "Fuji ZNO-SMS-10" der Fa. Fuji Pigment (ZnO nachbehandelt mit Siliciumdioxid und Polymethylsilesquioxan);

◦ Unbehandeltes Ceroxide Mikropigment z.B. mit der Bezeichnung "Colloidal Cerium Oxide" der Fa Rhone Poulenc

◦ Unbehandelte und/oder nachbehandelte Eisenoxide mit der Bezeichnung Nanogar der Fa. Arnaud.

Beispielhaft können auch Mischungen verschiedener Metalloxide , wie z.B. Titandioxid und Ceroxid mit und ohne Nachbenhandlung eingesetzt werden, wie z.B. das Produkt Sunveil A der Fa. Ikeda. Außerdem können auch Mischungen von Aluminiumoxid, Siliciumdioxid und Silikonnachbehandelten Titandioxid, Zinkoxid-Mischungen wie z.B. das Produkt UV-Titan M262 der Fa. Sachtleben in Kombination mit dem erfindungsgemäßen UV-Schutzmittel verwendet werden.

Diese anorganischen UV-Filter werden in der Regel in einer Menge von 0,1 Gewichtsprozent bis 25 Gewichtsprozent, vorzugsweise 2 Gew.-% - 10 Gew.-%, in kosmetische Zubereitungen eingearbeitet. Insbesondere kann es dabei bevorzugt sein, wenn in Emulsionen in einer Phase ein erfindungsgemäßes nanopartikuläres Titandioxid und in der anderen Phase ein weiterer anorganischer UV-Filter enthalten ist.

Bevorzugte Zubereitungen, insbesondere mit Lichtschutzeigenschaften, enthalten dabei mindestens einen weiteren UV-Filter, insbesondere ein Dibenzoylmethanderivat. Die im Rahmen der vorliegenden Erfindung verwendeten Dibenzoylmethanderivate sind an sich bereits wohlbekannte Produkte, die insbesondere in den oben genannten Druckschriften FR-A-2 326 405, FR-A-2 440 933 und EP-A-0 114 607 beschrieben sind.
Die erfindungsgemäß verwendbaren Dibenzoylmethanderivate können insbesondere unter den Dibenzoylmethanderivaten der folgenden Formel ausgewählt sein:

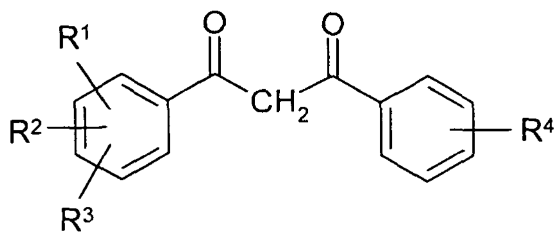

worin $R^1$, $R^2$, $R^3$ und $R^4$, die identisch oder voneinander verschieden sind, Wasserstoff, eine geradkettige oder verzweigte $C_{1-8}$-Alkylgruppe oder eine geradkettige oder verzweigte $C_{1-8}$-Alkoxygruppe bedeuten. Gemäß der vorliegenden Erfindung können selbstverständlich ein Dibenzoylmethanderivat oder mehrere Dibenzoylmethanderivate verwendet werden. Von den Dibenzoylmethanderivaten, auf die sich die vorliegende Erfindung spezieller bezieht, können insbesondere: 2-Methyldibenzoylmethan, 4-Methyldibenzoylmethan, 4-Isopropyldibenzoylmethan, 4-tert.-Butyldibenzoylmethan, 2,4-Dimethyldibenzoylmethan, 2,5-Dimethyldibenzoylmethan, 4,4'-Diisopropyldibenzoylmethan, 4,4'-Methoxy-tert.-butyldibenzoylmethan, 2-Methyl-5-isopropyl-4'-methoxydibenzoylmethan, 2-Methyl-5-tert.-butyl-4'-methoxydibenzoylmethan, 2,4-Dimethyl-4'-methoxydibenzoylmethan und 2,6-Dimethyl-4-tert. -butyl-4'-methoxydibenzoylmethan genannt werden, wobei diese Aufzählung nicht einschränkend ist.
Von den obengenannten Dibenzoylmethanderivaten wird erfindungsgemäß insbesondere das 4,4'-Methoxy-tert.-butyldibenzoylmethan und insbesondere das unter der Handelsbezeichnung Eusolex® 9020 von der Firma Merck im Handel befindliche 4,4'-Methoxy-tert.-butyldibenzoylmethan bevorzugt, wobei dieser Filter der folgenden Strukturformel entspricht:

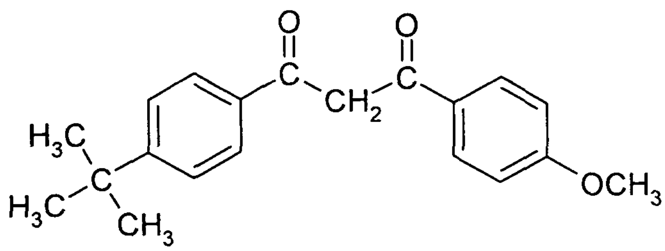

Ein weiteres bevorzugtes Dibenzoylmethanderivat ist das 4-Isopropyldibenzoylmethan.

Weitere bevorzugte Zubereitungen mit weiteren organischen UV-Filtern enthalten dabei mindestens ein Benzophenon oder Derivat des Benzophenon, wie insbesondere bevorzugt 2-Hydroxy-4-methoxybenzophenon (z.B. Eusolex® 4360) oder 2-Hydroxy-4-methoxybenzophenon-5-sulfonsäure und ihr Natriumsalz (z.B. Uvinul® MS-40).

Das oder die Dibenzoylmethanderivat(e) oder das oder die Benzophenonderivat(e) können in den erfindungsgemäßen Zubereitungen in Mengenanteilen vorliegen, die im Allgemeinen im Bereich von 0,1 Gew.-% bis 10 Gew.-% (Gewichtsprozent) liegen und vorzugsweise in Mengenanteilen, die im Bereich von 0,3 Gew.-% bis 5 Gew.-% liegen, wobei diese Mengenanteile auf das Gesamtgewicht der Zubereitung bezogen sind.

Die erfindungsgemäßen Zubereitungen können selbstverständlich einen oder mehrere zusätzliche(n) hydrophile(n) oder lipophile(n) Sonnenschutzfilter, die im UV-A-Bereich und/oder UV-B-Bereich und(/oder IR und/oder VIS-Bereich (Absorber) wirksam sind, enthalten. Diese zusätzlichen Filter können insbesondere unter Zimtsäurederivaten, Salicylsäurederivaten, Campherderivaten, Triazinderivaten, β,β-Diphenylacrylatderivaten, p-Aminobenzoesäurederivaten sowie polymeren Filtern und Siliconfiltern, die in der Anmeldung WO-93/04665 beschrieben sind, ausgewählt sein. Weitere Beispiele für organische Filter sind in der Patentanmeldung EP-A 0 487 404 angegeben. Im Folgenden werden die genannten UV-Filter meist nach der INCI-Nomenklatur benannt.

Insbesondere zu nennen sind hier:

- para-Aminobenzoesäure und deren Derivate: PABA, Ethyl PABA, Ethyl dihydroxypropyl PABA, Ethylhexyl dimethyl PABA, z. B. vetrieben unter dem Namen "Escalol 507" von der Fa. ISP, Glyceryl PABA, PEG-25 PABA, z. B. vetrieben unter dem Namen "Uvinul P25" von der Fa. BASF.

Salicylate: Homosalate vetrieben unter dem Namen "Eusolex HMS" von der Fa. Merck; Ethylhexyl salicylate, z. B. vetrieben unter dem Namen "Neo Heliopan OS" von der Fa. Haarmann and Reimer, Dipropylene glycol salicylate, z. B. vetrieben unter dem Namen "Dipsal" von der Fa. Scher, TEA salicylate, z. B. vetrieben unter dem Namen "Neo Heliopan TS" von der Fa. Haarmann and Reimer.

β,β-Diphenylacrylate Derivate: Octocrylene, z. B. vetrieben unter dem Namen "Eusolex OCR" von der Fa. Merck, "Uvinul N539" von der Fa. BASF, Etocrylene, z. B. vetrieben unter dem Namen "Uvinul N35" von der BASF.

Benzophenone Derivate: Benzophenone-1, z. B. vetrieben unter dem Namen "Uvinul 400"; Benzophenone-2, z. B. vetrieben unter dem Namen "Uvinul D50" ; Benzophenone-3 oder Oxybenzone, z. B. vetrieben unter dem Namen "Uvinul M40";Benzophenone-4, z. B. vetrieben unter dem Namen "Uvinul MS40" ; Benzophenone-9, z. B. vetrieben unter dem Namen "Uvinul DS-49" von der Fa. BASF, Benzophenone-5, Benzophenone-6, z. B. vetrieben unter dem Namen "Helisorb 11" von der Fa. Norquay, Benzophenone-8, z. B. vetrieben unter dem Namen "Spectra-Sorb UV-24" von der Fa. American Cyanamid, Benzophenone-12 n-hexyl 2-(4-diethylamino-2-hydroxybenzoyl) benzoate.

Benzylidencampher Derivate: 3-Benzylidenecamphor, z. B. vetrieben unter dem Namen "Mexoryl SD" von der Fa. Chimex, 4-Methylbenzylidenecamphor, z. B. vetrieben unter dem Namen "Eusolex 6300" von der Fa. Merck, Benzylidenecamphorsulfonsäure, z. B. vetrieben unter dem Namen "Mexoryl SL" von der Fa. Chimex, Camphor benzalkonium methosulfate, z. B. vetrieben unter dem Namen "Mexoryl SO" von der Fa. Chimex, Terephthalylidenedicamphorsulfonsäure, z. B. vetrieben unter dem Namen "Mexoryl SX" von der Fa Chimex, Polyacrylamidomethylbenzylidenecamphor vetrieben unter dem Namen "Mexoryl SW" von der Fa. Chimex.

Phenylbenzimidazol Derivate: Phenylbenzimidazolesulfonsäure, z. B. vetrieben unter dem Namen "Eusolex 232" von der Fa. Merck, Dinatrium phenyl dibenzimidazole tetrasulfonat, z. B. vetrieben unter dem Namen "Neo Heliopan AP" von der Fa. Haarmann and Reimer.

Phenylbenzotriazol Derivate: Drometrizole trisiloxane, z. B. vetrieben unter dem Namen "Silatrizole" von der Fa. Rhodia Chimie, Methylenebis(benzotriazolyl)tetramethylbutylphenol in fester Form, z. B. vetrieben unter dem Namen "MIXXIM BB/100" von der Fa. Fairmount Chemical, oder in mikronisierter Form als wässrige Dispersion, z. B. vetrieben unter dem Namen "Tinosorb M" von der Fa. Ciba Specialty Chemicals.

Triazine Derivate: Ethylhexyltriazone, z. B. vetrieben unter dem Namen "Uvinul T150" von der Fa. BASF, Diethylhexylbutamidotriazone, z. B. vetrieben unter dem Namen "Uvasorb HEB" von der Fa. Sigma 3V, 2,4,6-tris(diisobutyl 4'-aminobenzalmalonate)-s-triazine.

Anthranilin Derivate: Menthyl anthranilate, z. B. vetrieben unter dem Namen "Neo Heliopan MA" von der Fa. Haarmann and Reimer.

Imidazol Derivate: Ethylhexyldimethoxybenzylidenedioxoimidazoline propionat.

Benzalmalonat Derivate: Polyorganosiloxane enthaltend funktionelle benzalmalonate Gruppen, wie z.B. Polysilicone-15, z. B. vetrieben unter dem Namen "Parsol SLX" von der Hoffmann LaRoche.

4,4-Diarylbutadien Derivate: 1,1-Dicarboxy(2,2'-dimethylpropyl)-4,4-diphenylbutadiene.

Benzoxazole Derivate: 2,4-bis[5-(1-dimethylpropyl)benzoxazol-2-yl(4-phenyl) imino]-6-(2-ethylhexyl)imino-1,3,5-triazine, z. B. vetrieben unter dem Namen Uvasorb K2A von der Fa. Sigma 3V und Mischungen dieses enthaltend.

Piperazinderivate wie beispielsweise die Verbindung

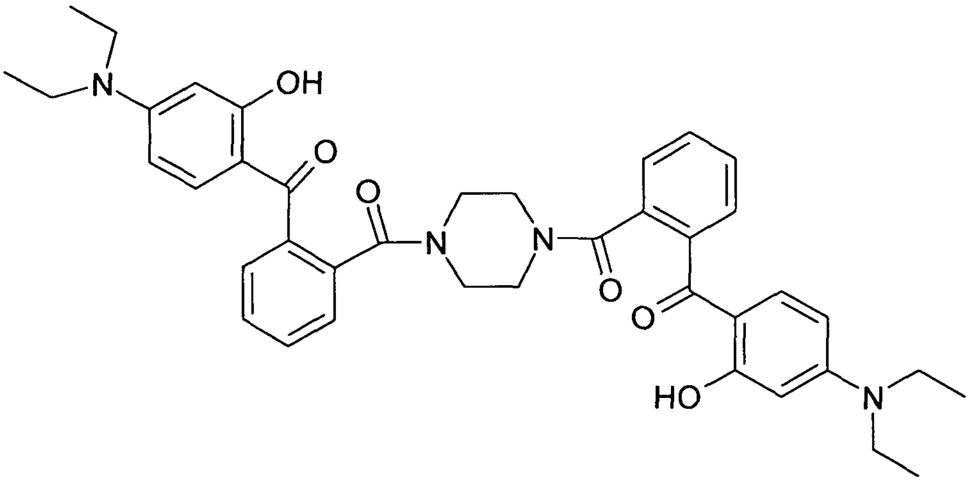

.

Die in der Liste aufgeführten Verbindungen sind nur als Beispiele aufzufassen. Selbstverständlich können auch andere UV-Filter verwendet werden. Insbesondere können vorteilhaft auch organische partikuläre UV-Filter, wie Sie beispielsweise in der Patentanmeldung WO 99/66896 beschrieben sind, mit den erfindunsgsgemäßen partikulären Titandioxid-Partikeln kombiniert werden.

Die zur Kombination mit dem erfindungsgemäßen UV-Schutzmittel geeigneten organischen UV-schützende Substanzen sind bevorzugt aus der folgenden Liste auszuwählen: Ethylhexyl salicylate, Octocrylene, Butylmethoxydibenzoylmethane, Phenylbenzimidazolesulfonic acid, Benzophenone-3, Benzophenone-4, Benzophenone-5, n-Hexyl 2-(4-diethylamino-2-hydroxybenzoyl)benzoate, 4-Methylbenzylidenecamphor, Terephthalylidenedicamphorsulfonic acid, Disodium phenyldibenzimidazoletetrasulfonate, Methylenebis(benzotriazolyl)tetramethylbutylphenol, Ethylhexyl Triazone, Diethylhexyl Butamido Triazone, Drometrizole trisiloxane, Polysilicone-15, 1,1-Dicarboxy(2,2'-dimethylpropyl)-4,4-diphenylbutadiene, 2,4-Bis[5-1 (dimethylpropyl)benzoxazol-2-yl(4-phenyl) imino]-6-(2-ethylhexyl)imino-1,3,5-triazine und Mischungen davon.

Diese organischen UV-Filter werden in der Regel in einer Menge von 0,01 Gewichtsprozent bis 20 Gewichtsprozent, vorzugsweise 1 Gew.-% - 10 Gew.-%, in Formulierungen eingearbeitet.

Organische UV-Filter werden insgesamt in der Regel in einer Menge von 0,01 Gewichtsprozent bis 20 Gewichtsprozent, vorzugsweise 0,5 Gew.-% bis 20 Gew.-%, in Formulierungen eingearbeitet.

Bevorzugte Zubereitungen können auch Verbindungen der Formel I enthalten,

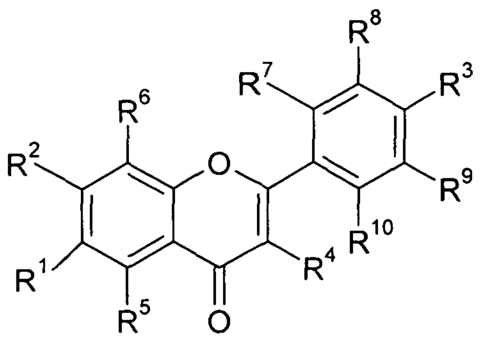

I,

wobei $R^1$ und $R^2$ ausgewählt sind aus

- H
- und $OR^{11}$, wobei $OR^{11}$ unabhängig voneinander steht für
  - OH
  - geradkettige oder verzweigte $C_1$- bis $C_{20}$-Alkyloxygruppen,
  - geradkettigen oder verzweigten $C_3$- bis $C_{20}$-Alkenyloxygruppen,
  - geradkettigen oder verzweigten $C_1$- bis $C_{20}$-Hydroxyalkoxygruppen, wobei die Hydroxygruppe(n) an ein primäre oder sekundäre Kohlenstoffatome der Kette gebunden sein können und weiter die Alkylkette auch durch Sauerstoff unterbrochen sein kann, und/oder

- $C_3$- bis $C_{10}$-Cycloalkyloxygruppen und/oder $C_3$- bis $C_{12}$-Cycloalkenyloxygruppen, wobei die Ringe jeweils auch durch $-(CH_2)_n$-Gruppen mit n = 1 bis 3 überbrückt sein können und/oder,
- Mono- und/oder Oligoglycosylreste,

mit der Maßgabe, dass mindestens ein Rest aus $R^1$ und $R^2$ steht für $OR^{11}$, und $R^3$ steht für einen Rest $OR^{11}$ und $R^4$ bis $R^7$ und $R^{10}$ gleich oder verschieden sein können, und unabhängig voneinander stehen für

- H
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei - die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können und
- $R^8$ und $R^9$ gleich oder verschieden sein können, und unabhängig voneinander stehen für
- H
- $OR^{11}$
- geradkettige oder verzweigte $C_1$- bis $C_{20}$-Alkylgruppen,
- geradkettige oder verzweigte $C_3$- bis $C_{20}$-Alkenylgruppen,
- geradkettige oder verzweigte $C_1$- bis $C_{20}$-Hydroxyalkylgruppen, wobei die Hydroxygruppe an ein primäres oder sekundäres Kohlenstoffatom der Kette gebunden sein kann und weiter die Alkylkette auch durch Sauerstoff unterbrochen sein kann, und/oder
- $C_3$- bis $C_{10}$-Cycloalkylgruppen und/oder $C_3$- bis $C_{12}$-Cycloalkenylgruppen, wobei die Ringe jeweils auch durch $-(CH_2)_n$-Gruppen mit n = 1 bis 3 überbrückt sein können.

Vorteile der Zubereitungen sind dabei insbesondere die UV-Licht filternde Wirkung und die gute Hautverträglichkeit. Zusätzlich sind die hier beschriebenen Verbindungen der zuvor genannten Formel farblos oder nur schwach gefärbt und führen so, im Gegensatz zu vielen bekannten natürlich vorkommenden Flavonoiden, nicht zu Verfärbungen der Zubereitungen.

Unter den erfindungsgemäß einzusetzenden Flavonoiden der zuvor genannten Formel finden sich dabei Breitband-UV-Filter, andere ebenfalls bevorzugte Verbindungen der zuvor genannten Formel zeigen ein Absorptionsmaximum im Grenzbereich zwischen der UV-B- und der UV-A-Strahlung. Als UV-A-II-Filter ergänzen sie daher vorteilhaft das Absorptionsspektrum von handelsüblichen UV-B- bzw. UV-A-I-Filtern. Bevorzugte Zubereitungen mit Lichtschutzeigenschaften enthalten zumindest eine Verbindung der zuvor genannten Formel, wobei $R^3$ steht für

- OH oder
- geradkettige oder verzweigte $C_1$- bis $C_{20}$-Alkoxygruppen, vorzugsweise Methoxy, Ethoxy oder Ethylhexyloxy oder
- Mono- und/oder Oligoglycosylreste, vorzugsweise Glucosylreste und
- $R^1$ und/oder $R^2$ vorzugsweise stehen für
- OH oder

- geradkettige oder verzweigte $C_1$- bis $C_{20}$-Alkoxygruppen, vorzugsweise Methoxy, Ethoxy oder Ethylhexyloxy oder

- Mono- und/oder Oligoglycosylreste, vorzugsweise Glucosylreste.

Diese bevorzugten Verbindungen zeichnen sich durch eine besonders intensive UV-Absorption aus.
Zusätzlich haben solche bevorzugten Verbindungen Vorteile bei der Einarbeitung in die Zubereitungen:

- Mono- und/oder Oligoglycosylreste verbessern die Wasserlöslichkeit der erfindungsgemäß einzusetzenden Verbindungen;

- geradkettige oder verzweigte $C_1$- bis $C_{20}$-Alkoxygruppen, insbesondere die langkettigen Alkoxyfunktionen, wie Ethylhexyloxy-Gruppen erhöhen die Öllöslichkeit der Verbindungen;

d.h. über die geeignete Auswahl der Substituenten kann die Hydrophilie bzw. Lipophilie der Verbindungen nach Formel I gesteuert werden. Als Mono- oder Oligosaccharidreste bevorzugt sind dabei Hexosylreste, insbesondere Ramnosylreste und Glucosylreste. Aber auch andere Hexosylreste, beispielsweise Allosyl, Altrosyl, Galactosyl, Gulosyl, Idosyl, Mannosyl und Talosyl sind gegebenenfalls vorteilhaft zu verwenden. Es kann auch vorteilhaft sein, Pentosylreste zu verwenden. Die Glycosylreste können $\alpha$- oder $\beta$-glycosidisch mit dem Grundkörper verbunden sein. Ein bevorzugtes Disaccharid ist beispielsweise das 6-O-(6-deoxy-$\alpha$-L-mannopyranosyl)-$\beta$-D-glucopyranosid.
Es hat sich gezeigt, dass die Intensität der UV-Absorption insbesondere dann hoch ist, wenn $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$-bis $C_{20}$-Alkoxygruppen, vorzugsweise Methoxy, Ethoxy oder Ethylhexyloxy.
Daher sind Zubereitungen mit Lichtschutzeigenschaften enthaltend zumindest eine Verbindung der zuvor genannten Formel, die dadurch gekennzeichnet ist, dass $R^3$ steht für geradkettige oder verzweigte $C_1$- bis $C_{20}$-Alkoxygruppen, vorzugsweise Methoxy, Ethoxy oder Ethylhexyloxy, und $R^8$ und $R^9$ gleich sind und stehen für H oder geradkettige oder verzweigte $C_1$- bis $C_{20}$-Alkoxygruppen, vorzugsweise Methoxy, Ethoxy oder Ethylhexyloxy, erfindungsgemäß besonders bevorzugt. Insbesondere ist es dabei bevorzugt, wenn und $R^8$ und $R^9$ für H stehen.
Die Verbindungen der zuvor genannten Formel werden typisch in Mengen von 0,01 Gew.-% bis 20 Gew.-%, vorzugsweise in Mengen von 0,5 Gew.-% bis 10 Gew.-% und insbesondere bevorzugt in Mengen von 1 Gew.-% bis 8 Gew.-% eingesetzt. Dabei bereitet es dem Fachmann keinerlei Schwierigkeiten die Mengen abhängig von dem beabsichtigten Lichtschutzfaktor der Zubereitung entsprechend auszuwählen.
Durch Kombination der erfindungsgemäßen Titandioxid-Partikel mit weiteren UV-Filtern kann die Schutzwirkung gegen schädliche Einwirkungen der UV-Strahlung optimiert werden. Optimierte Zubereitungen können beispielsweise die Kombination der organischen UV-Filter 4'-Methoxy-6-hydroxyflavon mit 1-(4-tert-Butylphenyl)-3-(4-methoxyphenyl)propan-1,3-dion und 3-(4'-Methylbenzyliden)-dl-kampfer enthalten.
Alle genannten UV-Filter können auch in verkapselter Form eingesetzt werden. Insbesondere ist es von Vorteil organische UV-Filter in verkapselter Form einzusetzen. Im Einzelnen ergeben sich die folgende Vorteile:

- Die Hydrophilie der Kapselwand kann unabhängig von der Löslichkeit des UV-Filters eingestellt werden. So können beispielsweise auch hydrophobe UV-Filter in rein wässrige Zubereitungen eingearbeitet werden. Zudem wird der häufig als unangenehm empfundene ölige Eindruck beim Auftragen der hydrophobe UV-Filter enthaltenden Zubereitung unterbunden.

- Bestimmte UV-Filter, insbesondere Dibenzoylmethanderivate, zeigen in kosmetischen Zubereitungen nur eine verminderte Photostabilität. Durch Verkapselung dieser Filter oder von Verbindungen, die die Photostabilität dieser Filter beeinträchtigen, wie beispielsweise Zimtsäurederivate, kann die Photostabilität der gesamten Zubereitung erhöht werden.

- In der Literatur wird immer wieder die Hautpenetration durch organische UV-Filter und das damit verbundene Reizpotential beim direkten Auftragen auf die menschliche Haut diskutiert. Durch die hier vorgeschlagene Verkapselung der entsprechenden Substanzen wird dieser Effekt unterbunden.

- Allgemein können durch Verkapselung einzelner UV-Filter oder anderer Inhaltstoffe Zubereitungsprobleme, die durch Wechselwirkung einzelner Zubereitungsbestandteile untereinander entstehen, wie Kristallisationsvorgänge, Ausfällungen und Agglomeratbildung vermieden werden, da die Wechselwirkung unterbunden wird.

Daher kann es erfindungsgemäß bevorzugt sein, wenn ein oder mehrere der Verbindungen gemäß Formel I

bzw. der oben genannten UV-Filter in verkapselter Form vorliegen. Vorteilhaft ist es dabei, wenn die Kapseln so klein sind, dass sie mit dem bloßen Auge nicht beobachtet werden können. Zur Erzielung der o.g. Effekte ist es weiterhin erforderlich, dass die Kapseln hinreichend stabil sind und den verkapselten Wirkstoff (UV-Filter) nicht oder nur in geringem Umfang an die Umgebung abgeben.

Geeignete Kapseln können Wände aus anorganischen oder organischen Polymeren aufweisen. Beispielsweise wird in US 6,242,099 B1 die Herstellung geeigneter Kapseln mit Wänden aus Chitin, Chitin-Derivaten oder polyhydroxylierten Polyaminen beschrieben. Erfindungsgemäß besonders bevorzugt einzusetzende Kapseln weisen Wände auf, die durch einen SolGel-Prozeß, wie er in den Anmeldungen WO 00/09652, WO 00/72806 und WO 00/71084 beschrieben ist, erhalten werden können. Bevorzugt sind hier wiederum Kapseln, deren Wände aus Kieselgel (Silica; undefiniertes Siliciumoxidhydroxid) aufgebaut sind. Die Herstellung entsprechender Kapseln ist dem Fachmann beispielsweise aus den zitierten Patentanmeldungen bekannt, deren Inhalt ausdrücklich auch zum Gegenstand der vorliegenden Anmeldung gehört.

Dabei sind die Kapseln in erfindungsgemäßen Zubereitungen vorzugsweise in solchen Mengen enthalten, die gewährleisten, dass die verkapselten UV-Filter in den oben angegebenen Mengen in der Zubereitung vorliegen.

Ferner können die erfindungsgemäßen Zubereitungen auch Farbstoffe und Farbpigmente enthalten. Die Farbstoffe und -pigmente können aus der entsprechenden Positivliste der Kosmetikverordnung bzw. der EG-Liste kosmetischer Färbemittel ausgewählt werden. In den meisten Fällen sind sie mit den für Lebensmittel zugelassenen Farbstoffen identisch. Vorteilhafte Farbpigmente sind beispielsweise Titandioxid, Glimmer, Eisenoxide (z. B. $Fe_2O_3$, $Fe_3O_4$, FeO(OH)) und/oder Zinnoxid. Vorteilhafte Farbstoffe sind beispielsweise Carmin, Berliner Blau, Chromoxidgrün, Ultramerinblau und/oder Manganviolett. Es ist insbesondere vorteilhaft, die Farbstoffe und/oder Farbpigmente aus der folgenden Liste zu wählen. Die Colour Index Nummern (CIN) sind dem Rowe Colour Index, 3. Auflage, Society of Dyers and Colourists, Bradford, England, 1971 entnommen.

Es kann ferner günstig sein, als Farbstoff eine oder mehrere Substanzen aus der folgenden Gruppe zu wählen:

2,4-Dihydroxyazobenzol, 1-(2'-Chlor-4'-nitro-1'phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfonsäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfonsäure, Aluminiumsalz der 4-(4-Sulfo-1-phenylazo)-2-(4-sulfophenyl)-5-hydroxy-pyrazolon-3-carbonsäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, rotes und schwarzes Eisenoxid (CIN: 77 491 (rot) und 77 499 (schwarz)), Eisenoxidhydrat (CIN: 77492), Manganammoniumdiphosphat und Titandioxid.

Ferner vorteilhaft sind öllösliche Naturfarbstoffe, wie z. B. Paprikaextrakt, ß-Carotin oder Cochenille.

Vorteilhaft im Sinne der vorliegenden Erfindung sind ferner Gelcremes mit einem Gehalt an Perlglanzpigmenten oder Interferenzpigmenten, insbesondere solchen Pigmenten, deren Verträglichkeit mit Dihydroxyaceton schon festgestellt wurde. Bevorzugt sind insbesondere die Perlglanzpigmente bzw. Interferenzpigmente, die auf einem plättchenförmigen Substrat basieren und mit Titandioxid und/oder Eisenoxid ($Fe_2O_3$) beschichtet sind und gegebenenfalls eine finale $SiO_2$-Schicht aufweisen.

Besonders bevorzugte Pigmente sind z. B. die von der Firma Merck unter den Handelsnamen Timiron®, Colorona®, Dichrona® oder Sirona® erhältlichen Pigmente.

Die Gesamtmenge der Farbstoffe und farbgebenden Pigmente wird vorteilhaft aus dem Bereich von z. B. 0,1 Gew.-% bis 30 Gew.-%, vorzugsweise von 0,5 Gew.-% bis 15 Gew.-%, insbesondere von 1,0 Gew.-% bis 10 Gew.-% gewählt, jeweils bezogen auf das Gesamtgewicht der Zubereitungen.

Die schützende Wirkung gegen oxidativen Stress bzw. gegen die Einwirkung von Radikalen kann weiter verbessert werden, wenn die Zubereitungen ein oder mehrere Antioxidantien enthalten.

Es gibt viele aus der Fachliteratur bekannte und bewährte Substanzen, die als Antioxidantien verwendet werden können, z.B. Aminosäuren (z.B. Glycin, Histidin, Tyrosin, Tryptophan) und deren Derivate, Imidazole, (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Buthioninsulfone, Penta-, Hexa-, Heptathioninsulfoximin) in sehr geringen verträglichen Dosierungen (z.B. pmol bis μmol/kg), ferner (Metall-) Chelatoren, (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, Pentanatriumethylendiamintetramethylenphosphonat (INCI: Pentasodium ethylenediamine tetramethylene phosphonate), ungesättigte Fettsäuren und deren Derivate, Vitamin C und Derivate (z.B. Ascorbylpalmitat, Magnesium-Ascorbylphosphat, Ascorbylacetat), Tocopherole und Derivate (z.B. Vitamin-E-acetat), Vitamin A und Derivate (z.B. Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordohydroguajaretsäure, Trihydroxybutyrophenon, Quercitin, Harnsäure und deren Derivate, Mannose und deren Derivate, Zink und dessen Derivate (z.B. ZnO, $ZnSO_4$), Selen und dessen Derivate (z.B. Selenmethionin), Stilbene und deren Derivate (z.B. Stilbenoxid, trans-Stilbenoxid).

Geeignete Antioxidantien sind auch Verbindungen der Formeln A oder B

A 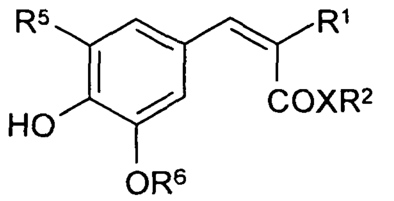 oder 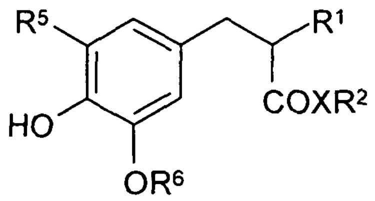 B

,

worin

$R^1$ aus der Gruppe $-C(O)CH_3$, $-CO_2R^3$, $-C(O)NH_2$ und $-C(O)N(R^4)_2$ ausgewählt werden kann,

X O oder NH,'

$R^2$ lineares oder verzweigtes Alkyl mit 1 bis 30 C-Atomen,

$R^3$ lineares oder verzweigtes Alkyl mit 1 bis 20 C-Atomen,

$R^4$ jeweils ungabhängig voneinander H oder lineares oder verzweigtes Alkyl mit 1 bis 8 C-Atomen,

$R^5$H oder lineares oder verzweigtes Alkyl mit 1 bis 8 C-Atomen oder lineares oder verzweigtes Alkoxy mit 1 bis 8 C-Atomen und

$R^6$ lineares oder verzweigtes Alkyl mit 1 bis 8 C-Atomen bedeutet, vorzugsweise Derivate der 2-(4-Hydroxy-3,5-dimethoxybenzyliden)-malonsäure und/oder 2-(4-Hydroxy-3,5-dimethoxybenzyl)-malonsäure, besonders bevorzugt 2-(4-Hydroxy-3,5-dimethoxybenzyliden)-malonsäure-bis-(2-ethylhexyl)ester (z.B. Oxynex® ST Liquid) und/oder 2-(4-Hydroxy-3,5-dimethoxybenzy)-malonsäure-bis-(2-ethylhexyl)ester (z.B. RonaCare® AP). Die genannten Verbindungen der Formel A oder B sind auch vorteilhafte Photostabilisatoren für organische UV-Filter, aber auch anderen photosensitiven Verbindungen, wie beispielsweise Parfum oder Farbstoffe.

Mischungen von Antioxidantien sind ebenfalls zur Verwendung in den erfindungsgemäßen kosmetischen Zubereitungen geeignet. Bekannte und käufliche Mischungen sind beispielsweise Mischungen enthaltend als aktive Inhaltsstoffe Lecithin, L-(+)-Ascorbylpalmitat und Zitronensäure, natürliche Tocopherole, L-(+)-Ascorbylpalmitat, L-(+)-Ascorbinsäure und Zitronensäure (z.B. Oxynex® K LIQUID), Tocopherolextrakte aus natürlichen Quellen, L-(+)-Ascorbylpalmitat, L-(+)-Ascorbinsäure und Zitronensäure (z.B. Oxynex® L LIQUID), DL-α-Tocopherol, L-(+)-Ascorbylpalmitat, Zitronensäure und Lecithin (z.B. Oxynex® LM) oder Butylhydroxytoluol (BHT), L-(+)-Ascorbylpalmitat und Zitronensäure (z.B. Oxynex® 2004).

Die erfindungsgemäßen Zubereitungen können als weitere Inhaltsstoffe Vitamine enthalten. Bevorzugt sind Vitamine und Vitamin-Derivate ausgewählt aus Vitamin A, Vitamin-A-Propionat, Vitamin-A-Palmitat, Vitamin-A-Acetat, Retinol, Vitamin B, Thiaminchloridhydrochlorid (Vitamin $B_1$), Riboflavin (Vitamin $B_2$), Nicotinsäureamid, Vitamin C (Ascorbinsäure), Vitamin D, Ergocalciferol (Vitamin $D_2$), Vitamin E, DL-α-Tocopherol, Tocopherol-E-Acetat, Tocopherolhydrogensuccinat, Vitamin $K_1$, Esculin (Vitamin P-Wirkstoff), Thiamin (Vitamin $B_1$), Nicotinsäure (Niacin), Pyridoxin, Pyridoxal, Pyridoxamin, (Vitamin $B_6$), Panthothensäure, Biotin, Folsäure und Cobalamin (Vitamin $B_{12}$) in den erfindungsgemäßen kosmetischen Zubereitungen enthalten, insbesondere bevorzugt Vitamin-A-Palmitat, Vitamin C, DL-α-Tocopherol, Tocopherol-E-Acetat, Nicotinsäure, Pantothensäure und Biotin.

Die erfindungsgemäßen Zubereitungen können darüber hinaus weitere übliche hautschonende oder hautpflegende Wirkstoffe enthalten. Dies können prinzipiell alle den Fachmann bekannten Wirkstoffe sein.

Besonders bevorzugte Wirkstoffe sind Pyrimidincarbonsäuren und/oder Aryloxime.

Dabei wird vorzugsweise eine Pyrimidincarbonsäure gemäß der unten stehenden Formel II eingesetzt,

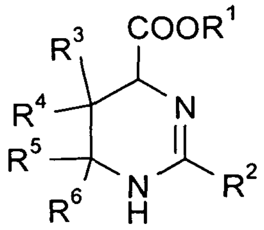

II

worin $R^1$ ein Rest H oder C1-8-Alkyl, $R^2$ ein Rest H oder C1-4-Alkyl und $R^3$, $R^4$, $R^5$ sowie $R^6$ jeweils unabhängig voneinander ein Rest aus der Gruppe H, OH, $NH_2$ und C1-4-Alkyl sind. Bevorzugt werden Pyrimidincarbonsäuren eingesetzt, bei denen $R^2$ eine Methyl- oder eine Ethylgruppe ist und $R^1$ bzw. $R^5$ und $R^6$ H sind. Insbesondere bevorzugt werden die Pyrimidincarbonsäuren Ectoin ((S)-1,4,5,6-Tetrahydro-2-methyl-4-pyrimidin-carbonsäure) und Hydroxyectoin ((S, S)-1,4,5,6-Tetrahydro-5-hydroxy-2-methyl-4-pyrimidin-carbonsäure) eingesetzt. Dabei enthalten die erfindungsgemäßen Zubereitungen derartige Pyrimidincarbonsäuren vorzugsweise in Mengen bis zu 15 Gew.-%.

Unter den Aryloximen wird vorzugsweise 2-Hydroxy-5-methyllauro-phenonoxim, welches auch als HMLO, LPO oder F5 bezeichnet wird, eingesetzt. Seine Eignung zum Einsatz in kosmetischen Mitteln ist beispielsweise aus der Deutschen Offenlegungsschrift DE-A-41 16 123 bekannt. Zubereitungen, die 2-Hydroxy-5-methyllaurophenonoxim enthalten, sind demnach zur Behandlung von Hauterkrankungen, die mit Entzündungen einhergehen, geeignet. Es ist bekannt, dass derartige Zubereitungen z.B. zur Therapie der Psioriasis, unterschiedlicher Ekzemformen, irritativer und toxischer Dermatitis, UV-Dermatitis sowie weiterer allergischer und/oder entzündlicher Erkrankungen der Haut und der Hautanhangsgebilde verwendet werden können. Erfindungsgemäße Zubereitungen, die Aryloxime, vorzugsweise 2-Hydroxy-5-methyllaurophenonoxim enthalten, zeigen überraschende antiinflammatorische Eignung. Dabei enthalten die Zubereitungen vorzugsweise 0,01 Gew.-% bis 10 Gew.-% des Aryloxims, wobei es insbesondere bevorzugt ist, wenn die Zubereitung 0,05 Gew.-% bis 5 Gew-% Aryloxim enthält.

Alle hier beschriebenen Verbindungen oder Komponenten, die in den Zubereitungen verwendet werden können, sind entweder bekannt und käuflich erwerbbar oder können nach bekannten Verfahren synthetisiert werden.

Die erfindungsgemäßen Zubereitungen können nach Verfahren hergestellt werden, die dem Fachmann gut bekannt sind, insbesondere nach den Verfahren, die zur Herstellung von Öl-in-Wasser-Emulsionen oder Wasser-in-Öl-Emulsionen dienen.

Ein weiterer Gegenstand der vorliegenden Erfindung ist ein Verfahren zur Herstellung einer Zubereitung, welches dadurch gekennzeichnet ist, dass die erfindungsgemäßen Partikel, wie zuvor beschrieben, mit einem kosmetisch oder dermatologisch geeigneten Träger und gegebenenfalls weiteren Inhaltsstoffen, wie zuvor beschrieben, vermischt wird.

Diese Zubereitungen können insbesondere in Form von einfachen oder komplizierten Emulsionen (O/W, W/O, O/W/O oder W/O/W), wie Cremes, Milchen, Gelen oder Gel-Cremes, Pulvern und festen Stiften, vorliegen und gegebenenfalls können sie als Aerosole konfektioniert sein und in Form von Schäumen oder Sprays vorliegen. Vorzugsweise liegen diese Zubereitungen in Form einer O/W-Emulsion vor.

Die erfindungsgemäßen kosmetischen Zubereitungen können als Zubereitung zum Schutz der menschlichen Epidermis oder der Haare gegen UV-Strahlung, zur Hautpflege, als Sonnenschutzmittel, Selbstbräunungsmittel oder Schminkprodukte verwendet werden.

Es soll darauf hingewiesen werden, dass in den erfindungsgemäßen Formulierungen, die einen Träger vom Typ einer Öl-in-Wasser-Emulsion aufweisen, die wässrige Phase (die insbesondere die hydrophilen Filter enthält) im Allgemeinen 50 Gew.-% bis 95 Gew.-% und vorzugsweise 70 Gew.-% bis 90 Gew.-%, bezogen auf die gesamte Formulierung, die Ölphase (die insbesondere die lipophilen Filter enthält) 5 Gew.-% bis 50 Gew.-% und vorzugsweise 10 Gew.-% bis 30 Gew.-%, bezogen auf die gesamte Formulierung und der (Co)emulgator oder die (Co)emulgatoren 0,5 Gew.-% bis 20 Gew.-% und vorzugsweise 2 Gew.-% bis 10 Gew.-%, bezogen auf die gesamte Formulierung, ausmachen.

Geeignet sind Zubereitungen für eine äußerliche Anwendung, beispielsweise als Creme, Lotion, Gel oder als Lösung, die auf die Haut aufgesprüht werden kann. Für eine innerliche Anwendung sind Darreichungsformen wie Kapseln, Dragees, Pulver, Tabletten-Lösungen oder Lösungen geeignet.

Als Anwendungsform der erfindungsgemäßen Zubereitungen seien z.B. genannt: Lösungen, Suspensionen, Emulsionen, PIT-Emulsionen, Pasten, Salben, Gele, Cremes, Lotionen, Puder, Seifen, tensidhaltige Reinigungspräparate, Öle, Aerosole und Sprays. Weitere Anwendungsformen sind z.B. Sticks, Shampoos und Duschbäder. Der Zubereitung können beliebige übliche Trägerstoffe, Hilfsstoffe und gegebenenfalls weitere Wirkstoffe zugesetzt werden.

Vorzuziehende Hilfsstoffe stammen aus der Gruppe der Konservierungsstoffe, Antioxidantien, Stabilisatoren, Lösungsvermittler, Vitamine, Färbemittel, Geruchsverbesserer.

Salben, Pasten, Cremes und Gele können die üblichen Trägerstoffe enthalten, z.B. tierische und pflanzliche Fette, Wachse, Paraffine, Stärke, Traganth, Cellulosederivate, Polyethylenglykole, Silicone, Bentonite, Kieselsäure,

Talkum und Zinkoxid oder Gemische dieser Stoffe.

Puder und Sprays können die üblichen Trägerstoffe enthalten, z.B. Milchzucker, Talkum, Kieselsäure, Aluminiumhydroxid, Calciumsilikat und Polyamid-Pulver oder Gemische dieser Stoffe. Sprays können zusätzlich die üblichen Treibmittel, z.B. Chlorfluorkohlenwasserstoffe, Propan/Butan oder Dimethylether, enthalten.

Lösungen und Emulsionen können die üblichen Trägerstoffe wie Lösungsmittel, Lösungsvermittler und Emulgatoren, z.B. Wasser, Ethanol, Isopropanol, Ethylcarbonat, Ethlyacetat, Benzylalkohol, Benzylbenzoat, Propylenglykol, 1,3-Butylglykol, Öle, insbesondere Baumwollsaatöl, Erdnussöl, Maiskeimöl, Olivenöl, Rizinusöl und Sesamöl, Glycerinfettsäureester, Polyethylenglykole und Fettsäureester des Sorbitans oder Gemische dieser Stoffe enthalten.

Suspensionen können die üblichen Trägerstoffe wie flüssige Verdünnungsmittel, z.B. Wasser, Ethanol oder Propylenglykol, Suspendiermittel, z.B. ethoxylierte Isostearylalkohole, Polyoxyethylensorbitester und Polyoxyethylensorbitanester, mikrokristalline Cellulose, Aluminiummetahydroxid, Bentonit, Agar-Agar und Traganth oder Gemische dieser Stoffe enthalten.

Seifen können die üblichen Trägerstoffe wie Alkalisalze von Fettsäuren, Salze von Fettsäurehalbestern, Fettsäureeiweißhydrolysaten, Isothionate, Lanolin, Fettalkohol, Pflanzenöle, Pflanzenextrakte, Glycerin, Zucker oder Gemische dieser Stoffe enthalten.

Tensidhaltige Reinigungsprodukte können die üblichen Trägerstoffe wie Salze von Fettalkoholsulfaten, Fettalkoholethersulfaten, Sulfobernsteinsäurehalbestern, Fettsäureeiweißhydrolysaten, Isothionate, Imidazoliniumderivate, Methyltaurate, Sarkosinate, Fettsäureamidethersulfate, Alkylamidobetaine, Fettalkohole, Fettsäureglyceride, Fettsäurediethanolamide, pflanzliche und synthetische Öle, Lanolinderivate, ethoxylierte Glycerinfettsäureester oder Gemische dieser Stoffe enthalten.

Gesichts- und Körperöle können die üblichen Trägerstoffe wie synthetische Öle wie Fettsäureester, Fettalkohole, Silikonöle, natürliche Öle wie Pflanzenöle und ölige Pflanzenauszüge, Paraffinöle, Lanolinöle oder Gemische dieser Stoffe enthalten.

Weitere typische kosmetische Anwendungsformen sind auch Lippenstifte, Lippenpflegestifte, Mascara, Eyeliner, Lidschatten, Rouge, Puder-, Emulsions- und Wachs-Make up sowie Sonnenschutz-, Prä-Sun- und After-Sun-Präparate.

Zu den bevorzugten erfindungsgemäßen Zubereitungsformen gehören insbesondere Emulsionen.

Erfindungsgemäße Emulsionen sind vorteilhaft und enthalten z. B. die genannten Fette, Öle, Wachse und anderen Fettkörper, sowie Wasser und einen Emulgator, wie er üblicherweise für einen solchen Typ der Zubereitung verwendet wird.

Die Lipidphase kann vorteilhaft gewählt werden aus folgender Substanzgruppe:

- Mineralöle, Mineralwachse
- Öle, wie Triglyceride der Caprin- oder der Caprylsäure, ferner natürliche Öle wie z. B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fett-Ikoholen mit Alkansäuren niedriger C-Zahl oder mit Fettsäuren;
- Silikonöle wie Dimethylpolysiloxane, Diethylpolysiloxane, Diphenylpolysiloxane sowie Mischformen daraus.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigtem und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäure und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexaldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silikonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12-18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige

Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, $C_{12}$-$_{15}$-Alkylbenzoat, Capryl-Caprinsäure-triglycerid, Dicaprylether.

Besonders vorteilhaft sind Mischungen aus $C_{12}$-$_{15}$-Alkylbenzoat und 2-Ethylhexylisostearat, Mischungen aus $C_{12}$-$_{15}$-Alkylbenzoat und Isotridecylisononanoat sowie Mischungen aus $C_{12}$-$_{15}$-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann auch die Ölphase ferner einen Gehalt an cyclischen oder linearan Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die wässrige Phase der erfindungsgemäßen Zubereitungen enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z. B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Insbesondere werden Gemisch der vorstehend genannten Lösemittel verwendet. Bei alkoholischen Lösemitteln kann Wasser ein weiterer Bestandteil sein.

In einer bevorzugten Ausführungsform enthalten die erfindungsgemäßen Zubereitungen hydrophile Tenside.

Die hydrophilen Tenside werden bevorzugt gewählt aus der Gruppe der Alkylglucoside, der Acyllactylate, der Betaine sowie der Cocoamphoacetate.

Erfindungsgemäß besonders vorteilhaft verwendete Alkylglylcoside werden gewählt aus der Gruppe Octylglucopyranosid, Nonylglucopyranosid, Decylglucopyranosid, Undecylglucopyranosid, Dodecylglucopyranosid, Tetradecylglucopyranosid und Hexadecylglucopyranosid.

Es ist ebenfalls von Vorteil, natürliche oder synthetische Roh- und Hilfsstoffe bzw. Gemische einzusetzen, welche sich durch einen wirksamen Gehalt an den erfindungsgemäß verwendeten Wirkstoffen auszeichnen, beispielsweise Plantaren® 1200 (Henkel KGaA), Oramix® NS 10 (Seppic).

Die Acyllactylate werden ihrerseits vorteilhaft gewählt aus der Gruppe der Substanzen, welche sich durch die Strukturformel

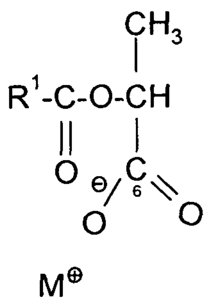

auszeichnen, wobei $R^1$ einen verzweigten oder unverzweigten Alkylrest mit 1 bis 30 Kohlenstoffatomen bedeutet und $M^+$ aus der Gruppe der Alkaliionen sowie der Gruppe der mit einer oder mehreren Alkyl- und/oder mit einer oder mehreren Hydroxyalkylresten substituierten Ammoniumionen gewählt wird bzw. dem halben Äquivalent eines Erdalkalions entspricht.

Vorteilhaft ist beispielsweise Natriumisostearyllactylat, beispielsweise das Produkt Pathionic® ISL von der Gesellschaft American Ingredients Company.

Für die Gruppe der Betaine ist beispielsweise Capramidopropylbetain, beispielsweise das Produkt Tego® Betain 810 von der Gesellschaft Th. Goldschmidt AG vorteilhaft.

Als erfindungsgemäß vorteilhaftes Cocoamphoacetat wird beispielsweise Natriumcocoamphoacetat gewählt, wie es unter der Bezeichnung Miranol® Ultra C32 von der Gesellschaft Miranol Chemical Corp. erhältlich ist.

Die erfindungsgemäßen Zubereitungen sind vorteilhaft dadurch gekennzeichnet, dass das hydrophile Tensid oder die hydrophilen Tenside in Konzentrationen von 0,01-20 Gew.-% bevorzugt 0,05-10 Gew.-%, besonders bevorzugt 0,1-5 Gew.-%, jeweils bezogen auf das Gesamtgewicht der Zubereitung, vorliegt oder vorliegen.

Zu Anwendung werden die erfindungsgemäßen kosmetischen und dermatologischen Zubereitungen in der für Kosmetika üblichen Weise auf die Haut und/oder die Haare in ausreichender Menge aufgebracht.

Erfindungsgemäße kosmetische und dermatologische Zubereitungen können in verschiedenen Formen vorliegen. So können sie z. B. eine Lösung, eine wasserfreie Zubereitung, eine Emulsion oder Mikroemulsion vom Typ Wasser-in-Öl (W/O) oder vom Typ Öl-in-Wasser (O/W), eine multiple Emulsion, beispielsweise vom Typ Waser-in-Öl-in-Wasser (W/O/W), ein Gel, einen festen Stift, eine Salbe oder auch ein Aerosol darstellen. Es ist auch vorteilhaft, Ectoine in verkapselter Form darzureichen, z. B. in Kollagenmatrices und anderen üblichen Verkapselungsmaterialien, z. B. als Celluloseverkapselungen, in Gelatine, Wachsmatrices oder liposomal verkapselt. Insbesondere Wachsmatrices wie sie in der DE-OS 43 08 282 beschrieben werden, haben sich als günstig herausgestellt. Bevorzugt werden Emulsionen. O/W-Emulsinen werden besonders bevorzugt. Emulsionen, W/O-Emulsionen und O/W-Emulsionen sind in üblicher Weise erhältlich.

Als Emulgatoren können beispielsweise die bekannten W/O- und O/W-Emulgatoren verwendet werden. Es ist vorteilhaft, weitere übliche Co-emulgatoren in den erfindungsgemäßen bevorzugten O/W-Emulsionen zu verwenden.

Als erfindungsgemäß besonders bevorzugter Emulgator für O/W-Emulsionen hat sich das Handelsprodukt Ceralution C der Firma Sasol erwiesen.

Erfindungsgemäß vorteilhaft werden als Co-Emulgatoren beispielsweise O/W-Emulgatoren gewählt, vornehmlich aus der Gruppe der Substanzen mit HLB-Werten von 11-16, ganz besonders vorteilhaft mit HLB-Werten von 14,5-15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkhole, Cetylalkohole, Cetylstearylalkohole (Cetearylalkohole) zu wählen. Insbesondere bevorzugt sind: Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17),Polyethylenglycol(18)stearylether (Steareth-18), Polyethylenglycol(19)-stearylether (Steareth-19), Polyethylenglycol(20)stearylether (Steareth-20), Polyethylenglycol(12)isostearylether (Isosteareth-12), Polyethylenglycol(13)isostearylether (Isosteareth-13), Polyethylenglycol(14)-isostearylether (Isosteareth-14), Polyethylenglycol(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol(20)-isostearylether (Isosteareth-20), Polyethylenglycol(13)cetylether (Ceteth-13), Polyethylenglycol(14)cetylether (Ceteth-14), Polyethylenglycol(15)-cetylether (Ceteth-15), Polyethylenglycol(16)cetylether (Ceteth-16), Polyethylenglycol(17)cetylether (Ceteth-17), Polyethylenglycol(18)-cetylether (Ceteth-18), Polyethylenglycol(19)cetylether (Ceteth-19), Polyethylen-glycol(20)cetylether (Ceteth-20), Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)-isocetylether (Isoceteth-16), Polyethylenglycol(17)isocetylether (Isoceteth-17), Polyethylenglycol(18)isocetylether (Isoceteth-18), Polyethylenglycol-(19)isocetylether (Isoceteth-19), Polyethylenglycol(20)isocetylether (Isoceteth-20), Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15), Polyethylenglycol(12)laurylether (Laureth-12), Polyethylenglycol(12)isolaurylether (Isolaureth-12), Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)cetylstearylether (Ceteareth-16), Polyethylenglycol(17)cetylstearylether (Ceteareth-17), Polyethylenglycol(18)cetylstearylether (Ceteareth-18), Polyethylenglycol-(19)cetylstearylether (Ceteareth-19), Polyethylenglycol(20)cetylstearylether (Ceteareth-20).

Es ist ferner von Vorteil, die Fettsäureethoxylate ausfolgender Gruppe zu wählen:

Polyethylenglycol(20)stearat, Polyethylenglycol(21)stearat,
Polyethylenglycol(22)stearat, Polyethylenglycol(23)stearat,
Polyethylenglycol(24)stearat, Polyethylenglycol(25)stearat,
Polyethylenglycol(12)isostearat, Polyethylenglycol(13)isostearat,
Polyethylenglycol(14)isostearat, Polyethylenglycol(15)isostearat,
Polyethylenglycol(16)isostearat, Polyethylenglycol(17)isostearat,
Polyethylenglycol(18)isostearat, Polyethylenglycol(19)isostearat,
Polyethylenglycol(20)isostearat, Polyethylenglycol(21)isostearat,
Polyethylenglycol(22)isostearat, Polyethylenglycol(23)isostearat,

Polyethylenglycol(24)isostearat, Polyethylenglycol(25)isostearat,
Polyethylenglycol(12)oleat, Polyethylenglycol(13)oleat,
Polyethylenglycol(14)oleat, Polyethylenglycol(15)oleat,
Polyethylenglycol(16)oleat, Polyethylenglycol(17)oleat,
Polyethylenglycol(18)oleat, Polyethylenglycol(19)oleat,
Polyethyleng lycol(20)oleat,

Als ethoxylierte Alkylethercarbonsäure bzw. deren Salz kann vorteilhaft das Natriumlaureth-11-carboxylat verwendet werden. Als Alkylethersulfat kann Natrium Laureth1-4sulfat vorteilhaft verwendet werden. Als ethoxyliertes Cholesterinderivat kann vorteilhaft Polyethylenglycol(30)Cholesterylether verwendet werden. Auch Polyethylenglycol(25)Sojasterol hat sich bewährt. Als ethoxylierte Triglyceride können vorteilhaft die Polyethylenglycol(60) Evening Primrose Glycerides verwendet werden (Evening Primrose = Nachtkerze).

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/cprinat, Polyethylenglycol-(20)glyceryloleat, Polyethylenglycol(20)glycerylisostearat, Polyethylenglycol(18)glyceryloleat(cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als fakultative, dennoch erfindungsgemäß gegebenenfalls vorteilhafte W/O-Emulgatoren können eingesetzt werden:

Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12-18 C-Atome, Diglycerinester gesättigter und/oder ungesättigter, verzweigter und/oder unverzweigter Alkancarbonsäuren einer Kettenlänge von 8 bis 24, insbesondere 12-18 C-Atomen, Monoglycerinether gesättigter und/oder ungesättigter, verzweigter und/oder unverzweigter Alkohole einer Kettenlänge von 8 bis 24, insbesondere 12-18 C-Atomen, Diglycerinether gesättigter und/oder ungesättigter, verzweigter und/oder unverzweigter Alkhole einer Kettenlänge von 8 bis 24, insbesondere 12-18 C-Atomen, Propylenglycolester gesättigter und/oder ungesättigter, verzweigter und/oder unverzweigter Alkancarbonsäuren einer Kettenlänge von 8 bis 24, insbesondere 12-18 C-Atomen sowie Sorbitanester gesättigter und/oder ungesättigter, verzweigter und/oder unverzweigter Alkancarbonsäuren einer Kettenlänge von 8 bis 24, insbesondere 12-18 C-Atomen.

Insbesondere vorteilhafte W/O-Emulgatoren sind Glycerylmonostearat, Glycerylmonoisostearat, Glycerylmonomyristat, Glycerylmonooleat, Diglycerylmonostearat, Diglycerylmonoisostearat, Propylenglycolmonostearat, Propylenglycolmonoisostearat, Propylenglycolmonocaprylat, Propylenglycolmonolaurat, Sorbitanmonoisostearat, Sorbitanmonolaurat, Sorbitanmonocaprylat, Sorbitanmonoisooleat, Saccharosedistearat, Cetylalkohol, Stearylalkohol, Arachidylalkohol, Behenylalkohol, Isobehenylalkohol, Selachylalkohol, Chimylalkohol, Polyethylenglycol(2)stearylether (Steareth-2), Glycerylmonolaurat, Glycerylmonocaprinat, Glycerylmonocaprylat.

Erfindungsgemäß bevorzugte Zubereitungen eignen sich besonders zum Schutz menschlicher Haut gegen UV-induzierte Alterungsprozesse sowie vor oxidativem Stress, d.h. gegen Schädigungen durch Radikale, wie sie z.B. durch Sonneneinstrahlung, Wärme oder andere Einflüsse erzeugt werden. Dabei liegt sie in verschiedenen, für diese Anwendung üblicherweise verwendeten Darreichungsformen vor. So kann sie insbesondere als Lotion oder Emulsion, wie als Creme oder Milch (O/W, W/O, O/W/O, W/O/W), in Form ölig-alkoholischer, ölig-wässriger oder wässrig-alkoholischer Gele bzw. Lösungen, als feste Stifte vorliegen oder als Aerosol konfektioniert sein.

Die Zubereitung kann kosmetische Adjuvantien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglykol, Glycerin und Sorbit.

Eine bevorzugte Ausführungsform der Erfindung ist eine Emulsion, welche als Schutzcreme oder -milch vorliegt und außer der oder den Verbindungen der Formel I beispielsweise Fettalkohole, Fettsäuren, Fettsäureester, insbesondere Triglyceride von Fettsäuren, Lanolin, natürliche und synthetische Öle oder Wachse und Emulgatoren in Anwesenheit von Wasser enthält.

Weitere bevorzugte Ausführungsformen stellen ölige Lotionen auf Basis von natürlichen oder synthetischen

Ölen und Wachsen, Lanolin, Fettsäureestern, insbesondere Triglyceriden von Fettsäuren, oder ölig-alkoholische Lotionen auf Basis eines Niedrigalkohols, wie Ethanol, oder eines Glycerols, wie Propylenglykol, und/oder eines Polyols, wie Glycerin, und Ölen, Wachsen und Fettsäureestern, wie Triglyceriden von Fettsäuren, dar.

Die erfindungsgemäße Zubereitung kann auch als alkoholisches Gel vorliegen, welches einen oder mehrere Niedrigalkohole oder -polyole, wie Ethanol, Propylenglykol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestern, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Die kosmetische Zubereitung kann auch zum Schutz der Haare gegen fotochemische Schäden verwendet werden, um Veränderungen von Farbnuancen, ein Entfärben oder Schäden mechanischer Art zu verhindern. In diesem Fall erfolgt geeignet eine Konfektionierung als Shampoo, Lotion, Gel oder Emulsion zum Ausspülen, wobei die jeweilige Zubereitung vor oder nach dem Shamponieren, vor oder nach dem Färben oder Entfärben bzw. vor oder nach der Dauerwelle aufgetragen wird. Es kann auch eine Zubereitung als Lotion oder Gel zum Frisieren und Behandeln, als Lotion oder Gel zum Bürsten oder Legen einer Wasserwelle, als Haarlack, Dauerwellenmittel, Färbe- oder Entfärbemittel der Haare gewählt werden. Die Zubereitung mit Lichtschutzeigenschaften kann verschiedene, in diesem Mitteltyp verwendete Adjuvantien enthalten, wie Grenzflächen aktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die Zubereitungen, wie zuvor beschrieben, können die genannten notwendigen oder optionalen Bestandteile/Inhaltsstoffe enthalten oder umfassen, daraus im Wesentlichen oder daraus bestehen.

Im folgenden wird die Erfindung anhand von Beispielen näher erläutert.

**Beispiele**

**Beispiel der $SiO_2$-Bestimmung bzw. der Bestimmung der Nachbehandlungssubstanz, enthaltend Si.**

0,1g bis 0,5g der Probe (auf 0,0001 genau gewogen), je nach dem zu erwartenden Gehalt an Si, werden in einem kleinen Platintiegel mit 5 g Soda/Borax-Mischung 7+1 versetzt und etwa 30 Minuten aufgeschlossen. Nach dem Abkühlen laugt an die Schmelze in 50 ml verdünnter Salzsäure kalt aus, überführt den Ansatz in einen 250 ml fassenden Maßkolben, füllt mit bidestilliertem Wasser zur Marke auf und schüttelt vorsichtig ($CO_2$) durch.
Die Bestimmung der Kieselsäure erfolgt je nach Gehalt entweder direkt oder nach entsprechender Verdünnung (die maximale Aufschlussmittelkonzentration für die Messung am ICP-OES beträgt 2%). Ein Chemikalienblindsatz ist obligatorisch.

Die Kalibrierung erfolgt mit Hilfe von Standardlösungen, deren Matrix der probenmatrix entspricht.

Gemessen wird an dem Gerät (ICP-OES) der Firma Perkin Elmer, Optima 3300DV, wobei die Bedienungsanleitung des Geräts benutzt wird. Der $SiO_2$-Gehalt wird hier auf den Gesamtpartikel ermittelt, d.h. die Werte liegen unterhalb der angegebnen Werte für den $SiO_2$-Gehalt bezogen auf den $TiO_2$-Gehalt der zu beschichtenden Partikel.

$$\text{Zur Berechnung Si} \rightarrow SiO_2 \quad 2{,}139$$

$$SiO_2 * 1{,}220 = \text{Simethicone}$$

Verwendete Reagenzien und Lösungen:

Soda, wasserfrei, p.a.
Borax, Natriumtetraborat, p.a.
Salzsäure, p.a. 1+1 verdünnt mit bidestilliertem Wasser.

**Beispiel der Ermittlung des Gehaltes an organischer Phosphorverbindung durch Bestimmung des Kohlenstoffgehaltes.**

Die Bedienung des eingesetzten CS-Bestimmungsautomaten, z.B. Eltra CS580, wird als bekannt vorausgesetzt oder ist der entsprechenden Bedienungsanleitung zu entnehmen.

0,1g bis 0,5g der Probe (auf 0,0001 genau gewogen) werden auf einem Trägerschiffchen eingewogen.
Die Probe wird in die Heizzone des Verbrennungsrohres geschoben, wo sie bei 1300°C im Sauerstoffstrom verbrannt wird. Der Ablauf der Kohlenstoffbestimmung erfolgt automatisch durch das Bestimmungsgerät und wird durch die Ausgabe des Kohlenstoffgehaltes abgeschlossen. Überprüfung und Kalibrierung des Messgerätes mit werkseigenen Standardproben und British Chemical Standards (B.C.S.) Referenz-Standard.

Zur Ermittlung des Gehalts an organischer Phosphorverbindung wird der erhaltene Kohlenstoffgehalt über den Kohlenstoffgehalt der organischen Phosphorverbindung berechnet. Da dieser, je nach eingesetzter Phosphorverbindung, unterschiedlich ist, wird die Berechnung anhand eines fiktiven Beispiels angegeben: Die als Ausgangstoff eingesetzte organische Phosphorverbindung enthält 67% C.
Bei der Bestimmung des Kohlenstoffgehaltes der nachbehandelten Probe wurde ein Wert von 6,7% C ermittelt.
67% C entspricht 100% organische Phosphorverbindung

$$6{,}7\%\ \text{C entspricht}\ \frac{6{,}7 \times 100}{67} = 10\%\ \text{organische Phosphorverbindung}$$

Die nachbehandelte Probe enthält somit 10% der organischen Phosphorverbindung.

Beispiel 1a: Herstellung Titandioxid-Ausgangsmaterial

x l einer wässrigen Suspension von Metatitansäure mit einem Gehalt von rund 370 g $TiO_2$/l werden mit 0,9x l Wasser und x l 50%iger Natronlauge gemischt, unter Rühren auf Siedetemperatur erhitzt und 2 h bei der Temperatur gehalten. Das dabei entstehende Natriumtitanat wird gewaschen und mit Wasser auf einen Gehalt von ca. 100 - 110 g $TiO_2$/l eingestellt.

Das Titanat wird auf 60 °C erhitzt. Danach wird mit 30%iger HCl bis pH 2 angesäuert, anschließend 45 min bei der Temperatur und dem pH-Wert gerührt.

Mit Wasser und 30%iger Salzsäure werden anschließend Gehalte von 45 g HCl/l und 90 g $TiO_2$/l eingestellt und die Suspension unter Rühren 6 h bei 85 °C gehalten ("Peptisierung").

Die peptisierte Suspension wird mit 50%iger NaOH langsam bis pH 5,5 neutralisiert.

Vergleichsbeispiel 1b: Titandioxid ohne Nachbehandlung

Das aus Beispiel 1a erhaltene Material wird abfiltriert und mit Wasser auf eine Leitfähigkeit < 100 µS/cm gewaschen. Der erhaltene Filterkuchen enthält ca. 18 % Produkt. Der Filterkuchen wird getrocknet und desaggregiert.

Beispiel 1c: Hydrothermalbehandlung (HT)

Das aus Beispiel 1a erhaltene Material wird auf 50 g $TiO_2$/l verdünnt und für die Dauer von 2 h einer Hydrothermalbehandlung im Druckbehälter bei 180°C unterzogen.

Beispiel 1d: Nachbehandlung mit 20% $SiO_2$

Eine x g $TiO_2$ entsprechende Menge des aus Beispiel 1a erhaltenen Materials wird auf 50 g $TiO_2$/l verdünnt und anschließend bei 80°C mit einer Menge Natronwasserglas-Lösung (Konzentration: ca. 270 g $SiO_2$/l) versetzt, die 0,2x g $SiO_2$ entspricht und der dabei steigende pH-Wert mit verdünnter Schwefelsäure konstant bei pH 9,0 gehalten.

Nach Zugabe der gesamten Wasserglas-Lösung wird mit $H_2SO_4$ ein pH-Wert von 6,5 eingestellt und die Suspension 2 h bei der Temperatur und dem pH-Wert unter Rühren reifen gelassen. Die erhaltene Suspension enthält ca. 6 bis 7% Produkt.

Beispiel 1e: Nachbehandlung mit 35% $SiO_2$

Eine x g $TiO_2$ entsprechende Menge des aus Beispiel 1a erhaltenen Materials wird auf 50 g $TiO_2$/l verdünnt und anschließend bei 80°C mit einer Menge Natronwasserglas-Lösung (Konzentration: ca. 270 g $SiO_2$/l) versetzt, die 0,35x g $SiO_2$ entspricht und der dabei steigende pH-Wert mit verdünnter Schwefelsäure konstant bei pH 9,0 gehalten.

Nach Zugabe der gesamten Wasserglas-Lösung wird mit $H_2SO_4$ ein pH-Wert von 6,5 eingestellt und die Suspension 2 h bei der Temperatur und dem pH-Wert unter Rühren reifen gelassen. Die erhaltene Suspension enthält ca. 6 bis 8% Produkt.

Beispiel 1f: Nachbehandlung mit 20% $SiO_2$ auf HT-behandeltem Grundpartikel

Eine x g $TiO_2$ entsprechende Menge des aus Beispiel 1c erhaltenen Materials wird bei 80°C mit einer Menge Natronwasserglas-Lösung (Konzentration: ca. 270 g $SiO_2$/l) versetzt, die 0,2x g $SiO_2$ entspricht und der dabei steigende pH-Wert mit verdünnter Schwefelsäure konstant bei pH 9,0 gehalten.

Nach Zugabe der gesamten Wasserglas-Lösung wird mit $H_2SO_4$ ein pH-Wert von 6,5 eingestellt und die Suspension 2 h bei der Temperatur und dem pH-Wert unter Rühren reifen gelassen. Die erhaltene Suspension enthält ca. 6 bis 7% Produkt.

Beispiel 1g: Nachbehandlung mit 35% $SiO_2$ auf HT-behandeltem Grundpartikel

Eine x g $TiO_2$ entsprechende Menge des aus Beispiel 1c erhaltenen Materials wird bei 80°C mit einer Menge Natronwasserglas-Lösung (Konzentration: ca. 270 g $SiO_2$/l) versetzt, die 0,35x g $SiO_2$ entspricht und der dabei steigende pH-Wert mit verdünnter Schwefelsäure konstant bei pH 9,0 gehalten.

Nach Zugabe der gesamten Wasserglas-Lösung wird mit $H_2SO_4$ ein pH-Wert von 6,5 eingestellt und die Suspension 2 h bei der Temperatur und dem pH-Wert unter Rühren reifen gelassen. Die erhaltene Suspension enthält ca. 7 bis 8% Produkt.

Vergleichsbeispiel 2a: Titandioxid mit 20% $SiO_2$

Das aus Beispiel 1d erhaltene Material wird abfiltriert und mit Wasser auf eine Leitfähigkeit < 100 µS/cm gewaschen. Der erhaltene Filterkuchen enthält ca. 18 % Produkt. Der Filterkuchen wird getrocknet und desaggregiert.

Vergleichsbeispiel 2b: Titandioxid mit 35% $SiO_2$

Das aus Beispiel 1e erhaltene Material wird abfiltriert und mit Wasser auf eine Leitfähigkeit < 100 µS/cm gewaschen. Der erhaltene Filterkuchen enthält ca. 18 % Produkt. Der Filterkuchen wird getrocknet und desaggregiert.

Vergleichsbeispiel 3a: HT-behandeltes Titandioxid mit 20% $SiO_2$

Das aus Beispiel 1f erhaltene Material wird abfiltriert und mit Wasser auf eine Leitfähigkeit < 100 µS/cm gewaschen. Der erhaltene Filterkuchen enthält ca. 18 % Produkt. Der Filterkuchen wird getrocknet und desaggregiert.

Vergleichsbeispiel 3b: HT-behandeltes Titandioxid mit 35% $SiO_2$

Das aus Beispiel 1g erhaltene Material wird abfiltriert und mit Wasser auf eine Leitfähigkeit < 100 µS/cm gewaschen. Der erhaltene Filterkuchen enthält ca. 18 % Produkt. Der Filterkuchen wird getrocknet und desaggregiert.

Beispiel 4a: Titandioxid mit 35% $SiO_2$ und 5% Phosphorsäuremonocetylester

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,05x g Hostaphat CC100 (Phosphorsäuremonocetylester) der Fa. Clariant zugegeben und 10 Minuten bei konstanter Temperatur nachgerührt. Die Suspension wird unter Rühren abgekühlt, getrocknet und desaggregiert.

Beispiel 4b: Titandioxid mit 35% $SiO_2$ und 10% Phosphorsäuremonocetylester

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,1x g Hostaphat CC100 (Phosphorsäuremonocetylester) der Fa. Clariant zugegeben und 10 Minuten bei konstanter Temperatur nachgerührt. Die Suspension wird unter Rühren abgekühlt, getrocknet und desaggregiert.

Beispiel 4c: Titandioxid mit 35% $SiO_2$ und 15% Phosphorsäuremonocetylester

Eine x g (salzfrei gewaschenem) Feststoff entsprechende Menge der Suspension aus Beispiel 1e wird auf 80°C erhitzt. In die Suspension werden unter Rühren 0,15x g Hostaphat CC100 (Phosphorsäuremonocetylester) der

Fa. Clariant zugegeben und 10 Minuten bei konstanter Temperatur nachgerührt. Die Suspension wird unter Rühren abgekühlt, abfiltriert und mit Wasser auf eine Leitfähigkeit < 100 µS/cm gewaschen. Der erhaltene Filterkuchen wird getrocknet und desaggregiert.

Beispiel 4d: Titandioxid mit 35% $SiO_2$ und 15% Phosphorsäuremono- und distearylester-Mischung

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,15x g Hostaphat CS120 (Phosphorsäuremono- und distearylester-Mischung) der Fa. Clariant zugegeben und 10 Minuten bei konstanter Temperatur nachgerührt. Die Suspension wird unter Rühren abgekühlt, getrocknet und desaggregiert.

Beispiel 4e: Titandioxid mit 35% $SiO_2$ und 15% 1-Hydroxyethan-1,1-diphosphonsäure

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,25x g Cublen K60 (enthält ca. 60% 1-Hydroxyethan-1,1-diphosphonsäure) der Fa. Zschimmer & Schwarz Mohsdorf zugegeben und 30 Minuten bei konstanter Temperatur nachgerührt. Die Suspension wird unter Rühren abgekühlt, getrocknet und desaggregiert.

Beispiel 4f: Titandioxid mit 35% $SiO_2$ und 15% 2-Phosphonobutan-1,2,4-tricarbonsäure

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,3x g Cublen P50 (enthält ca. 50% 2-Phosphonobutan-1,2,4-tricarbonsäure) der Fa. Zschimmer & Schwarz Mohsdorf zugegeben und 30 Minuten bei konstanter Temperatur nachgerührt. Die Suspension wird unter Rühren abgekühlt, getrocknet und desaggregiert.

Beispiel 4g:Titandioxid mit 35% $SiO_2$ und 15% Aminotrismethylenphosphonsäure

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,3x g Cublen AP5 (enthält ca. 50% Aminotrismethylenphosphonsäure) der Fa. Zschimmer & Schwarz Mohsdorf zugegeben und 30 Minuten bei konstanter Temperatur nachgerührt. Die Suspension wird unter Rühren abgekühlt, getrocknet und desaggregiert.

Beispiel 4h: Titandioxid mit 35% $SiO_2$ und 15% Laurvlphosphonsäure

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,15x g Laurylphosphonsäure der Fa. Rhodia zugegeben und 30 Minuten bei konstanter Temperatur nachgerührt. Die Suspension wird unter Rühren abgekühlt, getrocknet und desaggregiert.

Beispiel 4i: Titandioxid mit 35% $SiO_2$ und 15% Octylphosphonsäure

Das aus Beispiel 1e erhaltene Material wird abfiltriert und mit Wasser auf eine Leitfähigkeit < 100 µS/cm gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,15x g Octylphosphonsäure der Fa. Rhodia zugegeben und 30 Minuten bei konstanter Temperatur nachgerührt. Die Suspension wird unter Rühren abgekühlt, getrocknet und desaggregiert.

Beispiel 4k: Titandioxid mit 35% $SiO_2$ und 5% 1-Hvdroxvdodecan-1,1-Diphosphonsäure

Das aus Beispiel 1e erhaltene Material wird abfiltriert und mit Wasser auf eine Leitfähigkeit < 100 µS/cm

gewaschen. Der erhaltene Filterkuchen enthält ca. 18 % Feststoff. Eine x g Feststoff entsprechende Menge des Filterkuchens wird mit etwas Wasser verdünnt und auf 80°C erhitzt. In die Suspension werden unter Rühren 0,067x g Tensan AO Konzentrat (enthält ca. 75% 1-Hydroxydodecan-1,1-Diphosphonsäure) der Fa. Polygon Chemie zugegeben und 30 Minuten bei konstanter Temperatur nachgerührt. Die Suspension wird unter Rühren abgekühlt, getrocknet und desaggregiert.

Beispiel 5a: HT-Titandioxid mit 20% $SiO_2$ und 3% Phosphorsäuremonocetvlester

Das aus Beispiel 1f erhaltene Material wird abfiltriert und mit Wasser auf eine Leitfähigkeit < 100 μS/cm gewaschen. Eine x g Feststoff entsprechende Menge des Filterkuchens wird mit etwas Wasser verdünnt und auf 80°C erhitzt. In die Suspension werden unter Rühren 0,03x g Hostaphat CC100 (Phosphorsäuremonocetylester) der Fa. Clariant zugegeben und 10 Minuten bei konstanter Temperatur nachgerührt. Die Suspension wird unter Rühren abgekühlt, getrocknet und desaggregiert.

Beispiel 5b: HT-Titandioxid mit 20% $SiO_2$ und 6% Phosphorsäuremonocetylester

Eine x g (salzfrei gewaschenem) Feststoff entsprechende Menge der Suspension aus Beispiel 1f wird auf 80°C erhitzt. In die Suspension werden unter Rühren 0,06x g Hostaphat CC100 (Phosphorsäuremonocetylester) der Fa. Clariant zugegeben und 45 Minuten bei konstanter Temperatur nachgerührt. Die Suspension wird heiß abfiltriert und mit Wasser auf eine Leitfähigkeit < 100 μS/cm gewaschen. Der erhaltene Filterkuchen wird getrocknet und desaggregiert.

Beispiel 5c: HT-Titandioxid mit 20% $SiO_2$ und 9% Phosphorsäuremonocetylester

Das aus Beispiel 1f erhaltene Material wird abfiltriert und mit Wasser auf eine Leitfähigkeit < 100 μS/cm gewaschen. Eine x g Feststoff entsprechende Menge des Filterkuchens wird mit etwas Wasser verdünnt und auf 80°C erhitzt. In die Suspension werden unter Rühren 0,09x g Hostaphat CC100 (Phosphorsäuremonocetylester) der Fa. Clariant zugegeben und 10 Minuten bei konstanter Temperatur nachgerührt. Die Suspension wird unter Rühren abgekühlt, getrocknet und desaggregiert.

Beispiel 5d: HT-Titandioxid mit 35% $SiO_2$ und 3% Phosphorsäuremonocetylester

Das aus Beispiel 1g erhaltene Material wird abfiltriert und mit Wasser auf eine Leitfähigkeit < 100 μS/cm gewaschen. Eine x g Feststoff entsprechende Menge des Filterkuchens wird mit etwas Wasser verdünnt und auf 80°C erhitzt. In die Suspension werden unter Rühren 0,03x g Hostaphat CC100 (Phosphorsäuremonocetylester) der Fa. Clariant zugegeben und 10 Minuten bei konstanter Temperatur nachgerührt. Die Suspension wird unter Rühren abgekühlt, getrocknet und desaggregiert.

Beispiel 5e: HT-Titandioxid mit 35% $SiO_2$ und 6% Phosphorsäuremonocetylester

Eine x g (salzfrei gewaschenem) Feststoff entsprechende Menge der Suspension aus Beispiel 1g wird auf 80°C erhitzt. In die Suspension werden unter Rühren 0,06x g Hostaphat CC100 (Phosphorsäuremonocetylester) der Fa. Clariant zugegeben und 30 Minuten bei konstanter Temperatur nachgerührt. Die Suspension wird heiß abfiltriert und mit Wasser auf eine Leitfähigkeit < 100 μS/cm gewaschen. Der erhaltene Filterkuchen wird getrocknet und desaggregiert.

Beispiel 5f: HT-Titandioxid mit 35% $SiO_2$ und 9% Phosphorsäuremonocetylester

Das aus Beispiel 1g erhaltene Material wird abfiltriert und mit Wasser auf eine Leitfähigkeit < 100 μS/cm gewaschen. Eine x g Feststoff entsprechende Menge des Filterkuchens wird mit etwas Wasser verdünnt und auf 80°C erhitzt. In die Suspension werden unter Rühren 0,09x g Hostaphat CC100 (Phosphorsäuremonocetylester) der Fa. Clariant zugegeben und 10 Minuten bei konstanter Temperatur nachgerührt. Die Suspension wird unter Rühren abgekühlt, getrocknet und desaggregiert.

Vergleichsbeispiel 6a: Titandioxid mit 20% $SiO_2$ und 10% Simethicone

Das aus Beispiel 1d erhaltene Material wird abfiltriert und mit Wasser auf eine Leitfähigkeit < 100 μS/cm gewaschen. Eine x g Feststoff entsprechende Menge des Filterkuchens wird mit etwas Wasser verflüssigt. In die Suspension werden unter starkem Rühren 0,33x g Sentry Simethicone Emulsion USP (enthält ca. 30% Simethicone) der

Fa. OSi zugegeben und 30 Minuten nachgerührt. Die Suspension wird getrocknet und desaggregiert.

Vergleichsbeispiel 6b: HT-Titandioxid mit 20% $SiO_2$ und 10% Simethicone

Das aus Beispiel 1f erhaltene Material wird abfiltriert und mit Wasser auf eine Leitfähigkeit < 100 μS/cm gewaschen. Eine x g Feststoff entsprechende Menge des Filterkuchens wird mit etwas Wasser verflüssigt. In die Suspension werden unter starkem Rühren 0,33x g Sentry Simethicone Emulsion USP (enthält ca. 30% Simethicone) der Fa. OSi zugegeben und 30 Minuten nachgerührt. Die Suspension wird getrocknet und desaggregiert.

Vergleichsbeispiel 6c: HT-Titandioxid mit 35% $SiO_2$ und 10% Simethicone

Das aus Beispiel 1g erhaltene Material wird abfiltriert und mit Wasser auf eine Leitfähigkeit < 100 μS/cm gewaschen. Eine x g Feststoff entsprechende Menge des Filterkuchens wird mit etwas Wasser verflüssigt. In die Suspension werden unter starkem Rühren 0,33x g Sentry Simethicone Emulsion USP (enthält ca. 30% Simethicone) der Fa. OSi zugegeben und 30 Minuten nachgerührt. Die Suspension wird getrocknet und desaggregiert.

Beispiel 7a: Titandioxid mit 12% $Al_2O_3$ und 10% Octylphosphonsäure

Eine x g $TiO_2$ entsprechende Menge des aus Beispiel 1a erhaltenen Materials wird bei 80°C mit einer Menge Natriumaluminat-Lösung (Konzentration ca. 260 g $Al_2O_3$/l) versetzt, die 0,12x g $Al_2O_3$ entspricht und der dabei steigende pH-Wert mit verdünnter Schwefelsäure konstant bei pH 7,0 gehalten.

Nach Zugabe der gesamten Aluminat-Lösung wird mit $H_2SO_4$ ein pH-Wert von 6,5 eingestellt und die Suspension 2 h bei der Temperatur und dem pH-Wert unter Rühren reifen gelassen. Die Suspension wird abfiltriert und mit Wasser auf eine Leitfähigkeit < 100 μS/cm gewaschen. Eine y g Feststoff entsprechende Menge des Filterkuchens wird mit etwas Wasser verflüssigt. In die Suspension werden unter starkem Rühren 0,1y g Octylphosphonsäure der Fa. Albright & Wilson zugegeben und 30 Minuten nachgerührt. Die Suspension wird getrocknet und desaggregiert.

Verqleichsbeispiel 7b: Titandioxid mit 12% $Al_2O_3$ und 10% Stearinsäure

Kommerzielles Produkt: EUSOLEX T-S der Fa. Merck

Vergleichsbeispiel 8a: Titandioxid mit 10% $SiO_2$ und 9% Methicone

Kommerzielles Produkt: UV-Titan X195 der Fa. Kemira (jetzt Sachtleben Chemie)

Vergleichsbeispiel 8b: Titandioxid mit 10% $SiO_2$ und 9% Methicone

Kommerzielles Produkt: UV-Titan M195 der Fa. Kemira (jetzt Sachtleben Chemie)

Vergleichsbeispiel 8d: Titandioxid mit 12% $SiO_2$ und 2% Dimethicone

Kommerzielles Produkt: Parsol TX der Fa. DSM

Vergleichsbeispiel 8e: Titandioxid mit 7% Aluminiumphosphat und 3% Polyvinylpyrrolidone (PVP)

Kommerzielles Produkt: UV-Titan M263 der Fa. Kemira (jetzt Sachtleben Chemie)

Vergleichsbeispiel 8f: Titandioxid mit 2,5% $SiO_2$ und Trimethoxycaprylsilan

Kommerzielles Produkt: Degussa T805 der Fa. Degussa (jetzt Evonik)

Vergleichsbeispiel 8q: Titandioxid mit $SiO_2$ und Dimethicone/Methicone Copolymer

Kommerzielles Produkt: T-Lite SF-S der Fa. BASF

Vergleichsbeispiel 8h: Titandioxid mit 10% $SiO_2$

Kommerzielles Produkt: UV-Titan M140 der Fa. Kemira (jetzt Sachtleben Chemie)

Vergleichsbeispiel 8i: Titandioxid mit 6% $Al_2O_3$ und 2% Dimethicone

Kommerzielles Produkt: UV-Titan M262 der Fa. Kemira (jetzt Sachtleben Chemie)

Vergleichsbeispiel 8i: Titandioxid mit 7% $Al_2O_3$ und 11% Methicone

Kommerzielles Produkt: UV-Titan M170 der Fa. Kemira (jetzt Sachtleben Chemie)

**Beispiel 9:**

Experimentelle Untersuchungen zum DHA-Abbau und/oder Verfärbungen der Zubereitungen enthaltend DHA und Titandioxid-Partikel, wie in den Tabellen angezeigt:
In allen Untersuchungen wurde die nachfolgend beschriebene O/W-Testformulierung verwendet:

| **Handelsname** | **INCI** | | |
|---|---|---|---|
| | | % | % |
| **A** | | | |
| **Erfindungsgemäßes $TiO_2$** | | ***5,00*** | ***1,00*** |
| Tego Care 150 | GLYCERYL STEARATE, STEARETH-25, CETETH-20; STEARYL ALCOHOL | 8,00 | 8,00 |
| Lanette O | *CETEARYL ALCOHOL* | *1,50* | *1,50* |
| Tegosoft liquid | *CETEARYL ETHYLHEXANOATE* | *5,00* | *5,00* |
| Miglyol 812N | *CAPRYC/CAPRYLIC TRIGL YVCERIDE* | *5,00* | *5,00* |
| Abil-Wax 2434 | *STEAROXY DIMETHICONE* | *1,00* | *1,00* |
| DC 200 (100cs) | *DIMETHICONE* | *0,50* | *0,50* |
| Propylparaben | *PROPYLPARABEN* | *0,05* | *0,05* |
| **B** | | | |
| 1,2-Propandiol | *PROPYLEN GLYCOL* | *3,00* | *3,00* |
| Methylparaben | *METHYL PARABEN* | *0,15* | *0,15* |

(fortgesetzt)

| | | | |
|---|---|---|---|
| **B** | | | |
| Wasser | *AQUA* | *55,80* | *62,80* |
| **C** | | | |
| **DHA** | ***DIHYDROXYACETONE*** | ***5,00*** | ***2,00*** |
| Wasser | *AQUA* | *10,00* | *10,00* |
| | | *100,00* | *100,00* |

Für den Lagerversuch wird die oben genannte Testformulierung mit 5 Gewichtsprozent DHA zusammen mit unterschiedlichen Titandioxid-Qualitäten untersucht. Sollte sich ein Lagerversuch auf die Testformulierung enthaltend 2 Gewichtsprozent DHA beziehen, so ist dies entsprechend angegeben.

Der DHA-Abbau in den untersuchten Testformulierungen wird wie folgt festgestellt:

DHA wird durch eine enzymatische Bestimmungsmethode analysiert und basiert auf der folgenden Reaktion:

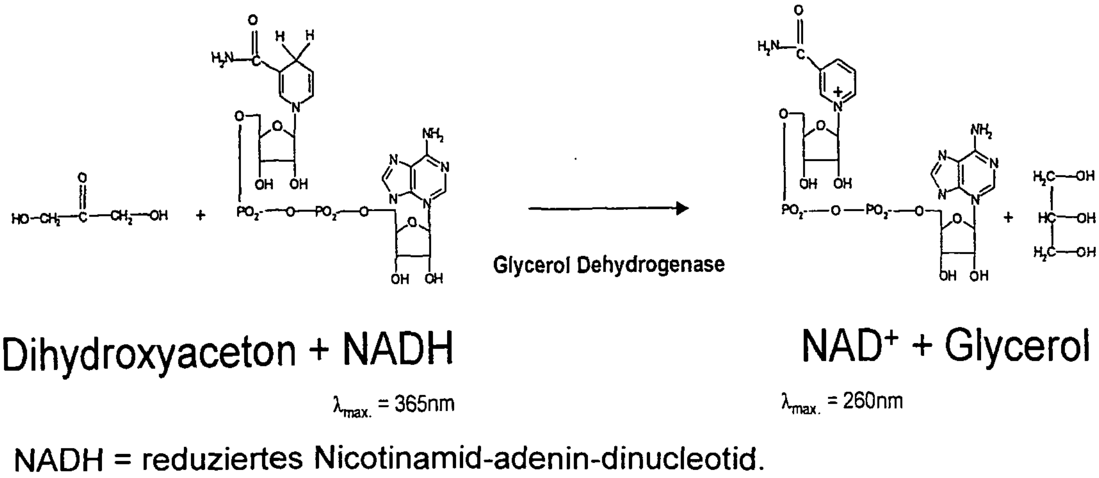


NADH = reduziertes Nicotinamid-adenin-dinucleotid.

Reagenzien:

Dihydroxyaceton (Merck), Glycylglycin (Merck), $(NH4)_2$ $SO_4$ (Merck), Sucrose (Merck), NADH-$Na_2$ (Merck), di-Natriumhydrogenphosphat-Dihydrat (Merck), Natriumdihydrogenphosphat-Dihydrat (Merck), Zinkchlorid (Merck), Glyceroldehydrogenase (Unitika Enzyms LTD).

DHA-Assay:

Die Bestimmung der DHA-Konzentration in einer Formulierung, basierend auf dem enzymatischen DHA-Assay, wie folgt beschrieben, basiert auf der folgenden Reaktion von Dihydroxyaceton:

DHA + NADH + H+ → NAD+ + Glycerol

NADH zeigt einen Absorptionskoeffizienten von 365 nm. Das Enzym Glycerol-Dehydrogenase katalysiert die oben genannte Reaktion des Dihydroxyacetons mit NADH. Je mehr DHA vorhanden ist, desto mehr NADH wird in der Reaktion umgesetzt und die NADH Absorption bei 365 nm nimmt ab.

A. **Probenpuffer-Lösung:** 1,06g Glycylglycin, 0,42g $(NH_4)_2$ $SO_4$ und 10,0g Sucrose werden in demineralisiertem Wasser gelöst und der pH-Wert wird mit NaOH-Lösung (10%) auf 9.0 eingestellt und die Probe auf ein Volumen von 100ml aufgefüllt.

B. **NADH - $Na_2$-Lösung:** 0.060 g NADH-$Na_2$ wird in 10.0 ml demineralisiertem Wasser gelöst.
C. **Puffer für die Glycerin Dehydrogenase-Lösung:**

a. 4.43 g Di-natriumhydrogenphosphat,
b. 3.90 g Natriumdihydrogenphosphat-Dihydrat,
c. 100.0 g Sucrose
d. 0.003 g Zinkchlorid.

Alle genannten Substanzen werden in 800 ml demineralisiertem Wasser gelöst und die Lösung mit 1 N Salzsäure oder Natronlauge auf einen pH-Wert von 7,0 eingestellt. Danach wird das Volumen der Probe mit demineralisiertem Wasser auf 1000ml aufgefüllt.
D. **Glycerin-Dehydrogenase-Lösung:** 10 mg Glycerindehydrogenase in 1ml GlycerinDehydrogenasePuffer (Lösung C) .
E. **DHA-Standards:** 0.200 g DHA (Art.-No. 1.10150 Merck) wird in 100 ml demineralisiertem Wasser gelöst. Diese Lösung wird 1:10 verdünnt. Der Standard wird zweifach, unabhängig von einander, hergestellt. Die Abweichung beider Standards vom therotischen DHA-Gehalt muss 100% +/- 5% betragen.

Probenzubereitung :

0.5 g - 1.0 g der Testemulsion wird in einen kalibrierten 100 ml Kolben eingewogen. 60 ml heisses (70°C) demineralisiertes Wasser wird eingefüllt und es wird gerührt, wobei die Temperatur bei 60°C für 15 Minuten konstant bleibt. Wenn die Lösung abgekühlt ist, wird bis zur Kalibrierungsmarke aufgefüllt. Die erhaltenen Lösungen werden über einen Membranfilter bis zur klaren Lösung filtriert. Von dieser Lösung wird ein Volumen von 0.1 ml zur DHA-Bestimmung verwendet.

Messbedingungen:

Cary 300 UV-Vis Spektrophotometer (Varian) (Wellenlänge = 365 nm, d = 1cm). Als Referenz wird demineralisiertes Wasser verwendet.

Piettenrozedur:

| | Leerwert Lösung | Probe / Standard-Lösung |
|---|---|---|
| Probenpuffer | 1.00 ml | 1.00 ml |
| NADH-$Na_2$ | 0.10 ml | 0.10 ml |
| Demineralisiertes Wasser | 2.00 ml | 1.90 ml |
| Probe oder Standard | ----- | 0.10 ml |

Die Absorption dieser Lösungen ist E1 (Messung 5 Minuten nach Mischung)
Die Lösungen werden wie oben beschrieben gemischt und vermessen bevor das Enzym hinzugegeben wird. Für jede Probe ist die Extinktion vor Enzymzugabe E1.
Nachfolgende Zugabe der Glycerin-Dehydrogenase-Lösung jeweils 0.01 ml.
Die Absorption dieser Lösungen ist E2 (Messung nach 15 Minuten nach Zugabe der Enzymlösung).

$$\Delta E = E1\text{-}E2$$

V = Totalvolumen : 3.11 ml

v = Probenvolumen : 0.1 ml

$\varepsilon = \varepsilon$ (NADH $Na_2$) = 3.42 $l^*mmol^{-1}*cm^{-1}$

MW = Molmasse (DHA) : 90.08 g / mol

EW = Probenlösungkonzentration (g/L)

$$\text{Gehalt von DHA [Gew.-\% in Testformulierung]} = \frac{\Delta E * V * MW * 100}{\varepsilon * d * v * 1000 * EW \text{ (g/L)}}$$

Mit dieser beschriebenen enzymbasierten Methode wird der Abbau in den Testformulierungen bestimmt, wie in den folgenden Beispielen angegeben:

Die Abkürzung RT entspricht dem Begriff Raumtemperatur.

Die verwendeten Formulierungen werden in Dunkelheit gelagert.

Beispiel 9A: DHA-Abbau von Testformulierungen

| Typ anorganischer Filter/DHA | Gehalt anorganischer UV-Filter | DHA-Gehalt | DHA-Gehalt Initialwert | DHA-Gehalt Nach 3 Monaten bei RT | DHA-Gehalt Nach 3 Monaten bei 40°C |
|---|---|---|---|---|---|
| **DHA (pur)** | 0% | 5% | 4,8 | 4,7 | 4,8 |
| **DHA (pur)** | 0% | 2% | 2,0 | 2,0 | 1,9 |
| **Parsol TX ($TiO_2$/$SiO_2$/ dimethicone)** | 5% | 5% | 4,7 | 3,5 | 0,9 |
| **Parsol TX ($TiO_2$/$SiO_2$/ dimethicone)** | 1% | 2% | 2,0 | 2,2 | 1,0 |
| **Sachtleben X265 ($TiO_2$ /$Al_2O_3$/ Triethoxycaprylyl silan)** | 5% | 5% | 5,0 | 3,0 | 0,3 |
| **Sachtleben X265 ($TiO_2$ /$Al_2O_3$/ Triethoxycaprylyl silan)** | 1% | 2% | 1,9 | 1,6 | 0,6 |
| **Erfindungsgemäß (35% $SiO_2$ + 6% Hostaphat CC100)** | 5% | 5% | 5,3 | 5,2 | 4,3 |
| **Erfindungsgemäß (35% $SiO_2$ + 6% Hostaphat CC100)** | 1% | 2% | 2,0 | 2,2 | 2,0 |
| **Erfindungsgemäß (20% $SiO_2$ + 6% Hostaphat CC100)** | 5% | 5% | 4,8 | 4,9 | 3,5 |
| **Erfindungsgemäß (20% $SiO_2$ + 6% Hostaphat CC100)** | 1% | 2% | 2,0 | 2,2 | 1,9 |

Die erfindungsgemäßen Titandioxid-Partikel von Beispiel 3A wurden zuvor hydrothermal behandelt.

Beispiel 9B:

Die untersuchten Testformulierungen werden in den folgenden Tabellen nummeriert, wobei die Zusammensetzung jeweils angegeben ist. Bei der Bezeichnung Wirkstoff aus Tensan AO handelt es sich um 1-Hydroxydodecane-1,1-diphosphonic Acid die bis zu ca. 75% im Produkt der Fa. Polygon Chemie enthalten ist.

Zusammenfassend kann festgestellt werden, dass die erfindungsgemäßen Titandioxid-Partikel einen deutlich geringeren DHA-Abbau bewirken im Vergleich zu bisher bekannten Verbindungen, wie zuvor beschrieben.

Tabelle 1 beschreibt die Testformulierungen der Beispiele 0, 1b, 2b, 4a, 4b, 4c, 4d, 4e, 4f, 4g, 4h, 4i, 4k und 6a, wobei die Vergleichsversuche entsprechend vermerkt sind. Es werden gleichzeitig Daten angegeben, die den DHA-Gehalt nach Lagerung bei 40°C in Dunkelheit nach 0,5 Monaten, nach einem Monat und nach 3 Monaten angeben. In der letzten Spalte wird jeweils der DHA-Abbau nach 3 Monaten bei 40°C in Dunkelheit angegeben, in Bezug auf den Abbau von DHA der gleichen Testformulierung ohne Titandioxid-Probe (dies entspricht dem Beispiel 0 der Tabelle und entspricht der Testformulierung des Beispiels 9 ohne Titandioxid-Probe).

Tabelle 2 beschreibt die Testformulierungen mit den Beispiel-Nummern 0, 1b, 2a, 3b, 5a, 5b, 5c, 5d, 5e, 5f, 6b und 6c, wobei die Vergleichsversuche entsprechend vermerkt sind. Es werden gleichzeitig Daten angegeben, die den DHA-Gehalt nach Lagerung bei 40°C in Dunkelheit nach 0,5 Monaten, nach einem Monat und nach 3 Monaten angeben. In der letzten Spalte wird jeweils der DHA-Abbau nach 3 Monaten bei 40°C in Dunkelheit angegeben, inBezug auf den Abbau von DHA der gleichen Testformulierung ohne Titandioxid-Probe.

Tabelle 3 beschreibt die Testformulierungen mit den Beispiel-Nummern 0, 4c, 5c, 5f, 7a, 7b, 8a, 8b, 8d, 8e, 8f, 8g, 8h, 8i und 8j, wobei die Vergleichsversuche entsprechend vermerkt sind. Es werden gleichzeitig Daten angegeben, die den DHA-Gehalt nach Lagerung bei 40°C in Dunkelheit nach 0,5 Monaten, nach einem Monat und nach 3 Monaten angeben. In der letzten Spalte wird jeweils der DHA-Abbau nach 3 Monaten bei 40°C in Dunkelheit angegeben, inBezug auf den Abbau von DHA der gleichen Testformulierung ohne Titandioxid-Probe.

Beispiel 9C:

Gleichzeitig wird beobachtet, dass die Verfärbung der Testformulierung durch den DHA-Abbau nach Lagerung deutlich reduziert wird. Dies belegen die experimentellen Daten der folgenden Tabellen.

Zur Bestimmung der Lab-Werte, insbesondere des Unterschieds in den b-Werten (delta b*) werden die Testformulierungen im Minolta Chromameter CR-400 (Lichtart C, Beobachter 2°) untersucht.

Die Messungen für die Berechnungen von delta b* und der k/s-Werte (Tabellen 4 bis 9) wird wie folgt durchgeführt:

Geräte und Reagenzien

1. Probenteller, Kunststoff (Eigenproduktion)
2. Quarzglasplatten
3. Farbmessgerät (z. B. X-Rite Color Eye 7000) Lichtart C, Beobachter 2°

**Durchführung**

Die zu messende Probe wird in den Probenteller [1] gefüllt. Es ist darauf zu achten, dass sich möglichst keine Luftblasen in der Probe befinden. Die Probe wird mit einer Quarzglasplatte [2] abgedeckt und so der Luftkontakt unterbunden. Danach wird die Probe mit einem Farbmessgerät [3] (Lichtart C, 2° Normalbeobachter) vermessen.
Aus den Spektraldaten werden die L*a*b* Werte [DIN 6174] sowie die k/s - Werte nach Kubelka-Munk berechnet.

Die k/s-Werte werden wie folgt berechnet:

$$k/s\ [\lambda] = (1 - [\%\ \text{Remission bei}\ \lambda]/100)^2\ /\ 2 \times [\%\ \text{Remission bei}\ \lambda]/100$$

k = Absorptionskoeffizient
s = Streukoeffizient
$\lambda$ = Wellenlänge

Die Stärke der Farbreaktion ergibt sich aus:

$$\text{Farbreaktion [DHA]} = k/s\ [450\text{nm}] - k/s\ [700\text{nm}]$$

Generell ist festzuhalten, dass die erfindungsgemäßen Titandioxid-Partikel die Testformulierung visuell weniger verändern, d.h. dass eine geringere Verfärbung stattfindet im Vergleich zu den bekannten Titandioxid-Partikeln des Standes der Technik.

Tabelle 4 beschreibt die Testformulierungen mit den Beispiel-Nummern 0, 1b, 2b, 4a, 4b, 4c, 4d, 4e, 4f, 4g, 4h, 4i, 4k und 6a, wobei die Vergleichsversuche entsprechend vermerkt sind. Es werden gleichzeitig Daten für delta b* und k/s-Werte angegeben, die dem Grad der Verfärbung angeben.

Tabelle 5 beschreibt die Testformulierungen mit den Beispiel-Nummern 0, 1b, 2a, 3b, 5a, 5b, 5c, 5d, 5e, 5f, 6b und 6c, wobei die Vergleichsversuche entsprechend vermerkt sind. Es werden gleichzeitig Daten für delta b* und k/s-Werte angegeben, die dem Grad der Verfärbung angeben.

Tabelle 6 beschreibt die Testformulierungen mit den Beispiel-Nummern 0, 4c, 5c, 5f, 7a, 7b, 8a, 8b, 8d, 8e, 8f, 8g, 8h, 8i und 8j, wobei die Vergleichsversuche entsprechend vermerkt sind. Es werden gleichzeitig Daten für delta b* und k/s-Werte angegeben, die dem Grad der Verfärbung angeben.

Beispiel 9D: Im folgenden werden Werte für die Testformulierungen gelistet, die die Verfärbungen der Testformulierungen im Ascorbylpalmitat-Test angeben, dargestellt durch die angegebenen k/s-Werte.

Der Ascorbylpalmitattest wird wie folgt durchgeführt:

Geräte

1. Uhrgläser
2. Analysenwaage (z.B. Mettler AE200)
3. Wägepinsel
4. elastischer Apothekerspatel
5. Farbenausreibmaschine
6. Spritze mit 0,1ml Volumeneinteilung (z.B. Omnifix 2ml)
7. Halter für Quarzglasplatte mit Paste,
8. Quarzglasplatten
9. Farbmessgerät (z.B. X-rite Color Eye 7000)

Chemikalien

A Ascorbylpalmiat (z.B. Fa. Merck Materialnummer 5.00090.9999)
B Testmaterial (z.B. Titandioxid-Probe)
C $C_{12}$-$C_{15}$ Alkyl Benzoate (z.B. Tegosoft TN, Fa. Evonik)

Durchführung

Einwaage

| | | |
|---|---|---|
| 0,0250 g | Ascorbylpalmitat | [A] |
| 1,000 g | Testmaterial (z.B. Titandioxid-Probe) | [B] |
| 0,5-4,0ml | $C_{12}$-$C_{15}$ Alkyl Benzoate | [C] |

Herstellung

Das Ascorbylpalmitat [A] und das Testmaterial [B] werden jeweils in ein passendes Uhrglas [1] eingewogen. 0,5ml $C_{12}$-$C_{15}$ Alkyl Benzoate [C] wird mittels Spritze [6] in die Mitte der unteren Platte der Farbenausreibmaschine [5] gegeben. Das Testmaterial wird mittels Pinsel [3] in das $C_{12}$-$C_{15}$ Alkyl Benzoate gegeben und mittels Spatel [4] angepastet. Falls dazu weiteres $C_{12}$-$C_{15}$ Alkyl Benzoate nötig ist, wird es in 0,1ml-Schritten mittels Spritze hinzugegeben. Der Gesamtverbrauch an $C_{12}$-$C_{15}$ Alkyl Benzoate wird festgehalten. Sobald eine Paste vorliegt, wird das Ascorbylpalmitat mittels Pinsel auf diese gegeben und ebenfalls mittels Spatel eingepastet. Es erfolgen 4 Anreibungen ohne Gewichte mit jeweils 25 Umdrehungen. Nach jeder Anreibung wird die Paste in die Mitte der unteren Anreibplatte mittels Spatel zurückgeführt.

Anschließend wird die Probe in eine Probendose gefüllt und somit Dunkel gestellt, damit keine Reaktion mit

dem Tageslicht erfolgt, welches die Messwerte verfälschen könnte.

Messung

Die Messung der Spektraldaten erfolgt nach 24 h Dunkellagerung nach der Herstellung.

Dazu wird der Probenansatz mit einem Spatel homogen aufgerührt und in die Mitte der Quarzglasplatte [8] gegeben. Die Quarzglasplatte wird zur Messung mit einem Halter [7] versehen. Danach wird die Probe mit einem Farbmessgerät [9] (ohne Glanz, mit UV, Lichtart C, 2° Normalbeobachter) vermessen.

Die Bedienung des Farbmessgerätes wird als bekannt vorausgesetzt, bzw. ist der jeweiligen Bedienungsanleitung zu entnehmen.

**Auswertung**

Aus den Spektraldaten werden die L*a*b* Werte sowie die k/s -Werte berechnet. Die k/s-Werte werden wie folgt berechnet:

$$k/s\,[\lambda] = (1 - [\%\ \text{Remission bei}\ \lambda]/100)^2 / 2 \times [\%\ \text{Remission bei}\ \lambda]/100$$

k = Absorptionskoeffizient
s = Streukoeffizient
$\lambda$ = Wellenlänge

Die Stärke der Wechselwirkung mit Ascorbylpalmitat ergibt sich aus:

$$\text{Wechselwirkung [Ascorbylpalmitat]} = k/s\,[430\text{nm}] - k/s\,[700\text{nm}]$$

k/s [700nm] ist der Bereich, in dem keine Wechselwirkungen mit Ascorbylpalmitat auftreten. Um die Beiträge der geprüften Probe, sowie des $C_{12}$-$C_{15}$ Alkyl Benzoate zum k/s-Wert zu berücksichtigen, wird dieser Wert subtrahiert und fungiert als Basislinie.

Tabelle 7 beschreibt die Testformulierungen mit den Beispiel-Nummern 0, 1b, 2b, 4a, 4b, 4c, 4d, 4e, 4f, 4g, 4h, 4i, 4k und 6a, wobei die Vergleichsversuche entsprechend vermerkt sind. Es werden gleichzeitig die ermittelten k/s-Werte angegeben, die die Verfärbungen im Ascorbylpalmitat-Test nach 24 Stunden angegeben.

Tabelle 8 beschreibt die Testformulierungen mit den Beispiel-Nummern 0, 1b, 2a, 3b, 5a, 5b, 5c, 5d, 5e, 5f, 6b und 6c, wobei die Vergleichsversuche entsprechend vermerkt sind. Es werden gleichzeitig die ermittelten k/s-Werte angegeben, die die Verfärbungen im Ascorbylpalmitat-Test nach 24 Stunden angegeben.

Tabelle 9 beschreibt die Testformulierungen mit den Beispiel-Nummern 0, 4c, 5c, 5f, 7a, 7b, 8a, 8b, 8d, 8e, 8f, 8g, 8h, 8i und 8j, wobei die Vergleichsversuche entsprechend vermerkt sind. Es werden gleichzeitig die ermittelten k/s-Werte angegeben, die die Verfärbungen im Ascorbylpalmitat-Test nach 24 Stunden angegeben.

| Beispiel Nr. | | HT | %SiO2 | % Phosphorsäuremonocetylester (C16) [Hostaphat CC100] | % Phosphorsäure-mono – und di-stearyl ester Mischung [Hostaphat CS120] | % 1-Hydroxyethan-1,1-diphosphonsäure [Cublen K 60] | 2-Phosphonobutan-1,2,4-tricarbonsäure [Cublen P 50 ] | % Aminotrismethylenphosphonsäur [Cublen AP 5] | % Laurylphosphonsäure (Rhodia) | % Octylphosphonsäure (Rhodia) | % Wirkstoff aus Tensan AO | % simethicone | DHA-Gehalt ungelagert | DHA-Gehalt nach 0,5 Monaten bei 40 °C | DHA-Gehalt nach 1 Monaten bei 40 °C | **DHA-Gehalt nach 3 Monaten bei 40 °C** | DHA-Abbau nach 0,5 Monaten bei 40 °C | DHA-Abbau nach 1 Monaten bei 40 °C | DHA-Abbau nach 3 Monaten bei 40 °C | **DHA-Abbau nach 3 Monaten bei 40 °C [Bezug Abbau DHA ohne TiO2-Probe]** |
|---|---|---|---|---|---|---|---|---|---|---|---|---|---|---|---|---|---|---|---|---|
| **0** | | - | - | - | - | - | - | - | - | - | - | - | 5,8 % | 5,5 % | 5,5 % | **4,8 %** | 5 % | 5 % | 17 % | **0 %** |
| **1b** | Vergleichsbeispiel | nein | - | - | - | - | - | - | - | - | - | - | 5,7 % | 0,3 % | 0,0 % | **0,0 %** | 96 % | 100 % | 100 % | **83 %** |
| **2b** | Vergleichsbeispiel | nein | 35 | - | - | - | - | - | - | - | - | - | 5,7 % | 2,9 % | 2,1 % | **1,1 %** | 49 % | 63 % | 81 % | **63 %** |
| **4a** | | nein | 35 | 5 | - | - | - | - | - | - | - | - | 6,0 % | 4,1 % | 3,0 % | **2,0 %** | 32 % | 50 % | 67 % | **49 %** |
| **4b** | | nein | 35 | 10 | - | - | - | - | - | - | - | - | 5,7 % | 5,4 % | 5,0 % | **4,3 %** | 5 % | 12 % | 25 % | **7 %** |
| **4c** | | nein | 35 | 15 | - | - | - | - | - | - | - | - | 5,9 % | 5,6 % | 5,2 % | **4,4 %** | 5 % | 12 % | 25 % | **8 %** |
| **4d** | | nein | 35 | - | 15 | - | - | - | - | - | - | - | 5,9 % | 5,7 % | 5,4 % | **4,4 %** | 3 % | 8 % | 25 % | **8 %** |
| **4e** | | nein | 35 | - | - | 15 | - | - | - | - | - | - | 5,7 % | 5,4 % | 5,5 % | **4,0 %** | 5 % | 4 % | 30 % | **13 %** |
| **4f** | | nein | 35 | - | - | - | 15 | - | - | - | - | - | 5,7 % | 5,1 % | 5,1 % | **3,8 %** | 11 % | 11 % | 33 % | **16 %** |
| **4g** | | nein | 35 | - | - | - | - | 15 | - | - | - | - | 5,7 % | 5,3 % | 5,1 % | **3,8 %** | 7 % | 11 % | 33 % | **16 %** |
| **4h** | | nein | 35 | - | - | - | - | - | 15 | - | - | - | 5,7 % | 5,6 % | 5,4 % | **4,2 %** | 2 % | 5 % | 26 % | **9 %** |
| **4i** | | nein | 35 | - | - | - | - | - | - | 15 | - | - | 5,7 % | 5,2 % | 5,1 % | **4,2 %** | 9 % | 11 % | 26 % | **9 %** |
| **4k** | | nein | 35 | - | - | - | - | - | - | - | 5 | - | 5,7 % | 5,2 % | 4,9 % | **3,8 %** | 9 % | 14 % | 33 % | **16 %** |
| **6a** | Vergleichsbeispiel | nein | 20 | - | - | - | - | - | - | - | - | 10 | 5,7 % | 3,7 % | 3,1 % | **2,0 %** | 35 % | 46 % | 65 % | **48 %** |

Tabelle 1: Angabe des DHA-Gehalts nach Lagerung bei 40°C (Dunkelheit)

| **Beispiel Nr.** | | HT | %SiO2 | % Phosphorsäuremonocetylester (C16) [Hostaphat CC100] | % simethicone | DHA-Gehalt ungelagert | DHA-Gehalt nach 0,5 Monaten bei 40 °C | DHA-Gehalt nach 1 Monaten bei 40 °C | **DHA-Gehalt nach 3 Monaten bei 40 °C** | DHA-Abbau nach 0,5 Monaten bei 40 °C | DHA-Abbau nach 1 Monaten bei 40 °C | DHA-Abbau nach 3 Monaten bei 40 °C | **DHA-Abbau nach 3 Monaten bei 40 °C [Bezug Abbau DHA ohne TiO2-Probe]** |
|---|---|---|---|---|---|---|---|---|---|---|---|---|---|
| **0** | | - | - | - | - | 5,8 % | 5,5 % | 5,5 % | **4,8 %** | 5 % | 5 % | 17 % | **0 %** |
| **1b** | Vergleichsbeispiel | nein | - | - | - | 5,7 % | 0,3 % | 0,0 % | **0,0 %** | 96 % | 100 % | 100 % | **83 %** |
| **2a** | Vergleichsbeispiel | ja | 20 | - | - | 6,1 % | 4,6 % | 3,8 % | **2,4 %** | 25 % | 38 % | 61 % | **43 %** |
| **3b** | Vergleichsbeispiel | ja | 35 | - | - | 5,7 % | 4,5 % | 4,2 % | **3,6 %** | 21 % | 26 % | 37 % | **20 %** |
| **5a** | | ja | 20 | 3 | - | 5,7 % | - | 3,5 % | **2,8 %** | - | 39 % | 51 % | **34 %** |
| **5b** | | ja | 20 | 6 | - | 4,9 % | 4,7 % | 4,8 % | **4,0 %** | 4 % | 2 % | 18 % | **1 %** |
| **5c** | | ja | 20 | 9 | - | 5,7 % | - | 4,6 % | **4,7 %** | - | 19 % | 18 % | **0 %** |
| **5d** | | ja | 35 | 3 | - | 5,7 % | 3,0 % | 2,8 % | **2,1 %** | 47 % | 51 % | 63 % | **46 %** |
| **5e** | | ja | 35 | 6 | - | 5,4 % | 5,2 % | 5,2 % | **4,4 %** | 4 % | 4 % | 19 % | **1 %** |
| **5f** | | ja | 35 | 9 | - | 5,7 % | 4,8 % | 4,7 % | **4,3 %** | 16 % | 18 % | 25 % | **7 %** |
| **6b** | Vergleichsbeispiel | ja | 20 | - | 10 | 5,7 % | 3,9 % | 3,1 % | **1,7 %** | 32 % | 46 % | 70 % | **53 %** |
| **6c** | Vergleichsbeispiel | ja | 35 | - | 10 | 5,7 % | 4,6 % | 4,2 % | **3,7 %** | 19 % | 26 % | 35 % | **18 %** |

Tabelle 2: Angabe des DHA-Gehalts nach Lagerung bei 40°C (Dunkelheit)

| | Auswahl | **Beispiel Nr.** | HT | %SiO2 | % Phosphorsäuremonocetylester (C16) [Hostaphat CC100] | % Octylphosphonsäure (Rhodia) | % Al2O3 | Beschichtung | DHA-Gehalt ungelagert | DHA-Gehalt nach 0,5 Monaten bei 40 °C | DHA-Gehalt nach 1 Monaten bei 40 °C | DHA-Gehalt nach 3 Monaten bei 40 °C | DHA-Abbau nach 0,5 Monaten bei 40 °C | DHA-Abbau nach 1 Monaten bei 40 °C | DHA-Abbau nach 3 Monaten bei 40 °C | DHA-Abbau nach 3 Monaten bei 40 °C [Bezug Abbau DHA ohne TiO2-Probe] |
|---|---|---|---|---|---|---|---|---|---|---|---|---|---|---|---|---|
| | ja | **0** | - | - | - | | | | 5,8 % | 5,5 % | 5,5 % | **4,8 %** | 5 % | 5 % | 17 % | **0 %** |
| | ja | **4c** | nein | 35 | 15 | | | | 5,9 % | 5,6 % | 5,2 % | **4,4 %** | 5 % | 12 % | 25 % | **8 %** |
| | ja | **5c** | ja | 20 | 9 | | | | 5,7 % | - | 4,6 % | **4,7 %** | - | 19 % | 18 % | **0 %** |
| | ja | **5f** | ja | 35 | 9 | | | | 5,7 % | 4,8 % | 4,7 % | **4,3 %** | 16 % | 18 % | 25 % | **7 %** |
| | ja | **7a** | nein | | | 10 | 12 | | 5,7 % | 5,5 % | 5,2 % | **4,6 %** | 4 % | 9 % | 19 % | **2 %** |
| Eusolex T-S | ja | **7b** | nein | | | | 12 | 10% Stearinsäure | 5,7 % | 3,2 % | 1,6 % | **0,4 %** | 44 % | 72 % | 93 % | **76 %** |
| UV-Titan X195 | ja | **8a** | nein | 10 | | | | 9% Methicone | 5,7 % | 3,5 % | 2,8 % | **1,8 %** | 39 % | 51 % | 68 % | **51 %** |
| UV-Titan M195 | ja | **8b** | nein | 10 | | | | 9% Methicone | 5,0 % | 3,6 % | 2,8 % | **1,7 %** | 28 % | 44 % | 66 % | **49 %** |
| Parsol TX | ja | **8d** | nein | 12 | | | | 2% Dimethicone | 5,2 % | 3,5 % | 2,8 % | **1,8 %** | 33 % | 46 % | 65 % | **48 %** |
| UV-Titan M263 | ja | **8e** | nein | | | | | 7% Al-phosphat + 3% PVP | 5,7 % | 2,7 % | 1,7 % | **0,3 %** | 53 % | 70 % | 96 % | **78 %** |
| Degussa T805 | ja | **8f** | nein | 2,5 | | | | Trimethoxycaprylsilane | 5,7 % | 4,6 % | 3,7 % | **1,7 %** | 19 % | 35 % | 70 % | **53 %** |
| T-Lite SF-S | ja | **8g** | nein | X | | | X | Dimethicone/ Methicone Copolymer | 5,1 % | 3,4 % | 2,3 % | **0,7 %** | 33 % | 55 % | 86 % | **69 %** |
| UV-Titan M140 | ja | **8h** | nein | 10 | | | | | 4,9 % | 2,1 % | 1,3 % | **0,3 %** | 57 % | 73 % | 95 % | **78 %** |
| UV-Titan M262 | ja | **8i** | nein | | | | 6 | 2% Dimethicone | 4,8 % | 2,4 % | 1,6 % | **0,3 %** | 50 % | 67 % | 95 % | **78 %** |
| UV-Titan M170 | ja | **8j** | nein | | | | 7 | 11% Methicone | 4,9 % | 2,6 % | 1,3 % | **0,3 %** | 47 % | 73 % | 95 % | **78 %** |

Tabelle 3: Angabe des DHA-Gehalts nach Lagerung bei 40°C (Dunkelheit)

| Beispiel Nr. | | HT | %SiO2 | % Phosphorsäuremonocetylester (C16) [Hostaphat CC100] | % Phosphorsäure-mono – und di-stearyl ester Mischung [Hostaphat CS120] | % 1-Hydroxyethan-1,1-diphosphonsäure [Cublen K 60] | 2-Phosphonobutan-1,2,4-tricarbonsäure [Cublen P 50 ] | % Aminotrismethylenphosphonsäur [Cublen AP 5] | % Laurylphosphonsäure (Rhodia) | % Octylphosphonsäure (Rhodia) | % Wirkstoff aus Tensan AO | % simethicone | delta b* ungelagert | delta b* nach 0,5 Monaten bei 40 °C | delta b* nach 1 Monaten bei 40 °C | **delta b* nach 3 Monaten bei 40 °C** | ungelagert | k/s-Werte nach 0,5 Monaten bei 40 °C | k/s-Werte nach 1 Monaten bei 40 °C | **k/s-Werte nach 3 Monaten bei 40 °C** |
|---|---|---|---|---|---|---|---|---|---|---|---|---|---|---|---|---|---|---|---|---|
| **0** | | - | - | - | - | - | - | - | - | - | - | - | 0 | -0,4 | -0,3 | **0,7** | -0,04 | -0,05 | -0,05 | **-0,04** |
| **1b** | Vergleichsbeispiel | nein | - | - | - | - | - | - | - | - | - | - | 0 | 7,0 | 8,6 | **11,5** | 0,37 | 2,02 | 2,42 | **3,80** |
| **2b** | Vergleichsbeispiel | nein | 35 | - | - | - | - | - | - | - | - | - | 0 | 7,7 | 10,4 | **17,6** | -0,02 | 0,11 | 0,21 | **0,50** |
| **4a** | | nein | 35 | 5 | - | - | - | - | - | - | - | - | 0 | 3,2 | 5,8 | **13,7** | 0,00 | 0,04 | 0,09 | **0,39** |
| **4b** | | nein | 35 | 10 | - | - | - | - | - | - | - | - | 0 | 2,2 | 3,0 | **5,6** | 0,00 | 0,02 | 0,03 | **0,08** |
| **4c** | | nein | 35 | 15 | - | - | - | - | - | - | - | - | 0 | 0,5 | 1,1 | **2,1** | 0,01 | 0,01 | 0,02 | **0,03** |
| **4d** | | nein | 35 | - | 15 | - | - | - | - | - | - | - | 0 | 1,0 | 2,0 | **1,1** | 0,00 | 0,00 | 0,02 | **0,01** |
| **4e** | | nein | 35 | - | - | 15 | - | - | - | - | - | - | 0 | 0,5 | 2,0 | **2,8** | -0,01 | 0,00 | 0,01 | **0,01** |
| **4f** | | nein | 35 | - | - | - | 15 | - | - | - | - | - | 0 | 1,3 | 1,5 | **3,0** | -0,01 | 0,01 | 0,01 | **0,02** |
| **4g** | | nein | 35 | - | - | - | - | 15 | - | - | - | - | 0 | 0,3 | 4,2 | **10,0** | 0,00 | 0,00 | 0,03 | **0,10** |
| **4h** | | nein | 35 | - | - | - | - | - | 15 | - | - | - | 0 | 0,4 | 1,9 | **3,8** | 0,00 | 0,01 | 0,02 | **0,04** |
| **4i** | | nein | 35 | - | - | - | - | - | - | 15 | - | - | 0 | 0,2 | -0,1 | **2,1** | 0,01 | 0,01 | 0,01 | **0,03** |
| **4k** | | nein | 35 | - | - | - | - | - | - | - | 5 | - | 0 | 0,7 | 1,9 | **6,4** | 0,00 | 0,01 | 0,02 | **0,10** |
| **6a** | Vergleichsbeispiel | nein | 20 | - | - | - | - | - | - | - | - | 10 | 0 | 8,3 | 16,3 | **23,2** | -0,02 | 0,13 | 0,31 | **1,10** |

Tabelle 4: Verfärbungen durch DHA-Abbau nach Lagerung bei 40°C (Dunkelheit)

| Beispiel Nr. | | HT | %SiO2 | % Phosphorsäuremonocetylester (C16) [Hostaphat CC100] | % simethicone | delta b* ungelagert | delta b* nach 0,5 Monaten bei 40 °C | delta b* nach 1 Monaten bei 40 °C | **delta b* nach 3 Monaten bei 40 °C** | ungelagert | k/s-Werte nach 0,5 Monaten bei 40 °C | k/s-Werte nach 1 Monaten bei 40 °C | **k/s-Werte nach 3 Monaten bei 40 °C** |
|---|---|---|---|---|---|---|---|---|---|---|---|---|---|
| **0** | | - | - | - | - | 0 | -0,4 | -0,3 | **0,7** | -0,04 | -0,05 | -0,05 | **-0,04** |
| **1b** | Vergleichsbeispiel | nein | - | - | - | 0 | 7,0 | 8,6 | **11,5** | 0,37 | 2,02 | 2,42 | **3,80** |
| **2a** | Vergleichsbeispiel | ja | 20 | - | - | 0 | 3,0 | 5,4 | **10,6** | -0,01 | 0,02 | 0,06 | **0,16** |
| **3b** | Vergleichsbeispiel | ja | 35 | - | - | 0 | 2,3 | 3,4 | **8,9** | -0,01 | 0,01 | 0,02 | **0,079** |
| **5a** | | ja | 20 | 3 | - | 0 | 2,5 | 5,0 | **13,4** | -0,01 | 0,01 | 0,04 | **0,22** |
| **5b** | | ja | 20 | 6 | - | 0 | 0,1 | 0,8 | **1,4** | -0,01 | -0,01 | -0,01 | **0,00** |
| **5c** | | ja | 20 | 9 | - | 0 | 0,0 | 0,9 | **0,8** | 0,00 | 0,00 | 0,01 | **-0,03** |
| **5d** | | ja | 35 | 3 | - | 0 | 2,5 | 4,8 | **12,6** | -0,01 | 0,01 | 0,04 | **0,22** |
| **5e** | | ja | 35 | 6 | - | 0 | -0,3 | 0,8 | **-1,7** | -0,01 | -0,01 | 0,00 | **0,01** |
| **5f** | | ja | 35 | 9 | - | 0 | 1,5 | 2,3 | **1,3** | -0,01 | 0,00 | 0,01 | **-0,01** |
| **6b** | Vergleichsbeispiel | ja | 20 | - | 10 | 0 | 6,7 | 9,1 | **18,1** | -0,01 | 0,08 | 0,12 | **0,47** |
| **6c** | Vergleichsbeispiel | ja | 35 | - | 10 | 0 | 2,2 | 3,1 | **8,8** | -0,02 | 0,00 | 0,01 | **0,072** |

Tabelle 5: Verfärbungen durch DHA-Abbau nach Lagerung bei 40°C (Dunkelheit)

| | Auswahl | Beispiel Nr. | HT | %SiO2 | % Phosphorsäuremonocetylester (C16) [Hostaphat CC100] | % Octylphosphonsäure (Rhodia) | % Al2O3 | Beschichtung | delta b* ungelagert | delta b* nach 0,5 Monaten bei 40 °C | delta b* nach 1 Monaten bei 40 °C | delta b* nach 3 Monaten bei 40 °C | ungelagert | k/s-Werte nach 0,5 Monaten bei 40 °C | k/s-Werte nach 1 Monaten bei 40 °C | k/s-Werte nach 3 Monaten bei 40 °C |
|---|---|---|---|---|---|---|---|---|---|---|---|---|---|---|---|---|
| | ja | 0 | - | - | - | | | | 0 | -0,4 | -0,3 | 0,7 | -0,04 | -0,05 | -0,05 | -0,04 |
| | ja | 4c | nein | 35 | 15 | | | | 0 | 0,5 | 1,1 | 2,1 | 0,01 | 0,01 | 0,02 | 0,03 |
| | ja | 5c | ja | 20 | 9 | | | | 0 | 0,0 | 0,9 | 0,8 | 0,00 | 0,00 | 0,01 | -0,03 |
| | ja | 5f | ja | 35 | 9 | | | | 0 | 1,5 | 2,3 | 1,3 | -0,01 | 0,00 | 0,01 | -0,01 |
| | ja | 7a | nein | | | 10 | 12 | | 0 | -1,3 | -2,4 | 0,1 | 0,02 | -0,01 | -0,03 | 0,00 |
| Eusolex T-S | ja | 7b | nein | | | | 12 | 10% Stearinsäure | 0 | 9,5 | 14,5 | 23,5 | 0,01 | 0,21 | 0,59 | 2,67 |
| UV-Titan X195 | ja | 8a | nein | 10 | | | | 9% Methicone | 0 | 5,6 | 11,3 | 19,1 | 0,00 | 0,06 | 0,18 | 0,61 |
| UV-Titan M195 | ja | 8b | nein | 10 | | | | 9% Methicone | 0 | 5,1 | 8,1 | 14,3 | -0,01 | 0,04 | 0,10 | 0,30 |
| Parsol TX | ja | 8d | nein | 12 | | | | 2% Dimethicone | 0 | 6,4 | 9,9 | 15,7 | 0,00 | 0,07 | 0,14 | 0,40 |
| UV-Titan M263 | ja | 8e | nein | | | | | 7% Al-phosphat + 3% PVP | 0 | 4,6 | 11,9 | 21,7 | 0,00 | 0,04 | 0,16 | 0,90 |
| Degussa T805 | ja | 8f | nein | 2,5 | | | | Trimethoxycaprylsilane | 0 | 2,9 | 7,6 | 13,3 | 0,00 | 0,03 | 0,12 | 0,37 |
| T-Lite SF-S | ja | 8g | nein | X | | | X | Dimethicone/ Methicone Copolymer | 0 | 2,9 | 12,2 | 26,5 | 0,00 | 0,03 | 0,20 | 1,25 |
| UV-Titan M140 | ja | 8h | nein | 10 | | | | | 0 | 17,0 | 23,0 | 27,1 | -0,02 | 0,59 | 1,30 | 4,13 |
| UV-Titan M262 | ja | 8i | nein | | | | 6 | 2% Dimethicone | 0 | 6,4 | 11,8 | 20,2 | 0,00 | 0,06 | 0,19 | 0,88 |
| UV-Titan M170 | ja | 8j | nein | | | | 7 | 11% Methicone | 0 | 11,9 | 18,8 | 25,4 | 0,00 | 0,25 | 0,81 | 2,52 |

Tabelle 6: Verfärbungen durch DHA-Abbau nach Lagerung bei 40°C (Dunkelheit)

| Beispiel Nr. | | HT | %SiO2 | % Phosphorsäuremonocetylester (C16) [Hostaphat CC100] | % Phosphorsäure-mono – und di-stearyl ester Mischung [Hostaphat CS120] | % 1-Hydroxyethan-1,1-diphosphonsäure [Cublen K 60] | 2-Phosphonobutan-1,2,4-tricarbonsäure [Cublen P 50 ] | % Aminotrismethylenphosphonsäur [Cublen AP 5] | % Laurylphosphonsäure (Rhodia) | % Octylphosphonsäure (Rhodia) | % Wirkstoff aus Tensan AO | % simethicone | Ascorbylpalmitat-Test k/s [430nm] | Ascorbylpalmitat-Test k/s [750nm] | Ascorbylpalmitat-Test k/s (430nm - 750nm) |
|---|---|---|---|---|---|---|---|---|---|---|---|---|---|---|---|
| 0 | | - | - | - | - | - | - | - | - | - | - | - | - | - | - |
| 1b | Vergleichsbeispiel | nein | - | - | - | - | - | - | - | - | - | - | 2,92 | 0,23 | 2,69 |
| 2b | Vergleichsbeispiel | nein | 35 | - | - | - | - | - | - | - | - | - | 0,67 | 0,11 | 0,56 |
| 4a | | nein | 35 | 5 | - | - | - | - | - | - | - | - | 0,64 | 0,10 | 0,54 |
| 4b | | nein | 35 | 10 | - | - | - | - | - | - | - | - | 0,38 | 0,09 | 0,29 |
| 4c | | nein | 35 | 15 | - | - | - | - | - | - | - | - | 0,47 | 0,13 | 0,34 |
| 4d | | nein | 35 | - | 15 | - | - | - | - | - | - | - | 0,25 | 0,07 | 0,18 |
| 4e | | nein | 35 | - | - | 15 | - | - | - | - | - | - | 0,15 | 0,08 | 0,07 |
| 4f | | nein | 35 | - | - | - | 15 | - | - | - | - | - | 0,21 | 0,07 | 0,14 |
| 4g | | nein | 35 | - | - | - | - | 15 | - | - | - | - | 0,18 | 0,07 | 0,11 |
| 4h | | nein | 35 | - | - | - | - | - | 15 | - | - | - | 0,18 | 0,08 | 0,10 |
| 4i | | nein | 35 | - | - | - | - | - | - | 15 | - | - | 0,15 | 0,08 | 0,08 |
| 4k | | nein | 35 | - | - | - | - | - | - | - | 5 | - | 0,33 | 0,08 | 0,25 |
| 6a | Vergleichsbeispiel | nein | 20 | - | - | - | - | - | - | - | - | 10 | 0,81 | 0,11 | 0,69 |

Tabelle 7: Verfärbungen Ascorbylpalmitat-Test nach 24h

| Beispiel Nr. | | HT | %SiO2 | % Phosphorsäuremonocetylester (C16) [Hostaphat CC100] | % simethicone | Ascorbylpalmitat-Test k/s [430nm] | Ascorbylpalmitat-Test k/s [750nm] | **Ascorbylpalmitat-Test k/s (430nm - 750nm)** |
|---|---|---|---|---|---|---|---|---|
| **0** | | - | - | - | - | - | - | **-** |
| **1b** | Vergleichsbeispiel | nein | - | - | - | 2,92 | 0,23 | **2,69** |
| **2a** | Vergleichsbeispiel | ja | 20 | - | - | - | - | **-** |
| **3b** | Vergleichsbeispiel | ja | 35 | - | - | 0,35 | 0,12 | **0,24** |
| **5a** | | ja | 20 | 3 | - | 0,38 | 0,10 | **0,28** |
| **5b** | | ja | 20 | 6 | - | 0,22 | 0,08 | **0,13** |
| **5c** | | ja | 20 | 9 | - | 0,20 | 0,09 | **0,11** |
| **5d** | | ja | 35 | 3 | - | 0,34 | 0,08 | **0,25** |
| **5e** | | ja | 35 | 6 | - | 0,11 | 0,10 | **0,02** |
| **5f** | | ja | 35 | 9 | - | 0,20 | 0,09 | **0,11** |
| **6b** | Vergleichsbeispiel | ja | 20 | - | 10 | 0,61 | 0,11 | **0,50** |
| **6c** | Vergleichsbeispiel | ja | 35 | - | 10 | 0,22 | 0,13 | **0,10** |

Tabelle 8: Verfärbungen Ascorbylpalmitat-Test nach 24h

| | Beispiel Nr. | HT | %SiO2 | % Phosphorsäuremonocetylester (C16) [Hostaphat CC100] | % Octylphosphonsäure (Rhodia) | % Al2O3 | Beschichtung | Ascorbylpalmitat-Test k/s [430nm] | Ascorbylpalmitat-Test k/s [750nm] | Ascorbylpalmitat-Test k/s (430nm - 750nm) |
|---|---|---|---|---|---|---|---|---|---|---|
| | 0 | - | - | - | | | | - | - | - |
| | 4c | nein | 35 | 15 | | | | 0,47 | 0,13 | 0,34 |
| | 5c | ja | 20 | 9 | | | | 0,20 | 0,09 | 0,11 |
| | 5f | ja | 35 | 9 | | | | 0,20 | 0,09 | 0,11 |
| | 7a | nein | | | 10 | 12 | | 0,13 | 0,07 | 0,06 |
| Eusolex T-S | 7b | nein | | | | 12 | 10% Stearinsäure | 0,82 | 0,12 | 0,70 |
| UV-Titan X195 | 8a | nein | 10 | | | | 9% Methicone | - | - | - |
| UV-Titan M195 | 8b | nein | 10 | | | | 9% Methicone | 2,92 | 0,23 | 2,69 |
| Parsol TX | 8d | nein | 12 | | | | 2% Dimethicone | - | - | - |
| UV-Titan M263 | 8e | nein | | | | | 7% Al-phosphat + 3% PVP | 0,14 | 0,09 | 0,05 |
| Degussa T805 | 8f | nein | 2,5 | | | | Trimethoxycaprylsilane | 1,56 | 0,15 | 1,41 |
| T-Lite SF-S | 8g | nein | X | | | X | Dimethicone/ Methicone Copolymer | 0,39 | 0,11 | 0,28 |
| UV-Titan M140 | 8h | nein | 10 | | | | | - | - | - |
| UV-Titan M262 | 8i | nein | | | | 6 | 2% Dimethicone | - | - | - |
| UV-Titan M170 | 8j | nein | | | | 7 | 11% Methicone | - | - | - |

Tabelle 9: Verfärbungen Ascorbylpalmitat-Test nach 24h

**Rezepturbeispiel 1: Sonnenschutz Softcreme (O/W)**

| **Rohstoff (INCI)** | **Gew.%** |
|---|---|
| A | |
| Titandioxid erfindungsgemäß | 3,00 |
| Steareth-10, Steareth-7, Stearyl alcohol | 2,00 |
| Glyceryl stearate, Ceteth-20 | 2,00 |
| Glyceryl stearate | 3,00 |
| Microwax | 1,00 |
| Oleyl oleate | 6,00 |
| Cetearyl octanoate | 14,00 |
| Caprylic/capric triglyceride | 4,00 |
| Propylparaben | 0,05 |
| B | |
| Propylene glycol | 4,00 |
| Allantoin | 0,20 |
| Wasser | 60,60 |
| Methylparaben | 0,15 |

**Herstellung:**

Phase A und Phase B auf 80 °C erhitzen. Phase B unter Rühren langsam zu Phase A geben, homogenisieren und unter Rühren abkühlen.

**Rezepturbeispiel 2: Sonnenschutz Softcreme (O/W)**

| **Rohstoff (INCI)** | % |
|---|---|
| A | |
| Titandioxid erfindungsgemäß | 10,00 |
| Steareth-10, steareth-7, stearyl alcohol | 3,00 |
| Glyceryl stearate, ceteth-20 | 3,00 |
| Glyceryl stearate | 3,00 |
| Microwax | 1,00 |
| Oleyl oleate | 4,00 |
| Cetearyl octanoate | 10,50 |
| Caprylic/capric triglyceride | 4,00 |
| Propylparaben | 0,05 |
| B | |
| Propylene glycol | 4,00 |
| Allantoin | 0,20 |
| Wasser | 57,10 |
| Methylparaben | 0,15 |

**Herstellung:**

Phase A und B auf 80 °C erhitzen. Phase B unter Rühren langsam zu Phase A geben, homogenisieren und unter Rühren abkühlen.

**Rezepturbeispiel 3: Sonnenschutzlotion (O/W)**

| **Rohstoff (INCI)** | % |
|---|---|
| A | |
| Octocrylene | 6,00 |
| Butyl methoxydibenzoylmethane | 2,00 |
| Polyglyceryl-3 methylglucose distearate | 4,00 |
| Ethylhexyl stearate | 8,00 |
| Cetearyl isononanoate | 2,00 |
| PVP/eicosene copolymer | 1,00 |
| Tocopheryl acetate | 1,00 |
| B | |
| Xanthan gum | 0,30 |
| Sodium cetearyl sulfate | 1,00 |
| Glycerin | 5,00 |
| Wasser | 65,70 |
| C | |
| Titandioxid erfindungsgemäß | 4,00 |
| D | |
| Phenoxyethanol, butylparaben, ethylparaben, propylparaben, methylparaben | 1,00 |

**Herstellung:**

Phase A auf 80 °C erhitzen. Das Keltrol der Phase B im Wasser vorquellen, dann die restlichen Rohstoffe zugeben und auf 80 °C erhitzen. Phase A zu Phase B gegen und 2 Min. homogenisieren (Stabmixer): unter Rühren abkühlen und bei 35 °C Phase C zufügen. Nochmals 1 Min. homogenisieren (Stabmixer). Auf Raumtemperatur abkühlen und Phase D einrühren.

**Rezepturbeispiel 4: Sonnenschutzlotion (O/W)**

| | |
|---|---|
| A | |
| Steareth-10, steareth-7, stearyl alcohol | 3,00 |
| Glyceryl stearate, ceteth-20 | 3,00 |
| Cetearyl octanoate | 13,00 |
| Glyceryl stearate | 3,00 |
| Oleyl oleate | 7,00 |
| Microwax | 1,00 |
| Caprylic/capric triglyceride | 6,00 |
| Konservierungsmittel | q.s |
| Butyl Methoxydibenzoylmethane | 2,00 |
| B | |
| 33%-ige wässrige Dispersion des Titandioxid erfindungsgemäß | 16,70 |
| Propylene glycol | 4,00 |
| Allantoin | 0,20 |
| Wasser | ad 100 |
| Konservierungsmittel | q.s. |
| C | |
| Wasser | 10,00 |
| Dihydroxyaceton | 4,00 |

**Herstellung:**

Phase A auf 75 °C und Phase B auf 80 °C erhitzen. Phase B langsam in Phase A einrühren. Homogenisieren und unter Rühren abkühlen.

**Rezepturbeispiel 5: Sonnenschutz Spray-Lotion (O/W)**

| | |
|---|---|
| A | |
| Titandioxid erfindungsgemäß | 5,00 |
| Ethylhexyl methoxycinnamate, BHT | 7,50 |
| Benzophenone-3 | 2,50 |
| PEG-100 stearate, glyceryl stearate | 2,80 |
| PPG-1-PEG-9 lauryl glycol ether | 0,40 |
| Dicapryl ether | 4,50 |
| Steareth-10 | 0,50 |
| Stearyl alcohol | 0,60 |
| Dimethicone | 2,00 |
| B | |
| Dimethicone copolyol phosphate | 2,50 |
| Chitosan glycolate | 2,00 |
| Glycerin | 2,50 |
| Wasser | ad 100 |
| C | |
| PPG-1 trideceth-6, polyquaternium-37, propylene glycol dicaprylate/dicaprate | 0,40 |
| D | |
| Konservierungsmittel | q.s. |
| Wasser demineralisiert | 10,00 |
| Dihydroxyacetone | 3,00 |

**Herstellung:**

Phase A bis auf das Titandioxid zusammen geben und auf 60 °C erhitzen. Titandioxid langsam in die geschmolzene Ölphase einarbeiten. Phase B-1 auf 60 °C erhitzen, dann Phase B-2 unter Rühren eindispergieren. Phase A unter hohem Energieeintrag in die Phase B einrühren. Unter rühren abkühlen, und bei 40 °C Phase C zugeben. Homogenisieren und unter rühren Phase D zugeben.

**Rezepturbeispiel 6: Sonnenschutzspray mit Bräunungsintensivierung**

| | |
|---|---|
| A) CERALUTION® C; Fa. Sasol | 15.0% |
| B) Titandioxid erfindungsgemäß | 5.0% |
| Ethylhexyl Methoxycinnamate | 4.8% |
| Ethylhexyl Salicylate | 4.8% |
| Tocopheryl Acetate | 0.6% |
| Cyclomethicone | 1.0% |
| C12-15 Alkyl Benzoate | 2.5% |
| Tridecyl Salicylate | 2.5% |
| C) Dihydroxyaceton | 3% |
| Water (Aqua), Deionized | ad 100 |

(fortgesetzt)

| | |
|---|---|
| Water (Aqua), Deionized with 4% Avicel CL 611 (Microcrystalline Cellulose (and) Cellulose Gum) | 25.0% |

D) Konservierungsmitttel q.s.

Herstellung: Phase B wird bei Raumtemperatur unter rühren langsam zu Phase A gegeben. Danach wird Phase C zugegeben. Anschließend die Phase D zugegeben.

INCI CERALUTION® C:

Aqua (and) Capric / Caprylic triglyceride (and) Glycerin (and) Ceteareth-25 (and) Sodium Dicocoylethylenediamine PEG-15 Sulfate (and) Sodium Lauroyl Lactylate (and) Behenyl Alcohol (and) Glyceryl Stearate (and) Glyceryl Stearate Citrate (and) Gum Arabic (and) Xanthan Gum (and) Phenoxyethanol (and) Methylparaben (and) Ethylparaben (and) Butylparaben (and) Isobutylparaben

## Patentansprüche

1. Verwendung der Metalloxid-Partikel und/oder Metallhydroxid-Partikel von Silizium, Titan, Zink, Aluminium, Cer, Eisen, Yttrium oder Zirkonium oder Mischungen hieraus mit einer Primärpartikelgröße nach der Scherrer-Methode im Bereich von 5 nm bis 100 nm, nachbehandelt mit einer organischen Phosphorverbindung, ausgewählt aus der Gruppe der Hydroxyalkyldiphosphonsäuren, Alkylphosphonsäuren, die durch mindestens eine COOH-Gruppe substituiert sein können, Aminoalkylenphosphonsäuren oder organische Phosphorsäureester oder deren Salze, zur Stabilisierung von Dihydroxyaceton oder Derivaten des Dihydroxyacetons in einer Zubereitung.

2. Metalloxid-Partikel und/oder Metallhydroxid-Partikel von Titan mit einer Primärpartikelgröße nach der Scherrer-Methode im Bereich von 5 nm bis 100 nm, nachbehandelt mit einer organischen Phosphorverbindung, ausgewählt aus Phosphorsäuremonoalkylestern.

3. Partikel nach Anspruch 2, **dadurch gekennzeichnet, dass** die organische Phosphorverbindung in einer Menge von 5 bis 20 Gewichtsprozent, bezogen auf den $TiO_2$-Gehalt der zu beschichtenden Partikel, aufgebracht wird.

4. Partikel nach Anspruch 2 oder 3, **dadurch gekennzeichnet, dass** vor der Nachbehandlung mit einer organischen Phosphorverbindung mindestens eine weitere Beschichtung aufgebracht ist, enthaltend Metalloxide bzw. -hydroxide von Silizium, Titan, Zink, Aluminium, Cer, Eisen, Yttrium, Mangan oder Zirkonium, wobei das Metall unterschiedlich zum Metall des Grundpartikels ausgewählt wird, organische Säuren, ausgewählt aus der Gruppe Stearinsäure, Laurinsäure, Capronsäure oder Palmitinsäure, Polyole, Polymere oder silikonorganische Verbindungen.

5. Verfahren zur Herstellung von Partikeln nach einem oder mehreren der Ansprüche 2 bis 4, **dadurch gekennzeichnet, dass** eine rührfähige Dispersion eines Metalloxid-Grundköprers und/oder eines Metallhydroxid-Grundkörpers von Titan, auf den gegebenenfalls mindestens eine weitere Beschichtung aufgebracht wurde, erhitzt wird, die organische Phosphorverbindung zugegeben wird und nach vollständiger Nachbehandlung gegebenenfalls gewaschen und getrocknet wird.

6. Zubereitung enthaltend Titandioxid-Partikel nach einem der Ansprüche 2 bis 4.

7. Zubereitung nach Anspruch 6, **dadurch gekennzeichnet, dass** es sich um eine wässrige oder ölige Dispersion handelt.

8. Zubereitung nach Anspruch 6, **dadurch gekennzeichnet, dass** es sich um eine topisch anwendbare Zubereitung handelt.

9. Zubereitung nach Anspruch 6, **dadurch gekennzeichnet, dass** es sich um eine Zubereitung ausgewählt aus der Gruppe Fasern, Textilien, einschließlich deren Beschichtungen, Anstrichstoffe, Beschichtungssysteme, Folien und Verpackungen für den Schutz von Lebensmitteln, Pflanzen oder technischen Gütern handelt.

**10.** Zubereitung nach Anspruch 6, 7 oder 8, **dadurch gekennzeichnet, dass** die Zubereitung Dihydroxyaceton oder ein Dihydroxyacetonderivat enthält.

**11.** Zubereitung nach Anspruch 10, **dadurch gekennzeichnet, dass** die Konzentration an Dihydroxyaxeton oder dem Dihydroxyacetonderivat im Bereich von 1 bis 12 Gewichtsprozent liegt, bezogen auf die Zubereitung.

**12.** Zubereitung nach einem oder mehreren der Ansprüche 6 bis 11, **dadurch gekennzeichnet, dass** die Zubereitung mindestens einen organischen UV-Filter enthält.

**13.** Verfahren zur Herstellung einer Zubereitung nach einem oder mehreren der Ansprüche 6 bis 8 und 10 bis 12, **dadurch gekennzeichnet, dass** die Partikel nach einem oder mehreren der Ansprüche 2 bis 4 mit einem kosmetisch oder dermatologisch geeignetem Träger und gegebenenfalls weiteren Inhaltstoffen vermischt wird.

## Claims

**1.** Use of metal oxide particles and/or metal hydroxide particles of silicon, titanium, zinc, aluminium, cerium, iron, yttrium or zirconium or mixtures thereof having a primary particle size according to the Scherrer method in the range from 5 nm to 100 nm, aftertreated with an organophosphorus compound selected from the group of the hydroxyalkyl-diphosphonic acids, alkylphosphonic acids, which may be substituted by at least one COOH group, aminoalkylenephosphonic acids or organic phosphoric acid esters or salts thereof, for the stabilisation of dihydroxyacetone or derivatives of dihydroxyacetone in a preparation.

**2.** Metal oxide particles and/or metal hydroxide particles of titanium having a primary particle size according to the Scherrer method in the range from 5 nm to 100 nm, aftertreated with an organophosphorus compound selected from phosphoric acid monoalkyl esters

**3.** Particles according to Claim 2, **characterised in that** the organophosphorus compound is applied in an amount of 5 to 20 per cent by weight, based on the $TiO_2$ content of the particles to be coated.

**4.** Particles according to Claim 2 or 3, **characterised in that**, before the aftertreatment with an organophosphorus compound, at least one further coating is applied comprising metal oxides or hydroxides of silicon, titanium, zinc, aluminium, cerium, iron, yttrium, manganese or zirconium, where the metal is selected differently from the metal of the primary particle, organic acids selected from the group stearic acid, lauric acid, caproic acid or palmitic acid, polyols, polymers or organosilicone compounds.

**5.** Process for the production of particles according to one or more of Claims 2 to 4, **characterised in that** a stirrable dispersion of a metal oxide primary body and/or a metal hydroxide primary body, to which at least one further coating has optionally been applied, is heated, the organophosphorus compound is added and, when the aftertreatment is complete, is optionally washed and dried.

**6.** Preparation comprising titanium dioxide particles according to one of Claims 2 to 4.

**7.** Preparation according to Claim 6, **characterised in that** it is an aqueous or oily dispersion.

**8.** Preparation according to Claim 6, **characterised in that** it is a preparation which can be applied topically.

**9.** Preparation according to Claim 6, **characterised in that** it is a preparation selected from the group fibres, textiles, including coatings thereof, paints, coating systems, films and packaging for the protection of foods, plants or industrial products.

**10.** Preparation according to Claim 6, 7 or 8, **characterised in that** the preparation comprises dihydroxyacetone or a dihydroxyacetone derivative.

**11.** Preparation according to Claim 10, **characterised in that** the concentration of dihydroxyacetone or the dihydroxyacetone derivative is in the range from 1 to 12 per cent by weight, based on the preparation.

**12.** Preparation according to one or more of Claims 6 to 11, **characterised in that** the preparation comprises at least

one organic UV filter

13. Process for preparing a preparation according to one or more of Claims 6 to 8 and 10 to 12, **characterised in that** the particles according to one or more of Claims 2 to 4 are mixed with a cosmetically or dermatologically suitable vehicle and optionally further ingredients.

**Revendications**

1. Utilisation de particules d'oxyde de métal et/ou de particules d'hydroxyde de métal de silicium, titane, zinc, aluminium, cérium, fer, yttrium ou zirconium ou des mélanges de ceux-ci ayant une taille de particule primaire selon la méthode de Scherrer dans la plage allant de 5 nm à 100 nm, post-traitées par un composé organophosphoré choisi dans le groupe constitué par les acides hydroxyalkyldiphosphoniques, les acides alkylphosphoniques, pouvant être substitués par au moins un groupement COOH, les acides aminoalkylènephosphoniques ou des esters d'acide phosphorique organiques ou des sels de ceux-ci, pour la stabilisation de dihydroxyacétone ou de dérivés de dihydroxyacétone dans une préparation.

2. Particules d'oxyde de métal et/ou particules d'hydroxyde de métal de titane ayant une taille de particule primaire selon la méthode de Scherrer dans la plage allant de 5 nm à 100 nm, post-traitées par un composé organophosphoré choisi parmi les monoalkyl esters d'acide phosphorique.

3. Particules selon la revendication 2, **caractérisées en ce que** le composé organophosphoré est appliqué selon une quantité allant de 5 à 20 pour cent en poids, sur la base de la teneur en $TiO_2$ des particules à revêtir.

4. Particules selon la revendication 2 ou 3, **caractérisées en ce que**, avant le post-traitement par un composé organophosphoré, au moins un autre revêtement est appliqué, comprenant des oxydes ou hydroxydes de métal de silicium, titane, zinc, aluminium, cérium, fer, yttrium, manganèse ou zirconium, où le métal est sélectionné différemment du métal de la particule primaire, des acides organiques sélectionnés dans le groupe constitué par l'acide stéarique, l'acide laurique, l'acide caproïque ou l'acide palmitique, des polyols, des polymères ou des composés organosiliconés.

5. Procédé de production de particules selon l'une ou plusieurs parmi les revendications 2 à 4, **caractérisé en ce qu'**une dispersion agitable d'un corps primaire d'oxyde de métal et/ou d'un corps primaire d'hydroxyde de métal, auquel au moins un revêtement supplémentaire a éventuellement été appliqué, est chauffée, le composé organophosphoré est ajouté et, une fois le post-traitement terminé, est éventuellement lavée et séchée.

6. Préparation comprenant des particules de dioxyde de titane selon l'une des revendications 2 à 4.

7. Préparation selon la revendication 6, **caractérisée en ce qu'**il s'agit d'une dispersion aqueuse ou huileuse.

8. Préparation selon la revendication 6, **caractérisée en ce qu'**il s'agit d'une préparation pouvant être appliquée par voie topique.

9. Préparation selon la revendication 6, **caractérisée en ce qu'**il s'agit d'une préparation choisie dans le groupe constitué par les fibres, les textiles, y compris les revêtements de ceux-ci, les peintures, les systèmes de revêtement, les films et les emballages destinés à la protection d'aliments, de plantes ou de produits industriels.

10. Préparation selon la revendication 6, 7 ou 8, **caractérisée en ce que** la préparation comprend de la dihydroxyacétone ou un dérivé de dihydroxyacétone.

11. Préparation selon la revendication 10, **caractérisée en ce que** la concentration en dihydroxyacétone ou en dérivé de dihydroxyacétone se trouve dans la plage allant de 1 à 12 pour cent en poids, sur la base de la préparation.

12. Préparation selon l'une ou plusieurs parmi les revendications 6 à 11, **caractérisée en ce que** la préparation comprend au moins un filtre anti-UV organique.

13. Procédé de préparation d'une préparation selon l'une ou plusieurs parmi les revendications 6 à 8 et 10 à 12, **caractérisé en ce que** les particules selon l'une ou plusieurs parmi les revendications 2 à 4 sont mélangées avec

un véhicule convenable sur le plan cosmétique ou dermatologique et éventuellement des ingrédients supplémentaires.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente

- WO 2007070204 A
- EP 0707051 A
- WO 02051945 A
- JP 2950495 B
- EP 154150 A
- DE 102007013368
- FR 2326405 A
- FR 2440933 A
- EP 0114607 A
- WO 9304665 A
- EP 0487404 A
- WO 9966896 A
- US 6242099 B1
- WO 0009652 A
- WO 0072806 A
- WO 0071084 A
- DE 4116123 A
- DE OS4308282 A

### In der Beschreibung aufgeführte Nicht-Patentliteratur

- Ullmanns Enzyklopädie der Technischen Chemie. 1978, vol. 15, 117 ff
- *Cosmetics & Toiletries,* Februar 1990, vol. 105, 53-64
- Die Colour Index Nummern (CIN) sind dem Rowe Colour Index. Society of Dyers and Colourists, 1971